# EUROPEAN PATENT APPLICATION

(11) **EP 4 272 551 A2**
(43) Date of publication of application: **08.11.2023**
(21) Application number: 23178750.8
(22) Date of filing: 26.02.2018
(51) Int. Cl.: A01K 67/027

(54) **DIFFERENTIATION AND USE OF HUMAN MICROGLIA-LIKE CELLS FROM PLURIPOTENT STEM CELLS AND HEMATOPOIETIC PROGENITORS**

(30) Priority: 28.02.2017 US 201762464925 P
(62) Divisional of application: 18760945.8
(71) Applicant: The Regents of the University of California, Oakland CA 94607-5200 (US)
(72) Inventor: BLURTON-JONES, Mathew, Oakland, CA 94607-5200 (US); ABUD, Edsel, Oakland, CA 94607-5200 (US); POON, Wayne, Oakland, CA 94607-5200 (US)
(74) Representative: Bartle Read

(57) **Abstract**

Disclosed herein are various embodiments relating to methods of producing iMGLs, for example, from pluripotent stem cells (PSCs), methods of using iMGLs, and compositions of iMGLs. Also disclosed herein are methods to study various neurological disorders, such as for studying Alzheimer's disease. In addition, disclosed herein are methods of investigating genotypic and phenotypic effects of microglia cells in various physiological and pathological environments in the CNS and brain.

## Description

### RELATED APPLICATIONS

This application claims the benefit of U.S. Provisional Application No. 62/464,925, filed February 28, 2017. The content of the aforementioned application is expressly incorporated herein by reference in its entirety.

### STATEMENT REGARDING FEDERALLY SPONSORED R&D

This invention was made with government support under AG048099 awarded by the National Institutes of Health. The government has certain rights in the invention.

### FIELD

Described herein are embodiments of (i) human microglial-like cells (iMGLs) and (ii) methods of making iMGLs.

### BACKGROUND

Microglia cells are innate immune cells of the CNS and are known to play roles in the physiological development of the CNS. In addition, microglial cells are known to play roles in neurological disorders such as Alzheimer's disease. There is a deficiency in the art of acquiring microglia cells to further investigate the roles microglia cells play in CNS development and neurological disorders.

### SUMMARY

In some embodiments, a method of producing human microglial-like (iMGLs) from pluripotent stem cells (PSCs) is provided. In some embodiments, the method comprises the steps: (i) differentiating PSCs using a media supplemented with hematopoietic differentiation factors to produce induced hematopoietic progenitor cells (iHPCs), (ii) isolating CD43⁺ iHPCs, (iii) differentiating the CD43⁺ iHPCs into iMGLs using a microglial differentiating media; and (iv) maturing the iMGLs.

In some embodiments, the method comprises the steps: (i) differentiating PSCs using a media supplemented with hematopoietic differentiation factors; and (ii) differentiating the CD43⁺ iHPCs into iMGLs using a microglial differentiating media.

In some embodiments, a method of producing a human microglial-like cell (iMGL) from a cell of a first type is provided. In some embodiments, the method comprises the steps of: (i) differentiating a cell of a first type into an induced hematopoietic progenitor cell (iHPC); and (ii) differentiating the iHPC to produce an iMGL.

In some embodiments, the PSCs are not derived from embryoid bodies. In some embodiments, the PSCs include single-cell PSCs.

In some embodiments, the PSCs include induced PSCs (iPSCs). In some embodiments, the PSCs include embryonic stem cells (ESCs). In some embodiments, the PSCs include mammalian PSCs. In some embodiments, the PSCs are of human origin. In some embodiments, the PSCs are mouse PSCs.

In some embodiments, a method of producing iMGLs from PSCs is provided comprising the steps: (i) differentiating PSCs into iHPCs and (ii) differentiating iHPCs into iMGLs.

In some embodiments, a composition of iMGLs is provided that comprises expression of any one, or any combination of two or more, of the following genes: RUNX1, SPI1, CSF1FR, CX3CR1, TGFBR1, RSG10, GAS6, MERTK, PSEN2, PROS1, P2RY12, P2RY13, GPR34, C1Q, CR3, CABLES1, BHLHE41, TREM2, TYROBP, ITGAM, APOE, SLCO2B1, SLC7A8, PPARD, TMEM119, GPR56, C9orf72, GRN, LRRK2, TARDBP, and CRYBB1.

In some embodiments, a method of assessing chemokine, cytokine, and other inflammatory molecule secretion is provided comprising the steps: (i) treating the iMGLs with lipopolysaccharide, IFNγ, or IL-1β, (ii) measuring chemokines, cytokines, and other secreted factors from iMGLs that can serve as potential biomarkers of inflammation or different neurodegenerative disease states. Some embodiments relate to a method of profiling secretion of inflammatory molecules from iMGLs comprising: (i) treating the iMGLs with lipopolysaccharide, IFNγ, TNFα, or IL-1β; and (ii) measuring inflammation markers secreted by the iMGLs.

In some embodiments, a method of assessing iMGLs migration is provided comprising the steps: (i) treating the iMGLs with ADP and (ii) measuring iMGL migration. Some embodiments relate to a method of assessing iMGL migration comprising: (i) treating the iMGLs with ADP; and (ii) assessing iMGL motility and migration in response to chemical stimuli.

In some embodiments, a method of producing calcium transients in iMGLs is provided comprising the steps: (i) treating the iMGLs with ADP and (ii) producing calcium transients in iMGLs. Some embodiments relate to a method of producing calcium transients in iMGLs comprising: (i) treating the iMGLS with ADP; and (ii) interrogating calcium flux signals in the iMGLs; wherein the calcium flux signals are produced in response to electrical, biological, or chemical stimulation.

In some embodiments, a method of differentially regulating gene expression in iMGLs is provided comprising the steps: (i) co-culturing iMGLs with neurons or astrocytes and (ii) differentially regulating genes in iMGLs.

In some embodiments, a method of integrating iMGLs into the CNS/brain (e.g., neuronal) environment is provided comprising the steps: (i) co-culturing iMGLs with hiPSC 3D brain-organoids (BORGs) and (ii) invading of the iMGLs into the BORGs. Some embodiments relate to a method of integrating iMGLs into a 3D CNS environment, comprising: co-culturing iMGLs with hiPSC 3D brain-organoids (BORGs), wherein the iMGLs migrate into the BORGs and populate the BORGS, or are incorporated into the BORGs.

In some embodiments, a method of differentially regulating gene expression in iMGLs is provided comprising the steps: (i) exposing iMGLs to any of the following compounds: Aβ, Tau, fluorescently labeled Aβ, pHrodo-labeled brain-derived tau oligomers and other brain-derived proteins implicated in neurodegenerative disease i.e. synuclein, huntingtin, prion (ii) differentially regulating genes in iMGLs. Some embodiments relate to a method of establishing an iMGL gene expression profile resembling the in vivo state of the iMGLs, comprising: co-culturing iMGLs with neurons, astrocytes, or other cells of the central nervous system, thereby recapitulating a more in vivo state for the iMGLs than would otherwise be present for the iMGLs if the iMGLs were not co-cultured with the neurons, astrocytes, or other cells of the central nervous system. Some embodiments relate to a method of studying microglia dysregulation in health and disease using iMGLs, comprising: (i) exposing iMGLs to a compound selected from the group consisting of Aβ, Tau, fluorescently labeled Aβ, pHrodo-labeled brain-derived tau oligomers, and alpha-synuclein; and (ii) profiling an iMGL -omic signature selected from RNA-seq, proteomics, metabolomics, and lipidomics.

In some embodiments, a method of phagocytosing human synaptosomes (hS) in iMGLs is provided comprising the steps: (i) exposing iMGLs to hS and (ii) measuring phagocytosis of hS. Some embodiments relate to a method of studying microglia phagocytosis of compounds comprising: (i) exposing iMGLs to a compound selected from the group consisting of Aβ, Tau, fluorescently labeled Aβ, and pHrodo-labeled brain-derived tau oligomers, wherein the compound is phagocytosed, endocytosed, or ingested by the iMGLs; and (ii) measuring the phagocytosis, endocytosis, or ingestion of the compound.

Some embodiments relate to a method of investigating the role of microglia in synaptic pruning and plasticity comprising: (i) exposing iMGLs to human synaptosomes and (ii) assessing human synaptosome phagocytosis by the iMGLs.

In some embodiments, a method of determining gene regulation is provided comprising (i) exposing iMGLs to one or more of the factors CX3CL1, CD200, and TGFβ, in any combination and (ii) assessing any one or more of the differentially regulated genes in any combination: P2ry12, EGR1, TGFβ1, ETV5, CX3CR1, APOE, BIN1, CD33, GPR84, COMT, APP, PSEN1, PSEN2, HTT, GRN, FUS, TARDP, VCP, SNCA, C9ORF72, LRRK2, and SOD1.

In some embodiments, a method of assessing engraftment of iMGLs into neural tissue (e.g., cortex) is provided comprising (i) transplanting iMGLs into the neural tissue and (ii) assessing engraftment of the iMGLs into the neural tissue.

In some embodiments, a method of assessing iMGL interaction with AD neuropathy is provided comprising (i) transplanting iMGLs into hippocampi and (ii) assessing interaction of iMGLs in the hippocampi.

In some embodiments, a method of studying human microglia in a 3D neuronal environment is provided comprising transplanting iMGLs into a mammalian brain.

Some embodiments of the methods, kits and compositions provided herein relate to a media for supporting generation of human iHPCs, the media comprising one or more of FGF2, BMP4, Activin A, and LiCl. Some embodiments of the methods and compositions provided herein relate to a media for supporting generation of human iHPCs, the media comprising one or more of FGF2 and VEGF. Some embodiments of the methods and compositions provided herein relate to a media for supporting generation of human iHPCs, the media comprising one or more of FGF2, VEGF, TPO, SCF, IL3, and IL6. Some embodiments relate to a kit for supporting generation of human iHPCs, with media that comprises one or more of FGF2, BMP4, Activin A, and LiCl. Some embodiments relate to a kit for supporting generation of iHPCs, with media that comprises one or more of FGF2 and VEGF. Some embodiments relate to a kit for supporting generation of human iHPCs, the kit including a media that comprises one or more of FGF2, VEGF, TPO, SCF, IL3, and IL6.

Some embodiments of the methods, kits and compositions provided herein relate to a media for supporting generation of human iMGLs, the media comprising one or more of CSF-1, IL-34, and TGFβ1. Some embodiments relate to a kit for supporting generation of human iMGLs, the kit including a media that comprises one or more of CSF-1, IL-34, and TGFβ1.

Some embodiments of the methods, kits and compositions provided herein relate to a media for supporting maturation or maintenance of iMGLs, the media comprising one or more of CD200 and CX3CL1. Some embodiments relate to a kit for supporting maturation or maintenance of iMGLs, the kit including a media that comprises one or more of CD200 and CX3CL1.

The compositions and related methods summarized above and set forth in further detail below describe certain actions taken by a practitioner; however, it should be understood that they can also include the instruction of those actions by another party. Thus, actions such as "transplanting iMGLs into a mammalian brain" include "instructing the transplantation iMGLs into a mammalian brain."

### BRIEF DESCRIPTION OF THE DRAWINGS

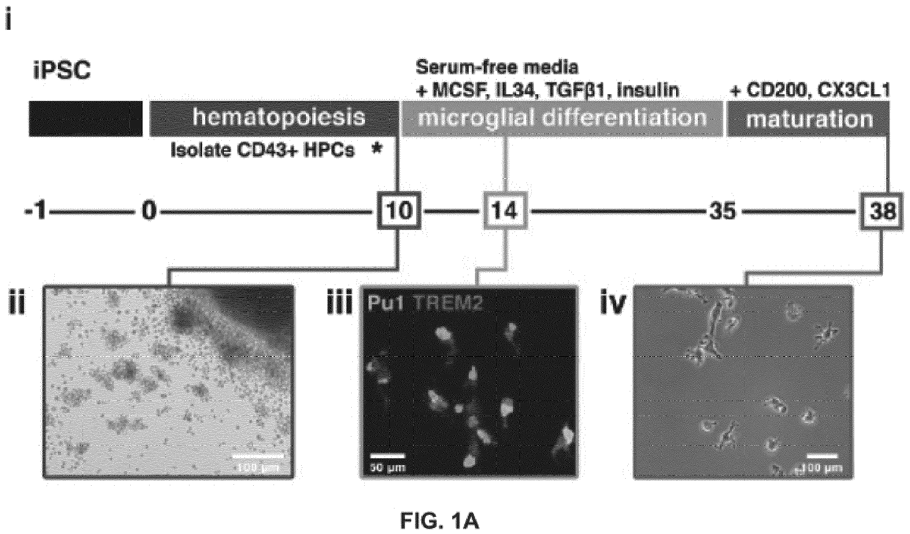

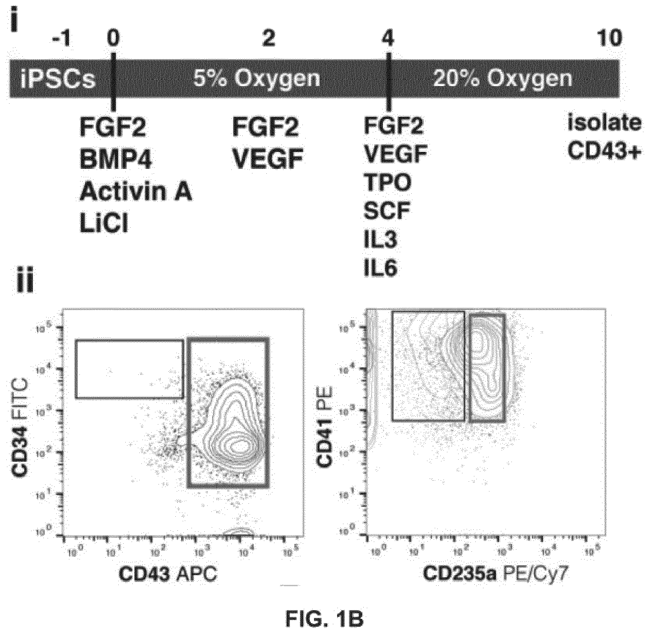

FIG. 1A. Schematic of fully-defined iMGL differentiation protocol. (i) Human iPSCs are differentiated to CD43⁺ iHPCs for 10 days and then cultured in serum-free microglia differentiation media containing human recombinant MCSF, IL-34, and TGFβ-1. Differentiation is carried out for an additional 25 days after which iMGLs are exposed to human recombinant CD200 and CX3CL1 for 3 days. (**ii**) Representative image of iHPCs in cell culture at day 10. Scale bar = 100 µm. (**iii**) By day 14, iMGLs express PU.1 (bright spots) and TREM2 (light spots). Scale bar = 50 µm. (**iv**) Representative phase contrast image of iMGL at day 38.
FIG. 1B. Schematic of differentiation of iPSCs to iHPCs. (**i**) Single-cell iPSCs are differentiated in a chemically defined media supplemented with hematopoietic differentiation factors, and using 5% O₂ (4 days), and 20% O₂ (6 days). (**ii**) After 10 days, CD43⁺ iHPCs are CD235a⁺/CD41a⁺.

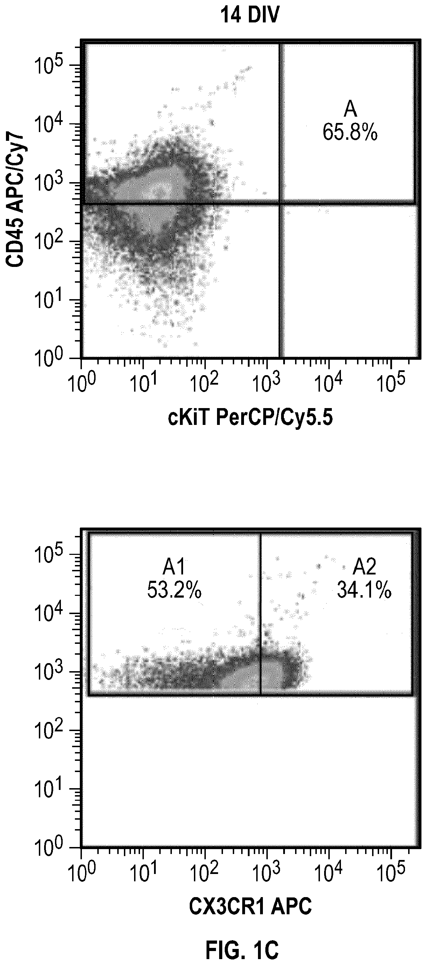

**FIG. 1C****.** iMGLs develop from CD45⁺/CX3CR1⁻ (A1) and CD45⁺/CX3CR1⁺ (A2) progenitors.

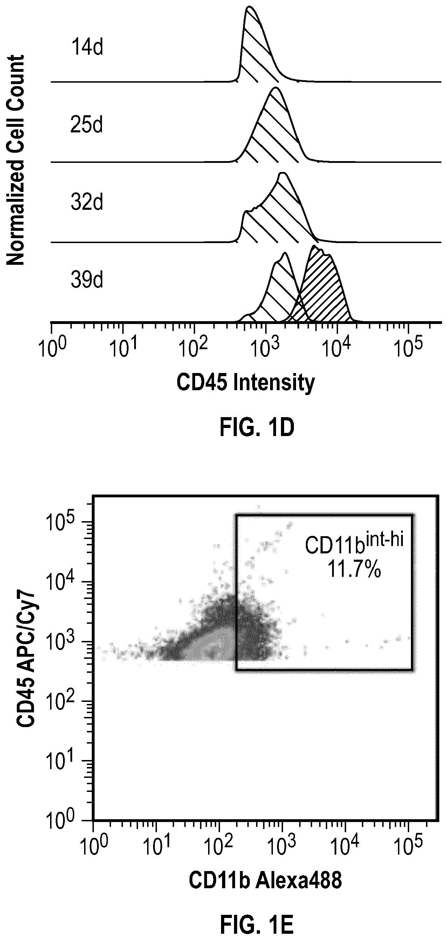

**FIG. 1D****.** CD45 fluorescence intensity shows that iMGLs (dark outer spots) maintain their CD45^{lo-int} profile when compared to monocyte-derived macrophage (MD-Mϕ).
**FIG. 1E** iMGL progenitors are CD11b^{lo} and increase their CD11b expression as they mature. At 14 DIV, a small population (-11%) cells with CD11b^{int-hi} are detected.

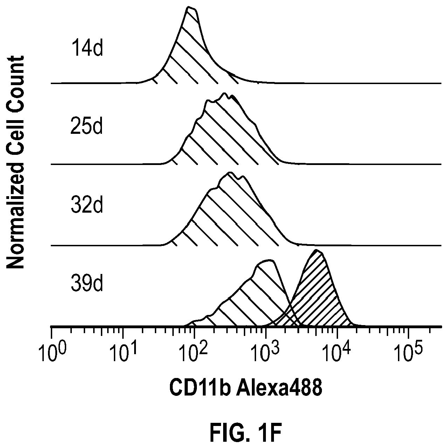

**FIG. 1F** CD11b fluorescence intensity demonstrates that CD11b expression increases as iMGLs age, resembling murine microglial progenitors.

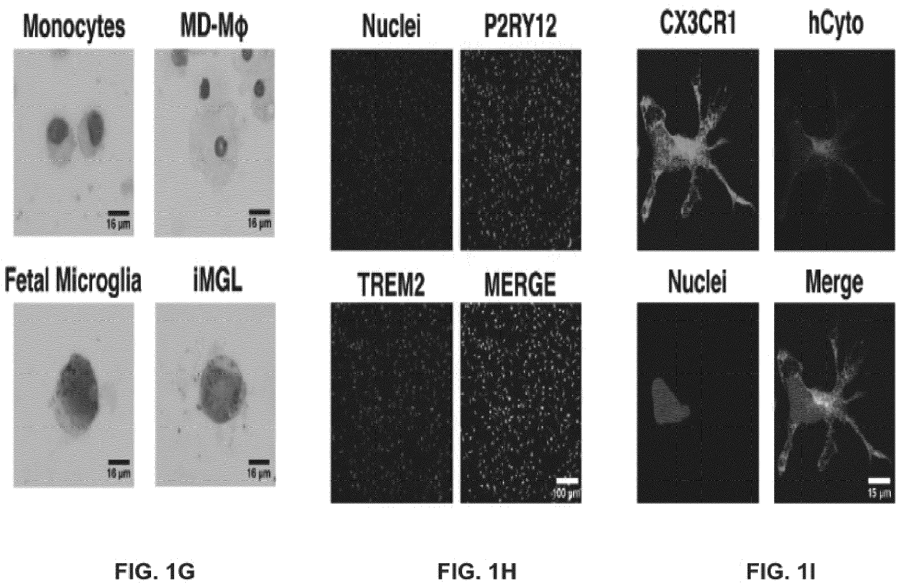

**FIG. 1G** Mary-Grunwald Giemsa stain of monocytes, MD-Mϕ, fetal microglia, and iMGLs. Both fetal microglia and iMGL exhibit a high nucleus to cytoplasm morphology compared to monocytes and MD-Mϕ. Scale bars = 16 µm.
**FIG. 1H****.** Differentiation yields >96% purity as assessed by co-localization of microglial-enriched protein P2ry12, microglial-enriched Trem2 (the merge panel shows overlay of the P2ry12, Trem2, and nuclei panels in which P2ry12 and Trem2 expression overlap).
**FIG. 1I**. iMGLs also exhibit extended processes and express Cx3Cr1 (upper left panel) and hCyto (Upper right panel). The merge panel shows that hCyto expression (bright spots) is localized to the same region as the Cx3Cr1 expression.

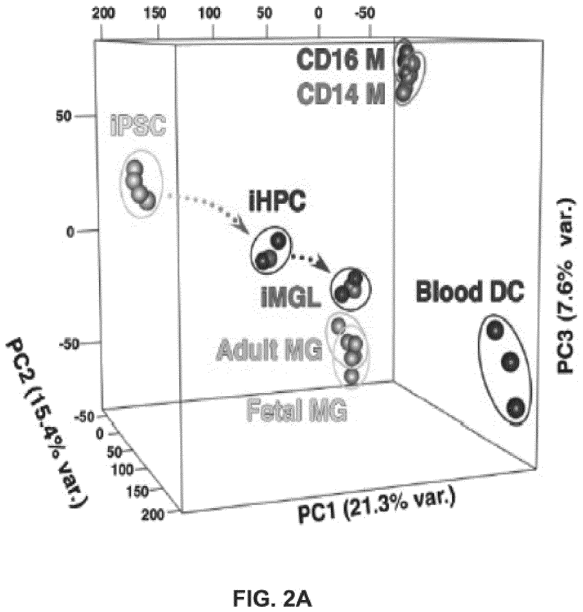

**FIG. 2A****.** 3D Principal Component Analysis (PCA) of iMGLs, human adult microglia (Adult MG) and human fetal microglia (Fetal MG) (Adult MG and Fetal MG are located in same circled cluster), CD14⁺/CD16⁻ monocytes (CD14 M), CD14⁺/16⁺ monocytes (CD16 M) (CD14 M and CD16 M are located in same circled cluster), blood dendritic cells (Blood DC), iHPCs, and iPSCs (FPKM ≥ 1, n=23,580 genes). PCA analysis reveals that iMGL cluster with Adult and Fetal MG and not with other myeloid cells. PC1 (21.3% var) reflects the time-series of iPSC differentiation to iHPC (arrow from iPSC cluster to iHPC cluster) and then to iMGLs (arrow from iHPC cluster to iMGL cluster). PC2 (15.4% var) reflects trajectory to Blood DCs. PC3 (7.6% var) reflects trajectory to monocytes.

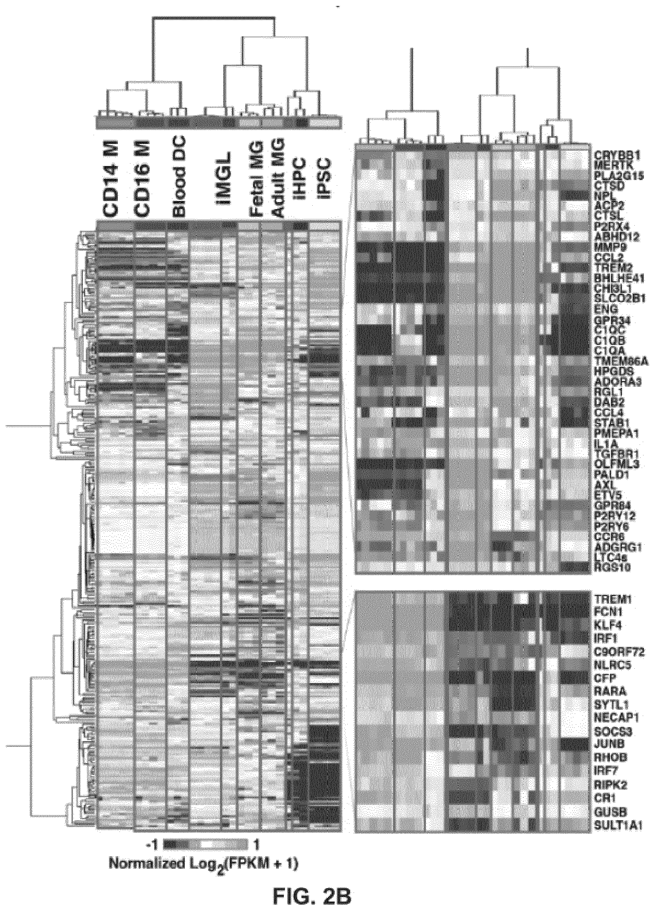

**FIG. 2B****.** Heatmap and biclustering (Euclidean-distance) on 300 microglia, myeloid, and other immune related genes (Butovsky et al., 2014, Hickman et al., 2013, Zhang et al., 2014). A pseudo-count was used for FPKM values (FPKM +1), log₂-transformed and each gene was normalized in their respective row (n=300). Representative profiles are shown for genes up and down regulated in both human microglia (fetal/adult) and iMGLs.

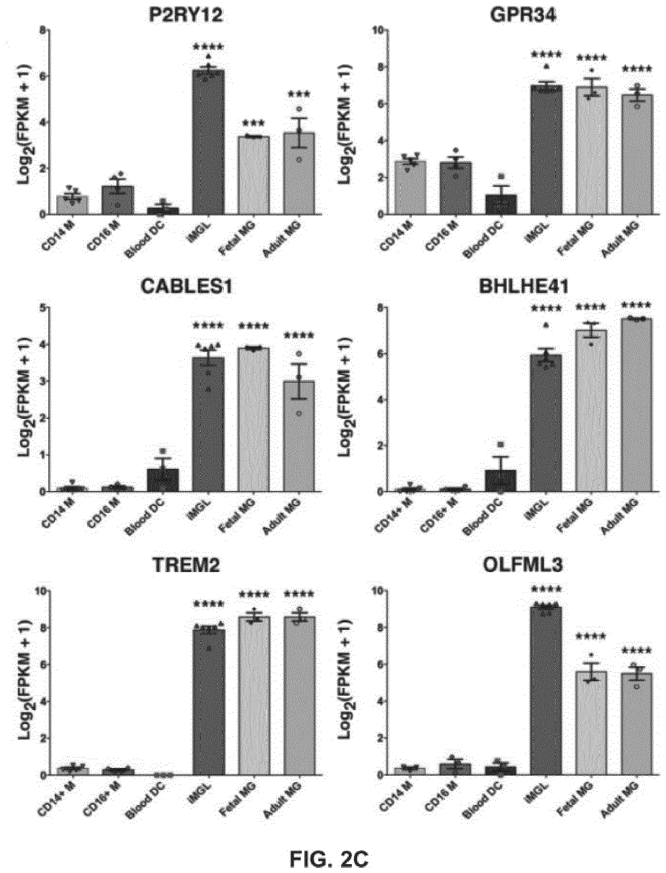

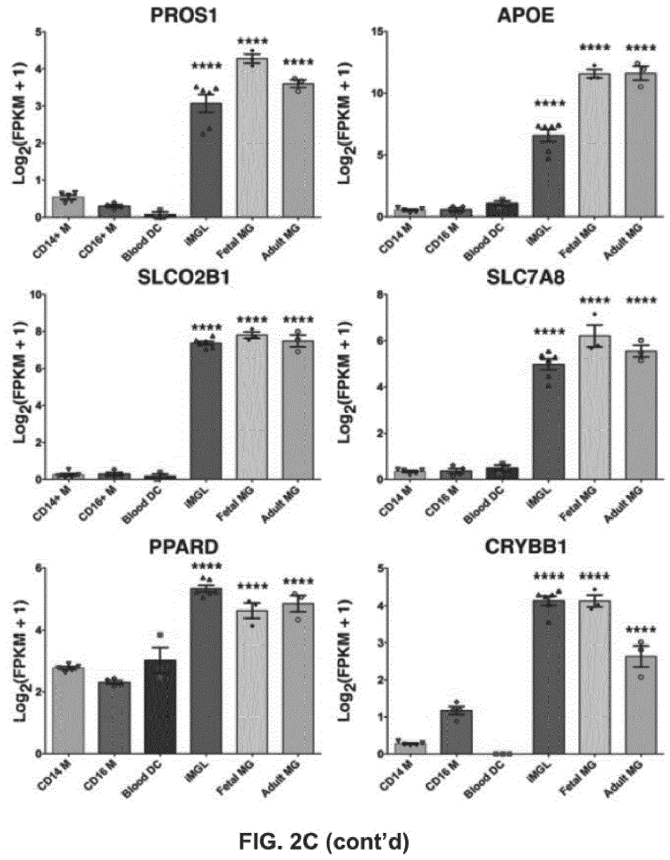

**FIG. 2C****.** Bar graphs of microglial-specific for enriched genes measured in iMGL, Fetal and Adult MG, Blood DC, CD14 M, and CD16 M as FPKM +1 followed by Log₂ transformation [Log₂ (FPKM +1)] presented as mean ± SEM. Data that was analyzed using 1-Way ANOVA followed by Tukey's corrected multiple comparison *post hoc* test. Statistical annotation represents greatest p-value for iMGL, Fetal MG, and Adult MG to other myeloid cells. CD14 M (n=5), CD16 M (n=4), Blood DC (n=3), iMGL (n=6), Fetal MG (n=3), and Adult MG (n=3). *p<0.05, **p<0.01, ***p<0.001, ****p<0.0001.

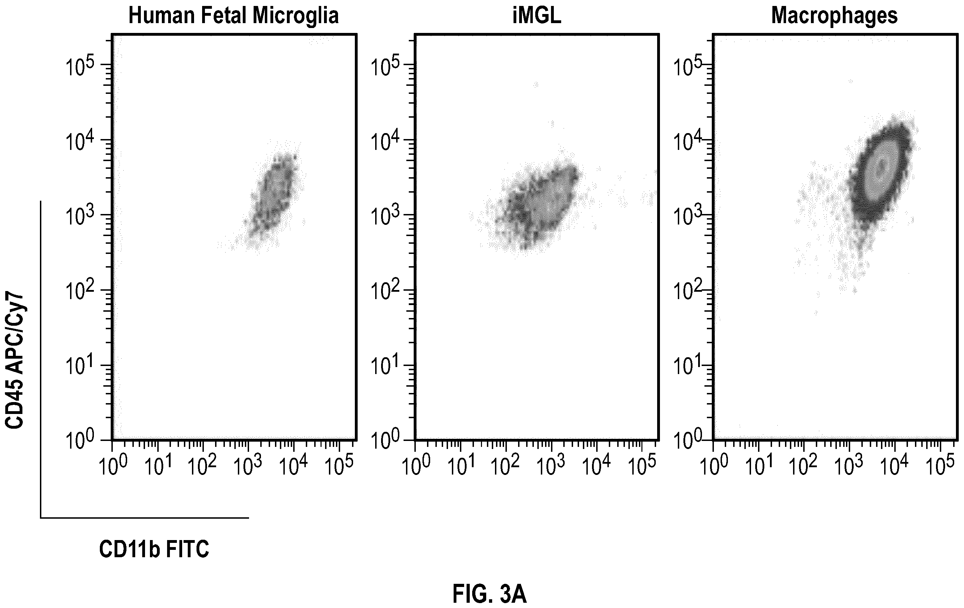

**FIG. 3A****.** By flow cytometry analysis, iMGL (outer dark spotted area in the three panels) are CD45^{lo-int} similar to fetal MG (inner most spotted area in the three panels).

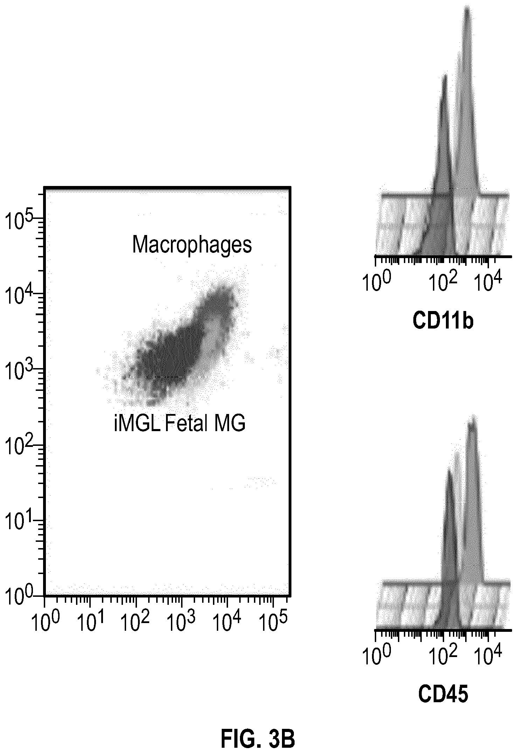

**FIG. 3B****.** iMGL (dark spots in left panel) are different from CD45-^{hi} MD-Mϕ (gray spots in left panel). Histogram of CD1 1b intensity (left histogram) reveals that fetal MG express slightly more CD1 1b than iMGL but less than MD-Mϕ.

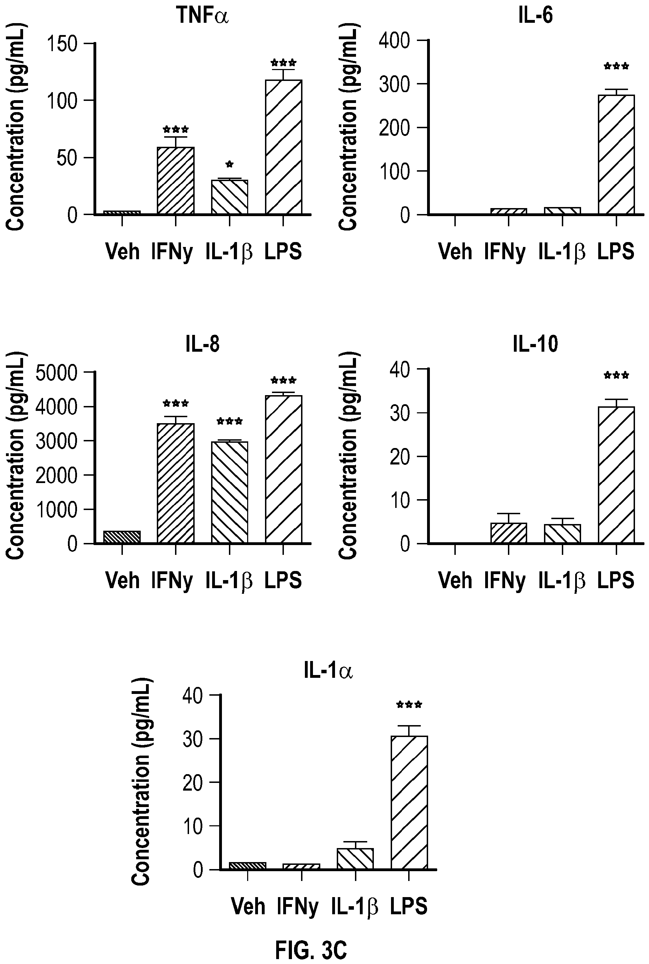

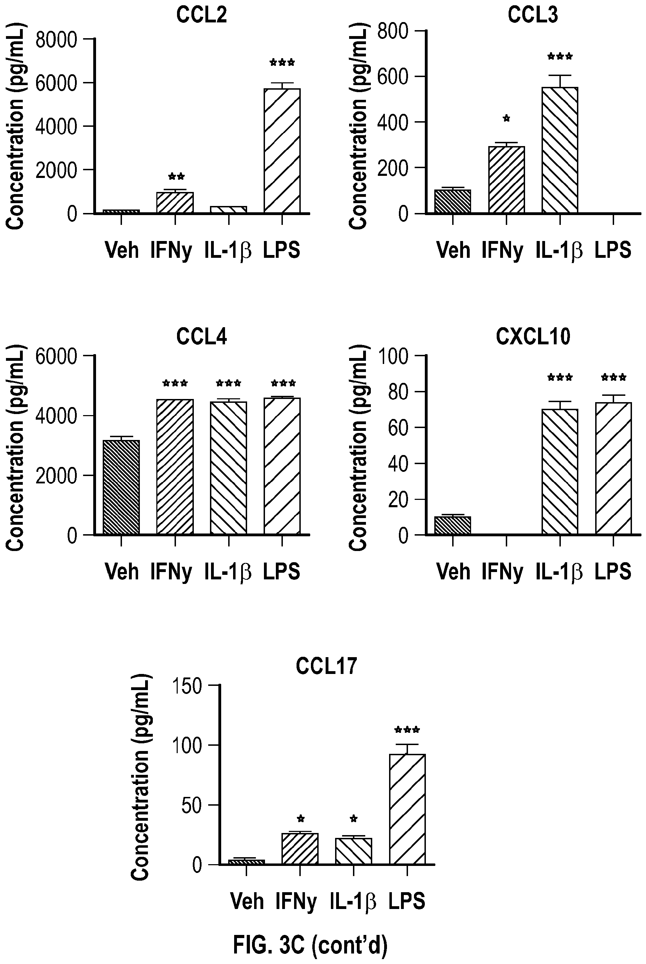

**FIG. 3C****.** iMGLs secrete cytokines and chemokines when stimulated for 24 hours with either IFNy (20 ng/ml), IL-1β (20 ng/ml), or LPS (100 ng/ml) by ELISA multiplex.

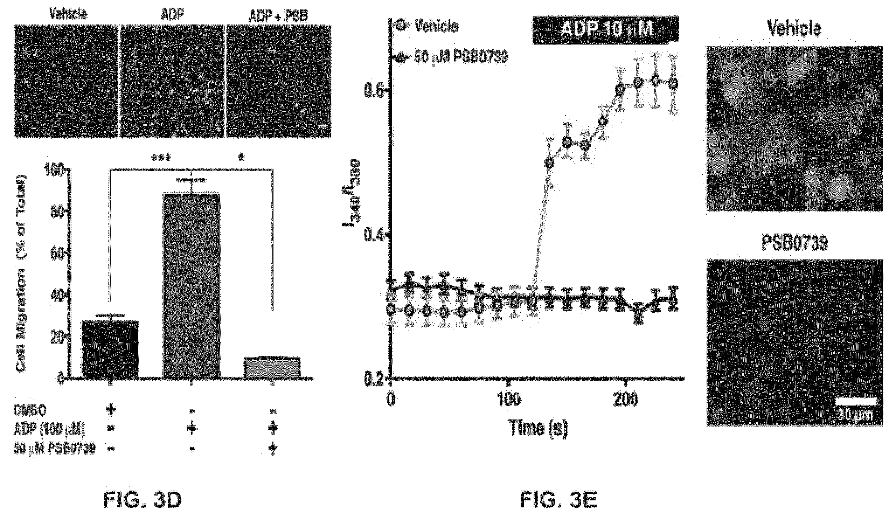

**FIG. 3D****.** ADP (100 µM) induces iMGL migration in a trans-well chamber (5 µm). Pre-exposure to the P2ry12 antagonist, PSB0739 (50 µM, 1 hr) completely abrogates ADP-induced iMGL migration (***p<0.0001).
**FIG. 3E****.** ADP induces calcium flux in iMGLs via P2ry12 receptors. (*Left*) Exposure to ADP leads to elevated calcium influx (I₃₄₀/I₃₈₀ ratio) in vehicle group (light trace) but not in PSB0739-treated group (dark trace). (*Right*) Representative images of ADP-induced calcium flux at 240 s in vehicle (top) and PSB0739 (bottom).

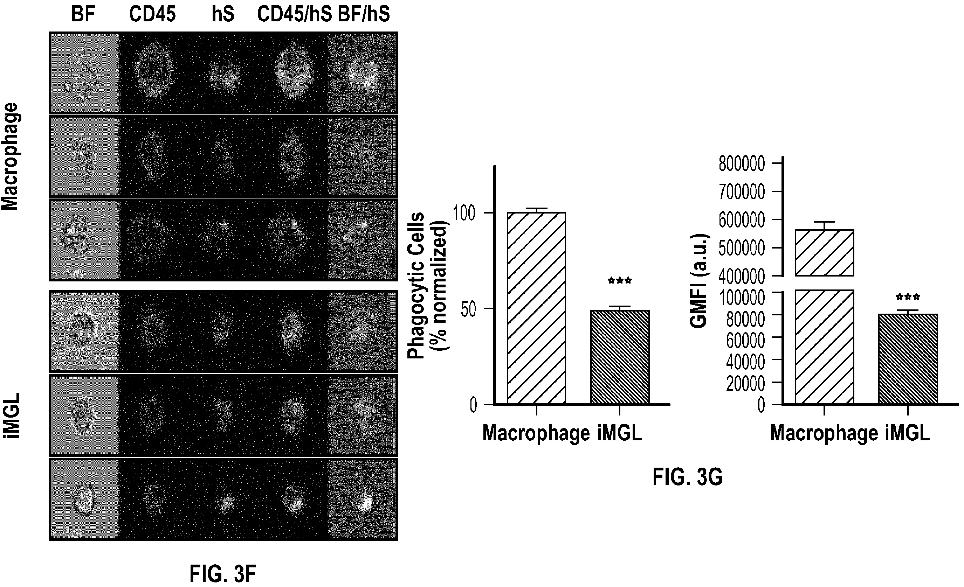

**FIG. 3F****.** iMGLs phagocytose human brain-derived synaptosomes (hS). Representative images captured on Amnis Imagestream display phagocytosis of hS by MD-Mϕ and iMGLs.
**FIG. 3G****.** Quantification of phagocyotsis shows that iMGLs internalize hS at 50% of macrophage capacity (p<0.0001).

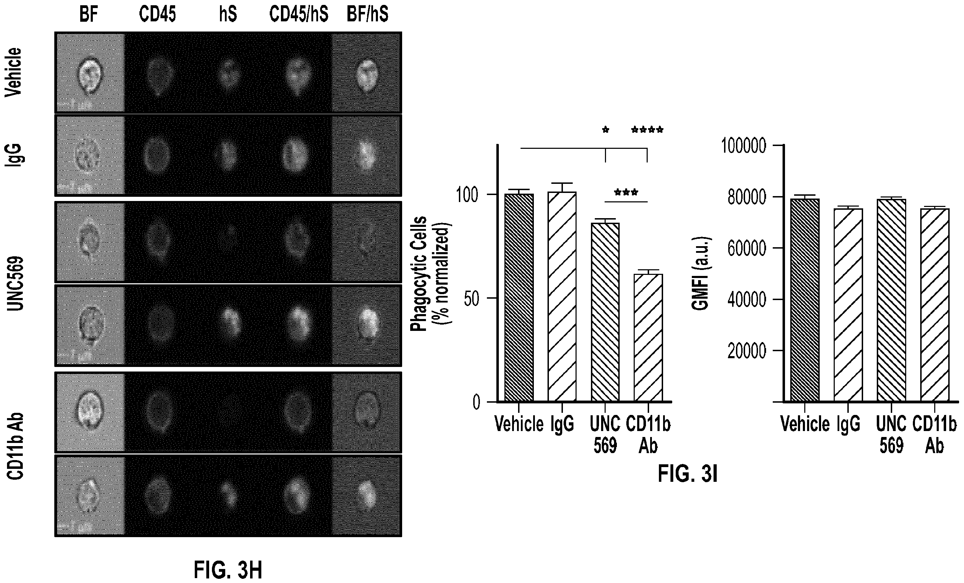

**FIG. 3H****.** Representative images of iMGL phagocytosis of hS in the prescence of either a MerTK inhibitor UNCS69 (top) or anti-CD11b antibody (bottom).
**FIG. 3I****.** (Top) iMGL phagocytosis of hS phagocytosis is reduced by approximately 12% (second bar from right, p<0.05) by blocking MerTK, but 40% (p<0.0001, right bar) by inhibiting CR3 via CD11b blockade. (Bottom) Sub-analysis of iMGLs exhibiting a phagocytic event reveals similar average amounts of internalization across treatment groups (p=0.1165). All histograms reported as mean ± SEM. Cytokine and migration assays 1-Way ANOVA, followed by Dunnett's multiple-comparison *post-hoc* test, ***p<0.0001, **p<0.001, *p<0.05; Cytokine assay: n=3 wells/group. Migration Assay: n=5 fields /condition. Calcium assay: vehicle (n=37 cells), PSB0739-treated (n=17 cells), I₃₄₀/I₃₈₀ represented as mean ± SEM at each time point. Phagocytosis assay: MD-Mϕ vs iMGL: Unpaired t-test, **p<0.001, n=3 wells/group. MERTK and CR3 assay, 1-Way ANOVA, followed by Tukey's multiple-comparison *post-hoc* test, ***p<0.0001; n= 6 for vehicle, n=3 wells/group.

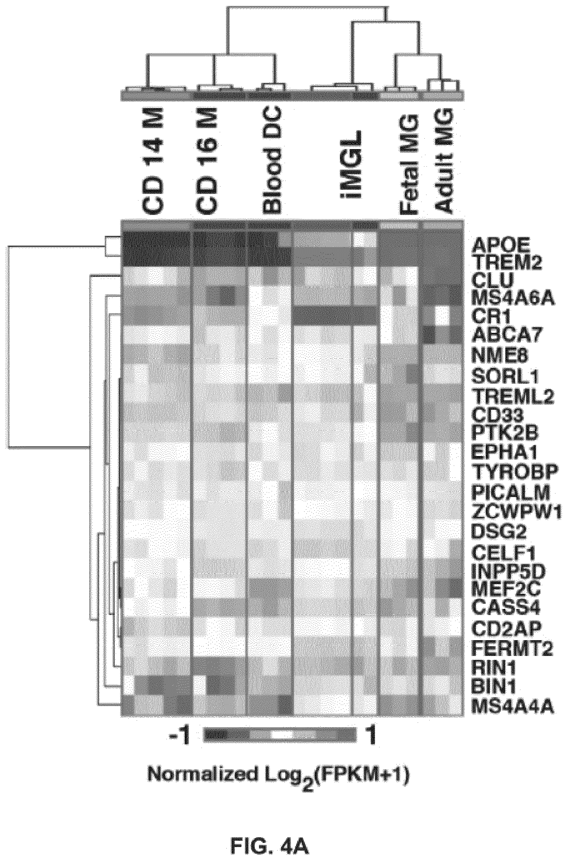

**FIG. 4A****.** Heatmap of 25 immune genes with variants associated with *LOAD* reveals that major risk factors APOE and TREM2 are highly expressed in iMGLs, Adult MG, and Fetal MG.

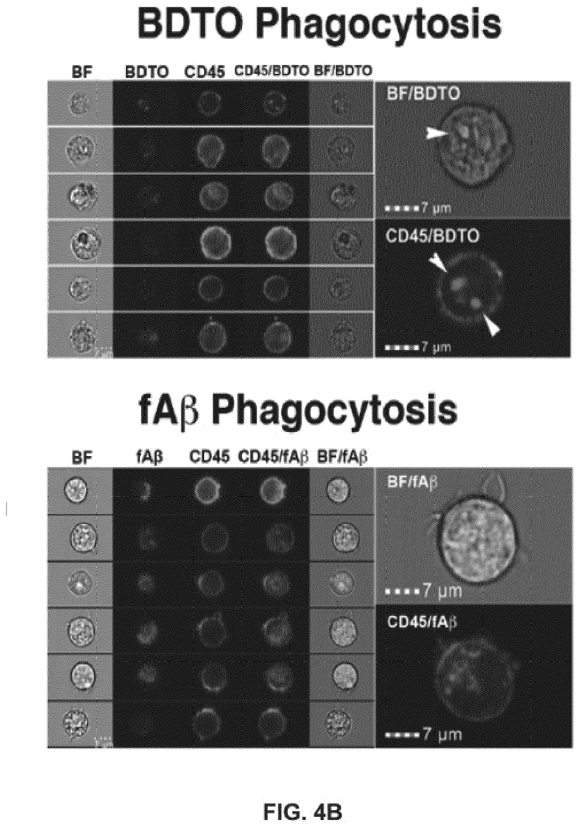

**FIG. 4B****.** iMGLs internalize fluorescent-labeled fAβ and pHrodo-dye BDTO. Representative images captured on Amnis Image StreamX Mark II.

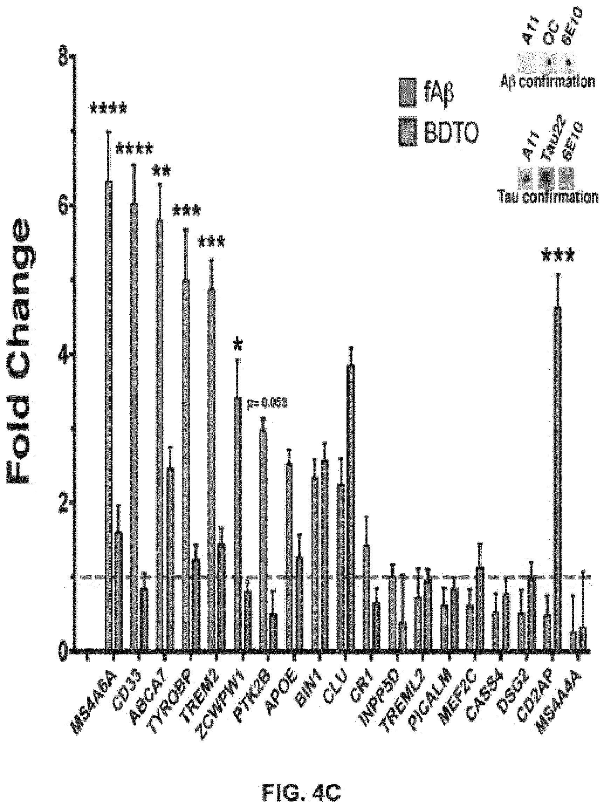

**FIG. 4C****.** iMGLs were exposed to unlabeled fAβ (5 µg-ml⁻¹) and BDTOs (5 µg/ml) for 24 h and mRNA expression of 19 GWAS genes was assessed via qPCR array. For each gene tested the bar corresponding to fAβ is on the left and the bar corresponding to BDTO treatment is on the right. fAβ treatment elevated the expression of 10 genes above 2-fold compared to vehicle, including MS4A6A (6.3 fold), CD33 (6.1 fold), ABCA7 (5.8 fold), TYROBP (4.98) and TREM2 (4.85 fold). Whereas, BDTO exposure elevated the expression of 4 genes above 2-fold compared to vehicle. 6 genes were differentially expressed in fAβ compared to BDTO. fAβ and BDTO preparations were confirmed via dot-blot analysis with conformation structural specific antibodies for oligomers (A11), fibrils (OC) and non-structural-specific antibodies for human Aβ (6E10) and tau oligomers (Tau22). Target genes were normalized to GAPDH and compared to vehicle expression by ΔΔCt. Bars show expression fold mean ± SEM. Red hash bar is ΔΔCt = 1. 2-Way ANOVA, followed by Sidak's multiple-comparison *post-hoc* test, ***p<0.0001, **p<0.001, *p<0.05; n=6 wells/group. Data represented as mean ± SEM.

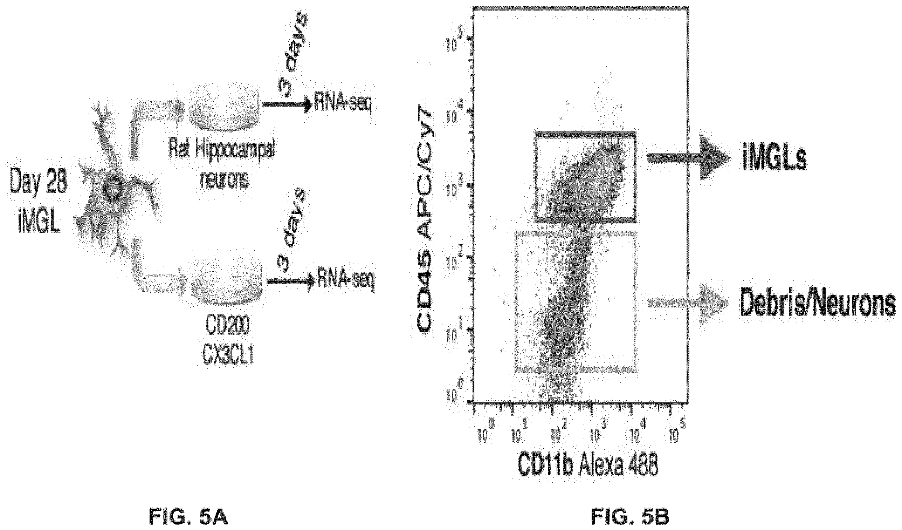

**FIG. 5A****.** Schematic of iMGL co-culture with or without rat hippocampal neurons.
**FIG. 5B****.** iMGLs co-cultured with neurons were collected, assessed by flow cytometry and transcriptomes evaluated via RNA-sequencing.

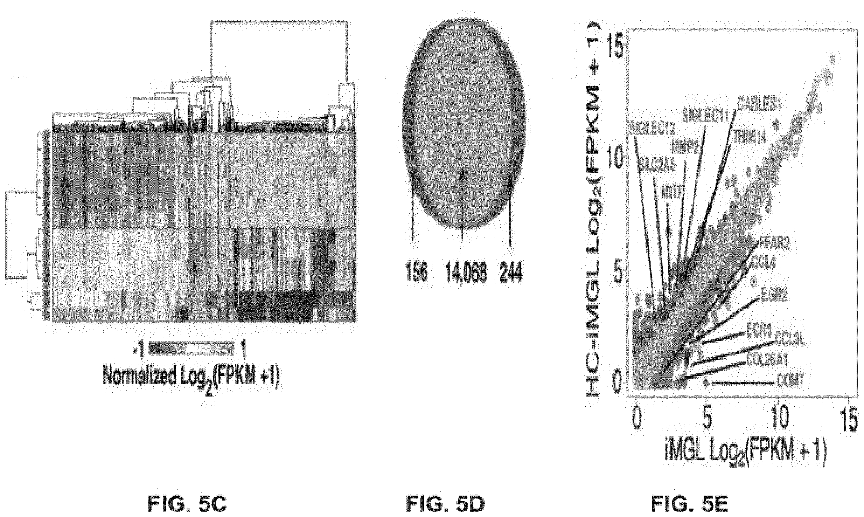

**FIG. 5C****.** Heat map of iMGLs and iMGL-HC gene expression highlights uniquely enriched genes.
**FIG. 5D****.** Differential gene expression analysis highlights 156 upregulated and 244 downregulated genes in iMGL-HCs.
**FIG. 5E****.** Scatter plot of differentially expressed genes [> 2 Log₂(FPKM+1)] highlight TRIM14, CABLES1, MMP2, SIGLEC 11 and 12, MITF, and SLC2A5 being enriched in iMGL-HCs, suggesting that iMGLs respond appropriately to a neuronal environment. Cells cultured alone are enriched for COMT, EGR2, EGR3, and FFAR2 suggesting a primed microglia phenotype.

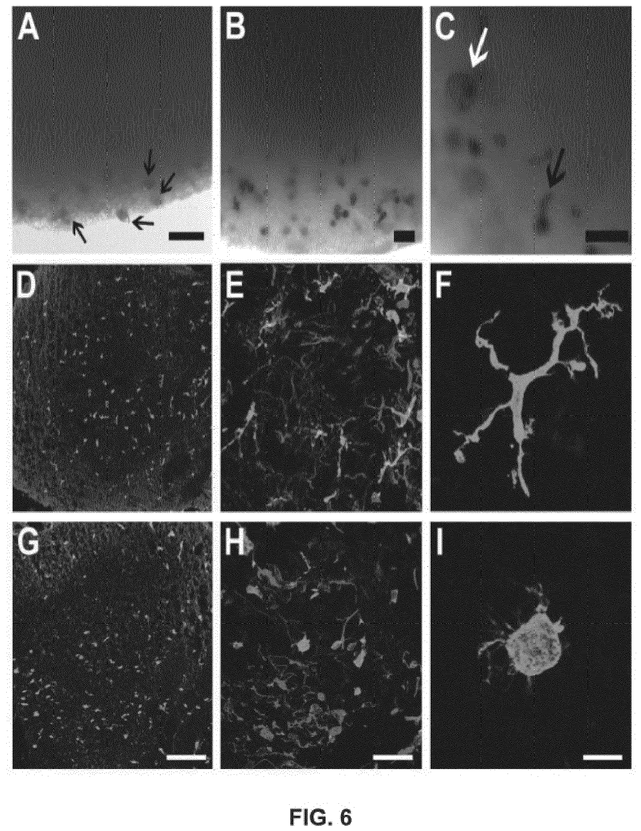

**FIG. 6****.** iMGLs (5 ×10⁵ cells) were added to media containing a single BORG for 7 days. **(Panel A)** Representative bright-field image of iMGLs detected in and near BORG after 3 days. iMGLs are found in and attached to BORG media interface (arrows), but not free floating in the media, suggesting complete chemotaxis of iMGLs. **(Panel B)** Representative image of iMGLs in outer and inner radius of BORG. **(Panel C)** Embedded iMGLs exhibit macrophage-like morphology (white arrow) and extend processes (black arrow) signifying ECM remodeling and surveillance respectively. Simultaneous assessment of embedded iMGL morphology in uninjured **(Panels D-F**) and injured **(Panels G-I)** BORGs. **(Panel D)** Immunohistochemical analysis of BORGs reveals iMGLs begin tiling evenly throughout the BORG and projecting ramified processes for surveillance of environment. BORGs are representative of developing brains *in vitro* and contain neurons and astrocytes, which self-organize into a cortical-like distribution, but lack microglia. iMGLs. **(Panels E-F)** Representative immunofluorescent images of iMGLs with extending processes within the 3D neuronal environment at higher magnification. **(Panels G-I)** Representative images of iMGL morphology observed in injured BORG. **(Panels H-I)** Round-bodied iMGLs reminiscent of amoeboid microglia are distributed in injured BORGs and closely resemble activated microglia, demonstrating that iMGLs respond appropriately to neuronal injury. Scale Bar = 50 µm in A-C, 200µm in D, G, 80µm in E, H, and 15µm in panels F, I.

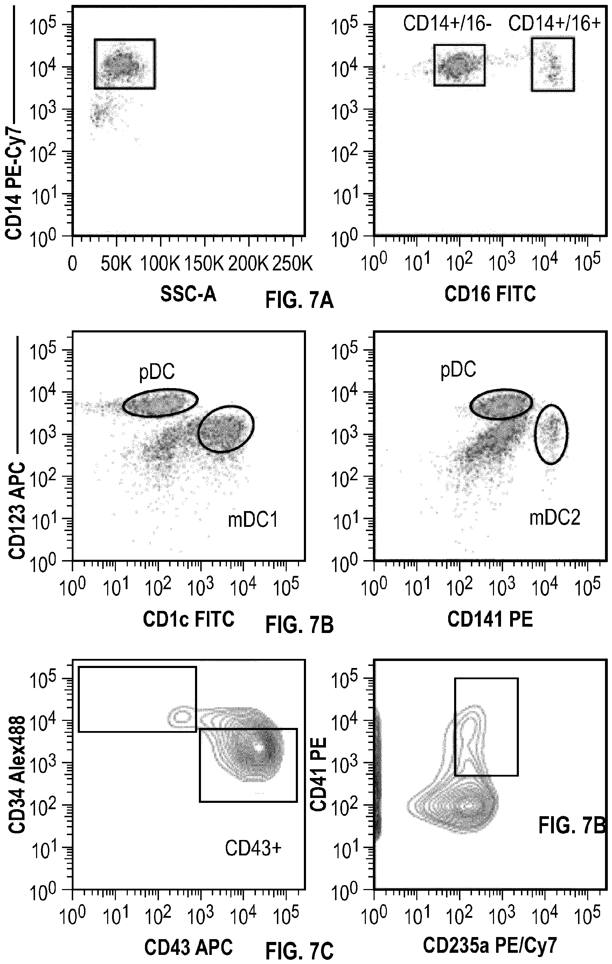

**FIG. 7A****.** Flow characterization of monocytes, dendritic cells, and commercial HPCs. Human CD14⁺/CD16⁻ monocytes and CD14⁺/CD16⁺ inflammatory monocytes were isolated from young healthy human blood (18-39 y.o.) by FACs. Cells were first gated on viability (not shown), then CD14 to avoid contaminating leukocytes, and finally isolated according to CD16 expression and collected for RNA.
**FIG. 7B****.** Flow characterization of monocytes, dendritic cells, and commercial HPCs. Human myeloid dendritic cells (Blood DCs) were isolated from young healthy human blood (18-39 y.o.) using untouched myeloid DC enrichment kit followed by FACs. To avoid plasmacytoid DC contamination, DCs were stained for CD123, and myeloid DC subtypes CD1c, and CD141 were collected for RNA.
**FIG. 7C****.** Flow characterization of monocytes, dendritic cells, and commercial HPCs. A commercial HPC source (CD43⁺/235a⁺/CD41⁺) cells were identified and used to compare to in-house HPC differentiation and further iMGL differentiation.

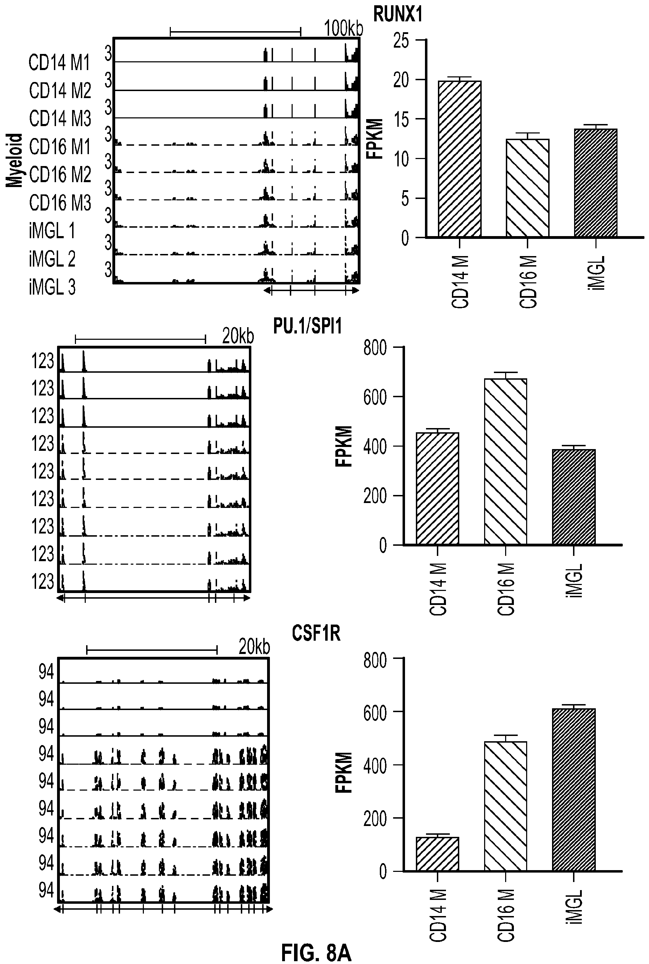

**FIG. 8A****.** RNA-seq coverage map and gene FPKM values in CD14⁺/16⁻ monocytes (CD14 M), CD14⁺/16⁺ monocytes (CD16 M), and iPS-derived microglia-like cells (iMGL) for the myeloid-specific genes RUNX1, PU.1, and CSF1R.

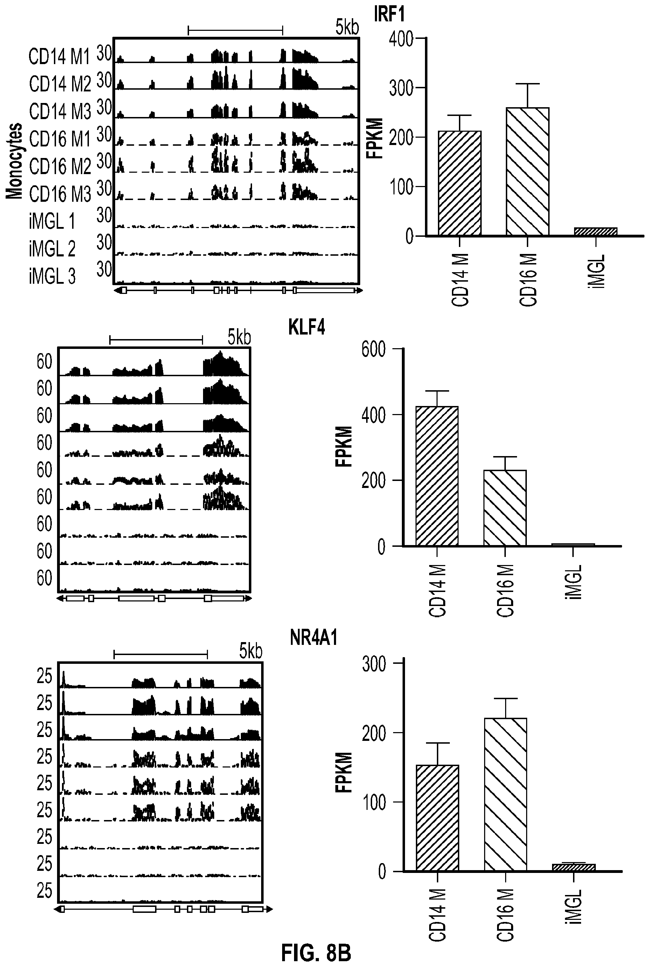

**FIG. 8B****.** RNA-seq coverage map and gene FPKM values in CD14⁺/16⁻ monocytes (CD14 M), CD14⁺/16⁺ monocytes (CD16 M), and iPS-derived microglia-like cells (iMGL) for the monocyte-specific genes IRF1, KLF4, and NR4A1.

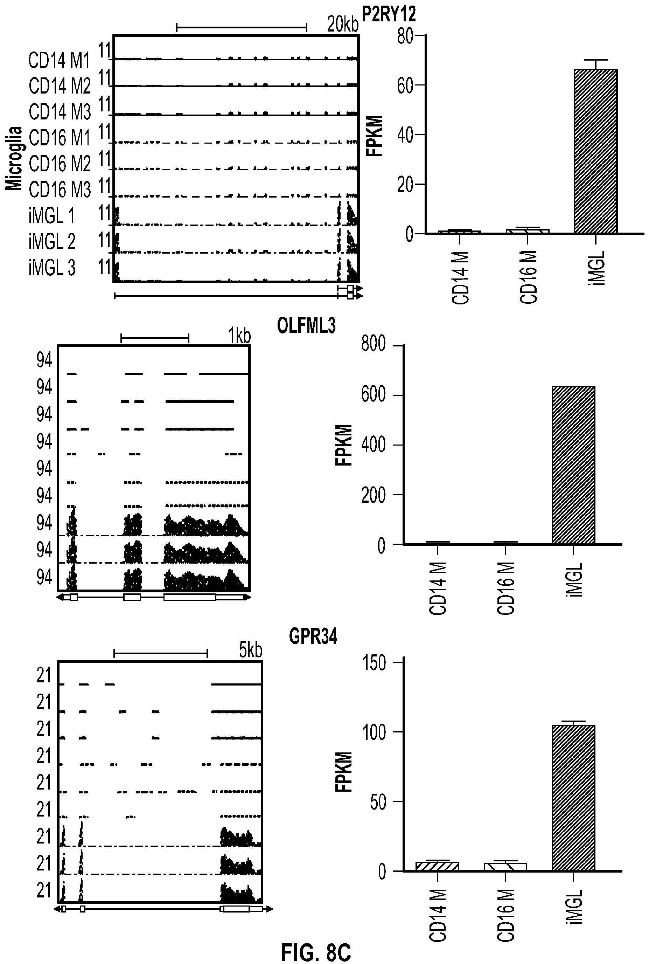

**FIG. 8C****.** RNA-seq coverage map and gene FPKM values in CD14⁺/16⁻ monocytes (CD14 M), CD14⁺/16⁺ monocytes (CD16 M), and iPS-derived microglia-like cells (iMGL) for the microglial-enriched genes P2RY12, OLFML3, and GPR34 in iMGL. For all RNA coverage maps (FIGS. 8A, 8B, and 8C), the y-axis represents Reads Per Million (RPM) scaled accordingly for all samples. Histogram comparisons using FPKM values for all genes are shown as the mean± s.e.m. Biological replicates for CD14 M (n=5), CD16 (n=4), and iMGL (n=6) are included for comparison by one-way ANOVA followed by Tukey's multiple-comparison post-hoc test. **p<0.001, ***p<0.0001.

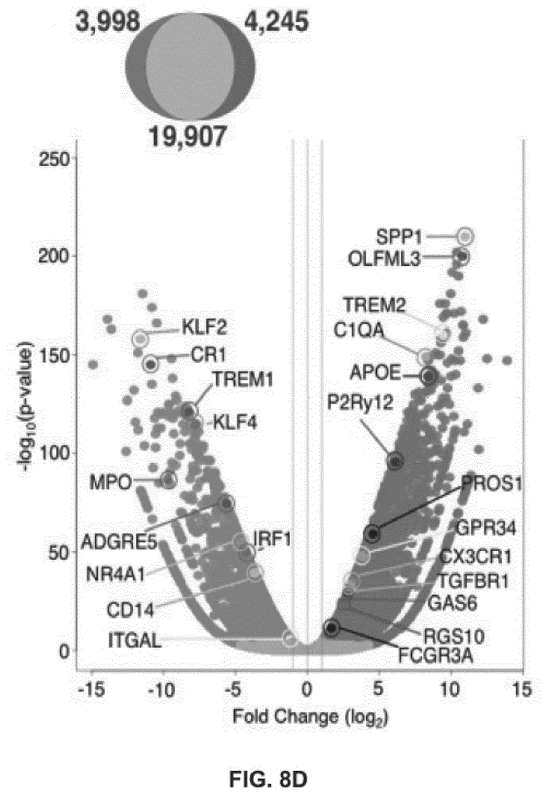

**FIG. 8D****.** Representative volcano plots of differentially expressed genes (p-value < 0.001, two-fold change) in iMGL (on right portion of plot), CD14 M (on left portion of plot), and non-significant (light portion at bottom of plot). Key genes are labeled. Fold change (log₂) and -log₁₀(p-value) indicate the x and y-axis respectively. Gray dashed vertical lines indicate a two-fold change in gene expression. Venn diagrams indicate total number of differentially expressed genes for each condition.

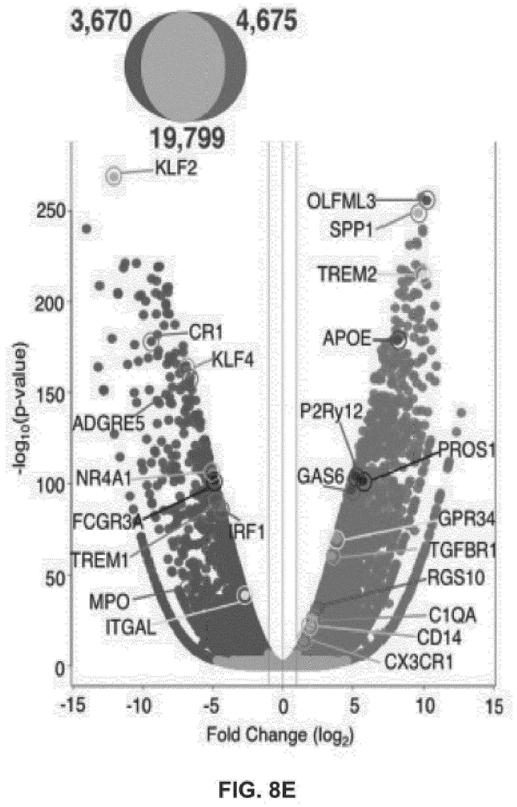

**FIG. 8E****.** Representative volcano plots of differentially expressed genes (p-value < 0.001, two-fold change) in iMGL (on right portion of plot), CD16 M (on left portion of plot), and those that are not significant (light portion at bottom of plot). Key genes are labeled. Fold change (log₂) and -log₁₀(p-value) indicate the x and y-axis respectively. Gray dashed vertical lines indicate a two-fold change in gene expression. Venn diagrams indicate total number of differentially expressed genes for each condition.

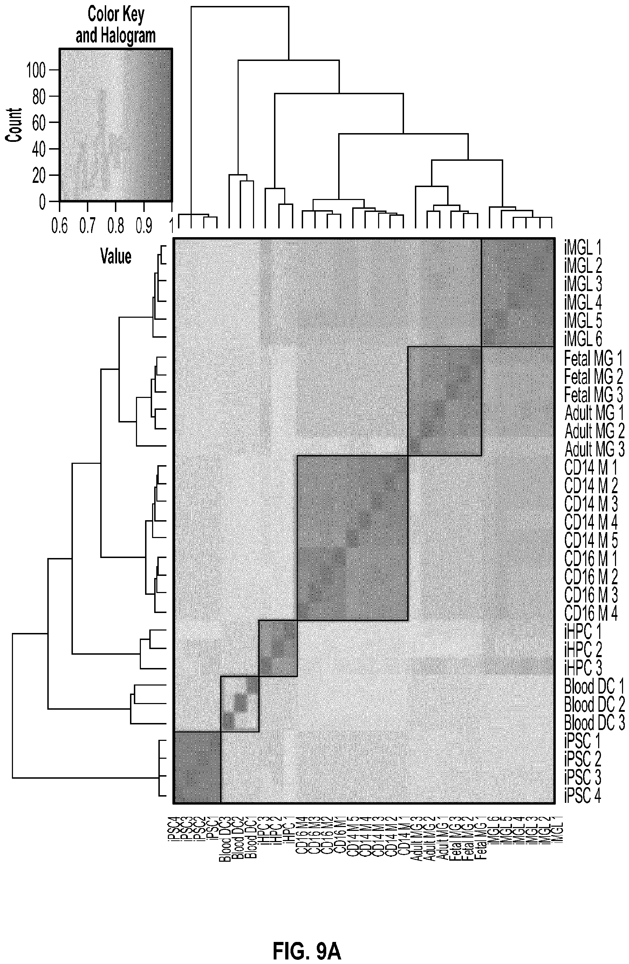

**FIG. 9A****.** Spearmen correlational matrix of biological samples used in RNA-sequencing highlights strong intra-group correlation. iMGLs correlate well with Fetal and Adult MGs suggesting strong gene expression similarity between samples.

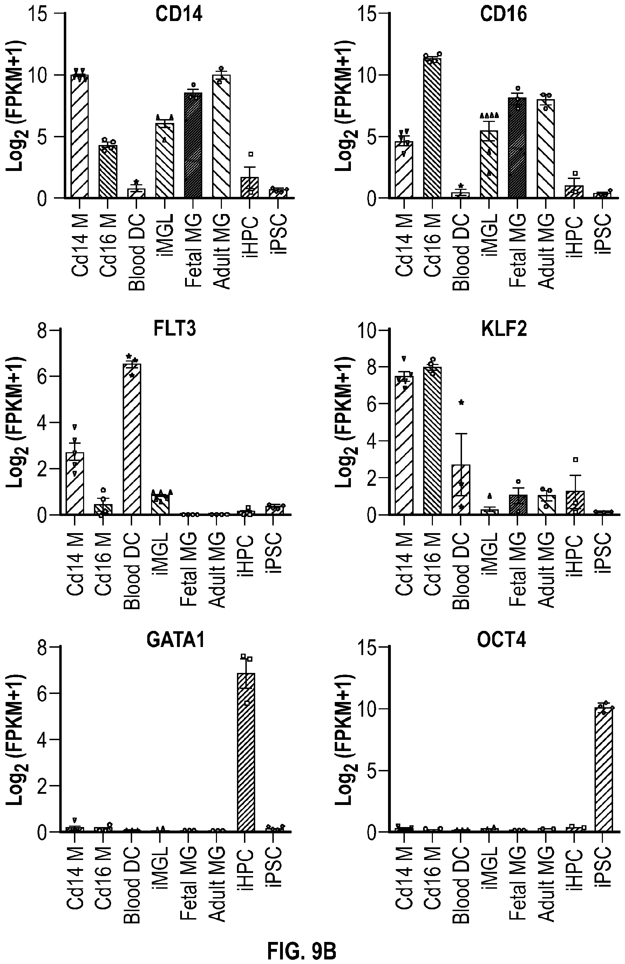

**FIG. 9B****.** Histograms of key genes found across different samples. CD14 and FCGR3A (also known as CD16) expressed in all myeloid cells including microglia, although enriched in CD14 M and CD16 M, respectively. As expected, FLT3 is highly expressed by Blood DCs and not in other cells and is barely detected in all three microglia groups. The monocyte/macrophage-specific transcription factor KLF2 was enriched in only CD14 M and CD16 M. Whereas GATA1 and OCT4 were only detected in iHPCs and iPSCs, respectively.

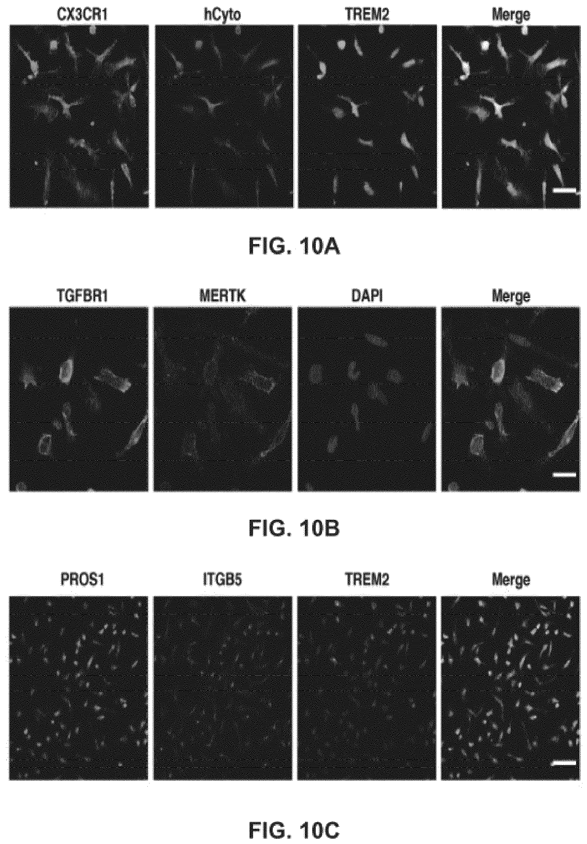

**FIG. 10A****.** Representative immunofluorescent images of iMGL expressing microglial markers CX3CR1 (left panel) and TREM 2 (middle, right panel). hCyto (middle, left panel) is a cytoplasmic marker. The merge panel (right panel) shows co-localization of CX3CR1, hCyto, and TREM2.
**FIG. 10B****.** Representative immunofluorescent images of iMGL expressing microlgial markers TGFBR1 (left panel), and MERTK (left, middle panel). Dapi (middle, right panel) is a nuclei marker. The merge panel (right panel) shows co-localization of TGFBR1, MERTK, and nuclei.
**FIG. 10C****.** Representative immunofluorescent images of iMGL expressing microglial markers PROS1 (left panel), ITGB5 (middle, left panel), TREM2 (middle, right panel). The merge panel (right panel) shows co-localization of PROS 1, ITGBS, and TREM2.

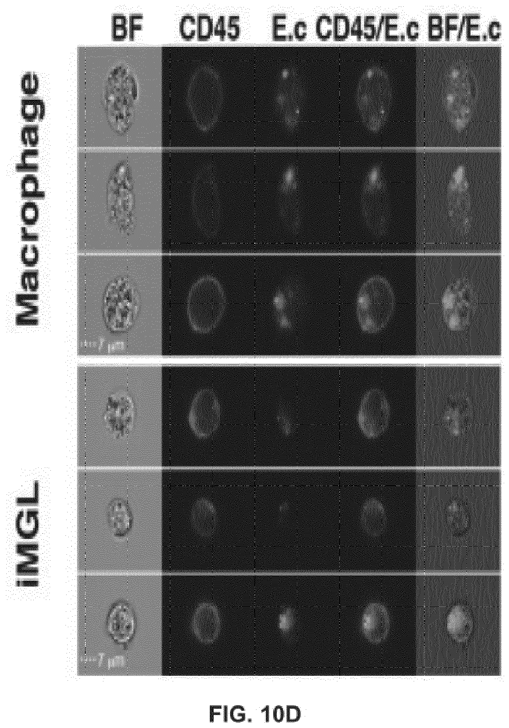

**FIG. 10D****.** Representative bright field and immunofluorescent images captured by Amnis Imagestream flow cytometer visualizing phagocytosis of E.c within macrophages (top) and iMGL (bottom).

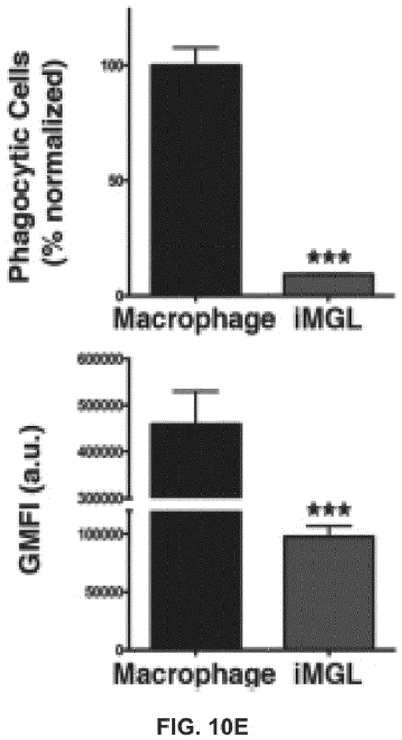

**FIG. 10E****.** Quantification of percent phagocytic cells (top) reveals that iMGLs (right bar) phagocytose E.c almost 10-fold less frequently than macrophages (left bar) as expected. The amount of E.c internalized by GMFI within phatocytic cells (bottom) further illustrates the greater phagocytic capacity of macrophages compared to iMGLs.

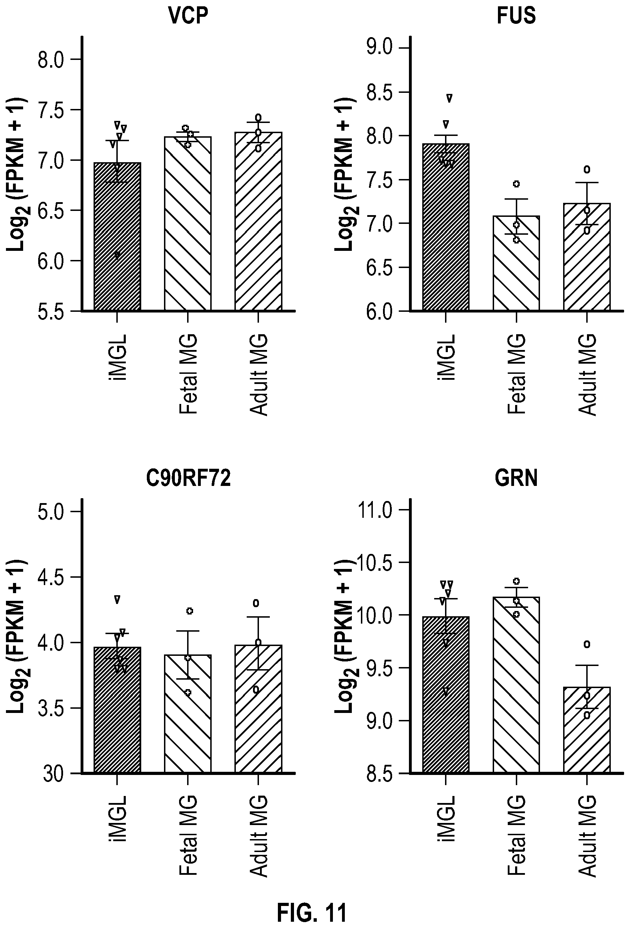

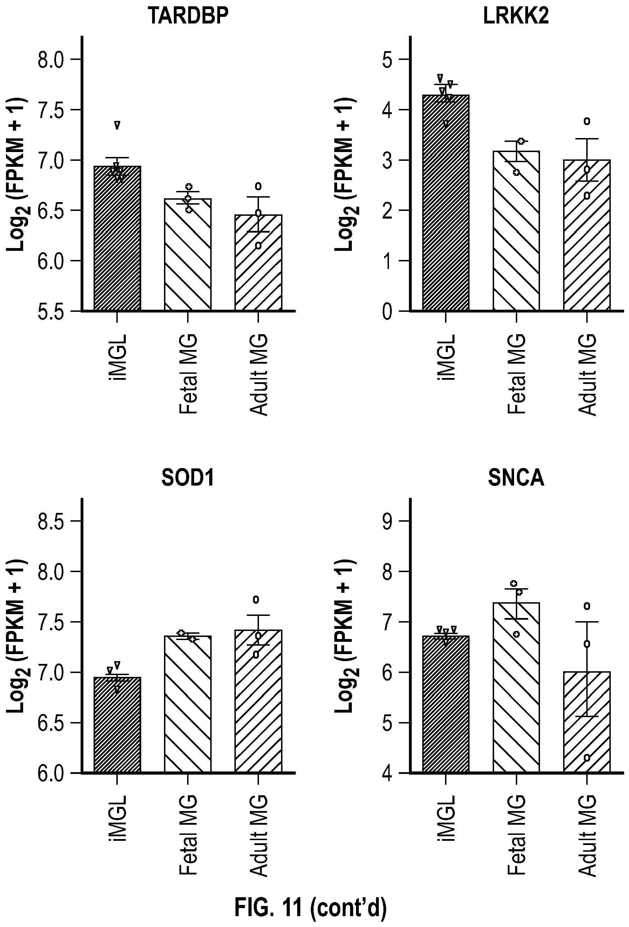

**FIG. 11****.** iMGLs express genes linked to Amylotrophic Lateral Sclerosis (ALS), Frontaltemporal Dementia (FTD), Parkinson's (PD), and Dementia with Lewy Bodies (DLB) and implicate microglia dysfunction. Bar graphs of genes implicated in neurodegenerative diseases that are detected in iMGL similarly to Fetal and Adult MG, and expressed as FPKM +1 followed by Log₂ transformation [Log₂ (FPKM +1)] presented as mean ± SEM. Similar to isolated human primary microglia, iMGLs express Valosin Containing Protein (VCP), (FUS), C9ORF72, proganulin (GRN), TDP-43 (TARDBP), LRRK2, Superoxide Dismutase (SOD), and synuclein (SNCA). Recent literature implicates microglia dysfunction related to mutations or loss of function of these genes playing a role in the pathogenesis of ALS (C9ORF72, SOD1, TARDBP, FUS), FTD (VCP, C9ORF72, GRN, TARDBP), PD (LRRK2, SNCA), and DLB (SNCA), suggesting the utility of iMGLs in studying the underlying mechanism of these genes in these neurological diseases.

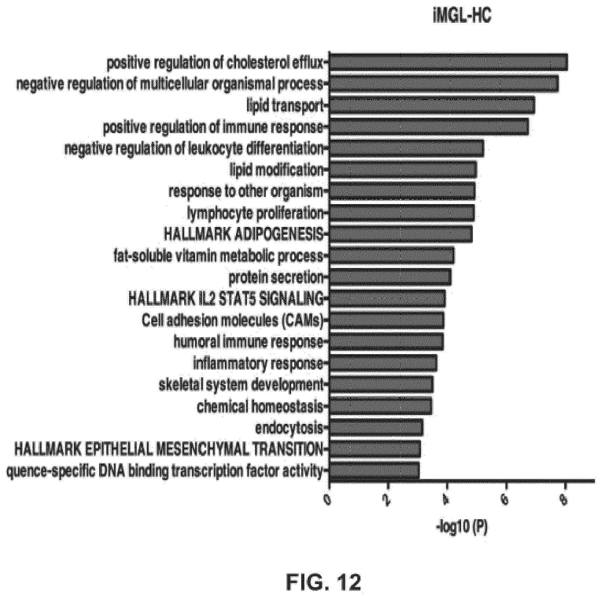

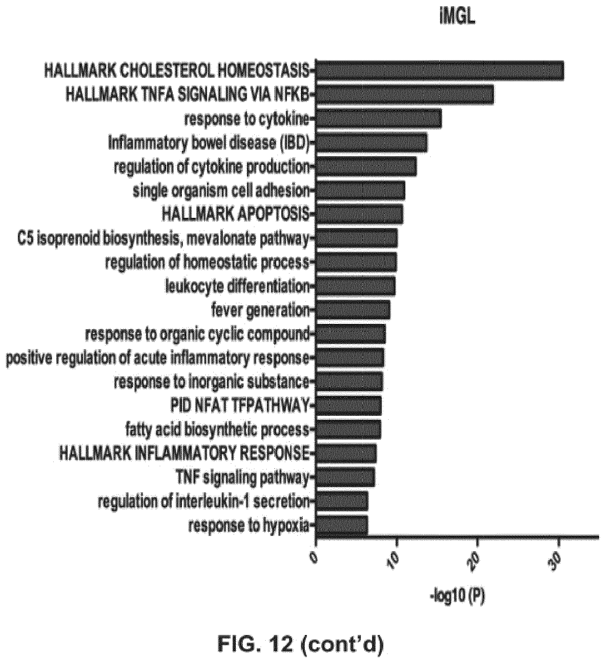

**FIG. 12****.** GO Terms from differential gene expression analysis of iMGLs cultured with hippocampal neurons. Gene expression profile of iMGLs co-cultured with rat-hippocampal neurons (iMGL-HC) was strengthened by soluble and insoluble factors present with neurons. Genes upregulated in iMGL-HC are associated with 20 statistically significant GO biological modules (iMGL-HC histogram) including positive cholesterol efflux, lipid transport, positive regulation of immune response, negative regulation of leukocyte differentiation and cell adhesion molecules. Cells cultured in absence of neurons had a complimentary gene profile with 20 statistically significant GO biological modules (iMGL histogram) were terms including hallmark cholesterol homeostasis, hallmark TNFα signaling via NF-κB, leukocyte differentiation and regulation of IL-1β secretion.

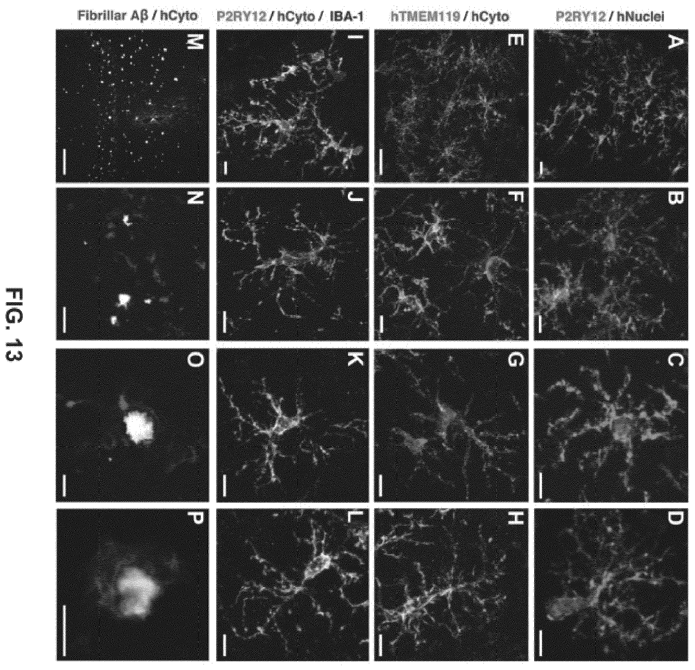

**FIGS. 13A-13P****.** iMGLs transplanted into the brains of either wild-type or AD transplant competent mice are similar to brain microglia. Within the brains of xenotransplantation compatible mice, transplanted iMGLs are ramified and interact with the neuronal environment. (**A-L**) After two months *in vivo,* iMGLs transplanted into mice display long-term viability with highly arborized processes resembling endogenous microglia found in the brain. (**A**) Transplanted iMGLs, labeled with P2ry12 (HPA HPA014518, Sigma) and human nuclei (ku80), exhibit long-term viability in mice. (**B-D**) At higher magnification, P2ry12 is highly expressed in iMGL arborized processes, both suggestive of homeostatic microglia surveying the brain environment. (**E-H**) Ramified iMGLs also express microglia-enriched Tmem119 recognized by a human-specific Tmem119 antibody (ab185333, Abcam, identified and validated in [Bennet et al, PNAS 2016]), and human cytoplasm maker SC121 (hCyto). (**I-L**) At higher magnification, representative iMGLs express P2ry12, hCyto, and Iba1 (ab5076, Abcam). (**M-P**) Human iMGLs (hCyto) transplanted into AD-immune-deficient mice (Marsh et al, PNAS 2016) interact with and phagocytose amyloid plaques. (**I-J**) Transplanted iMGLs extend projections and migrate to plaques. iMGLs fully encompass amyloid plaques (**O**) and begin to phagocytose amyloid (**P**). Scale bars; (**A,E,N**) = 30 µm, (**B-D, F-H, I-L, O,P**) = 10 µm, (**M**) = 300 µm. n=3 animals per study.

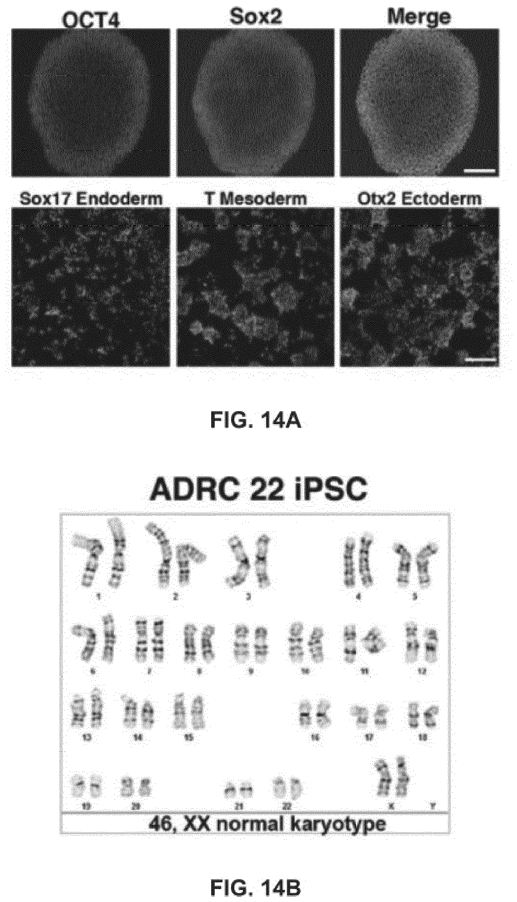

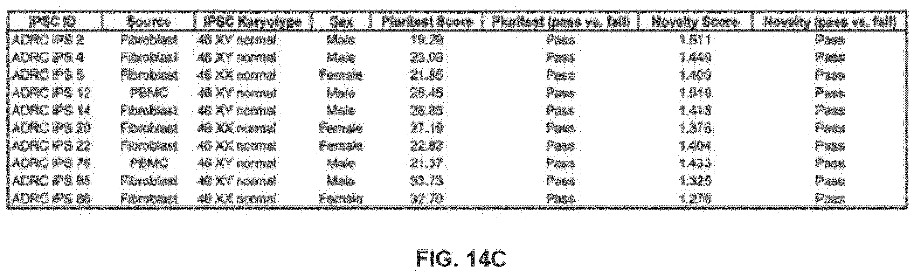

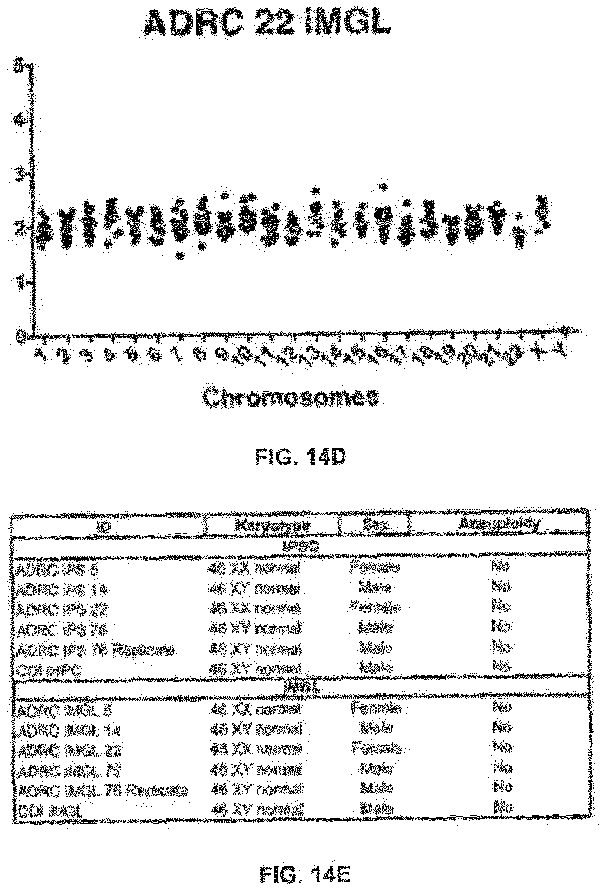

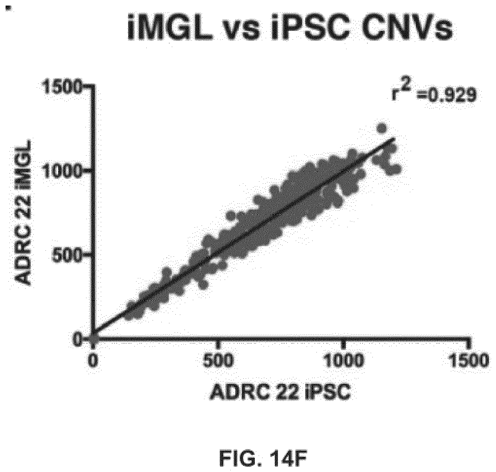

**FIGS. 14A-14F****.** Genomic stability of iPSCs and iMGLs. (**FIG. 14A**) Top: Representative fluorescent images of iPSCs expressing the pluripotent markers OCT4 and SOX2. Scale bar =300 µm. Bottom: Functional validation of pluripotency in iPSCs. Representative fluorescent images of iPSCs differentiated to endoderm, mesoderm and ectoderm and stained for Sox17, T (Brachyury), and Otx2 respectively to validate differentiation potential. Scale bar =200 µm. (FIGs. 14B-C) Karyotype and Pluritest scores indicate all iPS lines generated using Sendai virus and used in this study were karyotypically normal and pluripotent. The Pluritest is a microarray-based assessment of pluripotency based on iPS whole transcriptome analysis referenced to a library of functionally validated iPSCs (Muller, F.J. et al. 2011). (FIGs. 14D-E) Maintenance of genomic stability over the course of iMGL differentiation using pluripotent iPS or commercial hematopoietic progenitors. CNV assessment of differentiated iMGLs reveals genomic stability is maintained over the course of differentiation. (**FIG. D**) Representative Nanostring nCounterKaryotype results demonstrate that microglia derived from ADRC iPS line 22 do not inherit extrachromosomal DNA over the course of differentiation. (**FIG. 14E**) Quantification of the 338 probe sets across all 24 chromosomes do not reveal any chromosomal abnormalities (n=6). (**FIG. 14F**) Representative analysis of iMGL derived from its iPSC show strong CNV correlation (r²=0.929) showing sensitivity of assay and genomic stability of derived iMGLs.

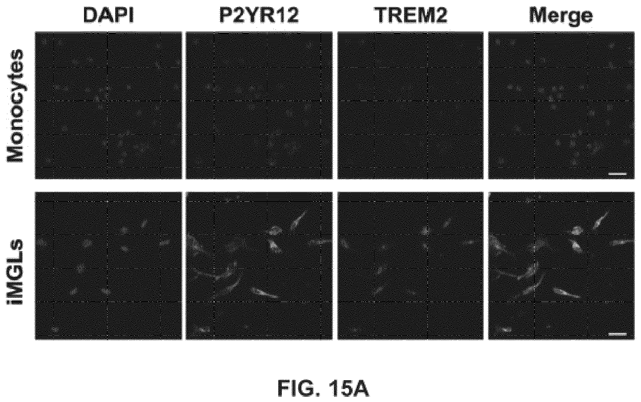

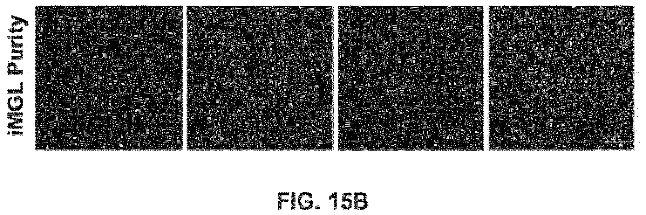

FIGS. 15A-B. Assessment of iMGL purity by P2RY12/TREM2 co-localization and flow cytometry characterization of monocytes, dendritic cells and commercial iHPCs. (**FIG. 15A**) Specificity assessment of rabbit anti-human P2ry12 (HPA014518, also recently validated by, and goat anti-human Trem2 (R&D, AF1828) in human monocytes and iMGLs. scale bar = 20 µm (**FIG. 15B**) Representative immunofluorescent images (from 5 representative lines) of iMGL purity by P2ry12/Trem2/DAPI co-localization. scale bar = 100µm

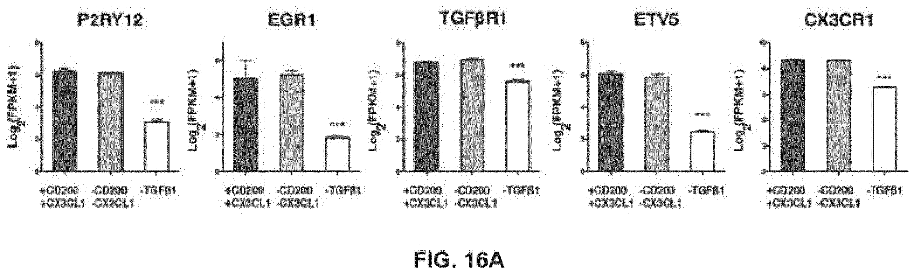

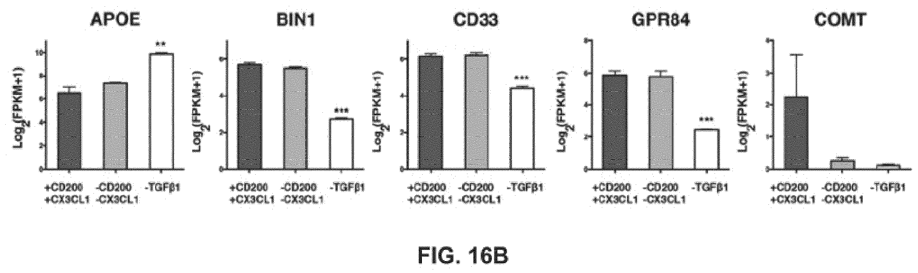

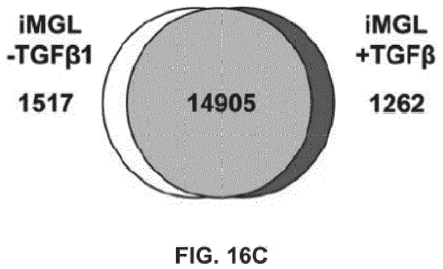

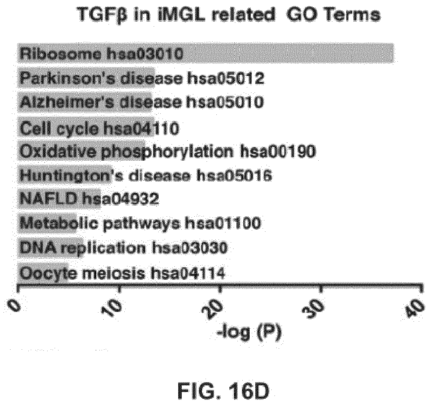

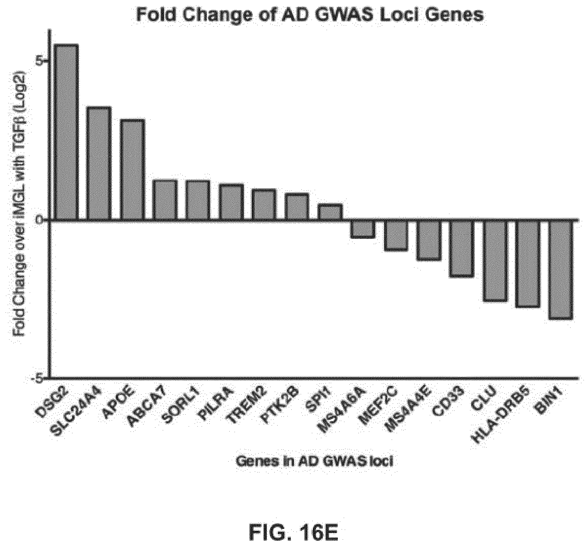

FIGS. 16A-E. TGFβ-1, CX3CL1, CD200 and their impact on key microglial genes are associated with modulating neuronal function and environment. (FIGs. 16A-B) TGFβ1 maintains core microglial genes. Withdrawal of TGFβ1 for 24 hours strongly influences microglial transcriptome. In agreement with mouse studies *in vivo,* TGFβ removal reduces expression of key microglia genes including surface receptors P2RY12, TGFβR1, and CX3CR1, while also reducing expression of microglia transcription factors EGR1 and ETVS. AD-associated pathway genes such as BIN1, CD33, and APOE are also influenced by the lack of TGFβ. Removal of CX3CL1 and CD200, does not change core microglia identity, but impacts state by influencing homeostatic gene expression such as, COMT, and APOE (FIG. 16B). (**FIG. 16C**) Differential gene expression analysis reveals that presence of TGFβ increases expression of 1262 genes in iMGLs, while lack of TGFβ reduces expression of 1517 genes, further supporting previous work highlighting the role of TGFβ in microglia development, gene signature, and function. (**FIG. 16D**) KEGG pathway analysis highlights that microglial-core genes, elevated with TGFβ, modulate pathways in CNS disease including Alzheimer's, Parkinson's, and Huntington's disease. (**FIG. 16E**) Fold change of AD GWAS loci genes over iMGL with TGFβ. Statistics reflect one-way ANOVA followed by Dunnett's multiple-comparison post-hoc test. * p<0.05**p<0.001, ***p<0.0001.

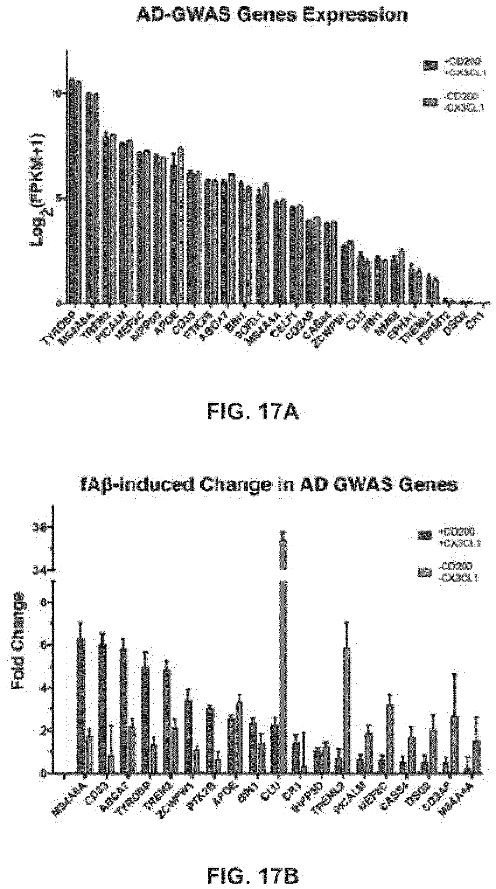

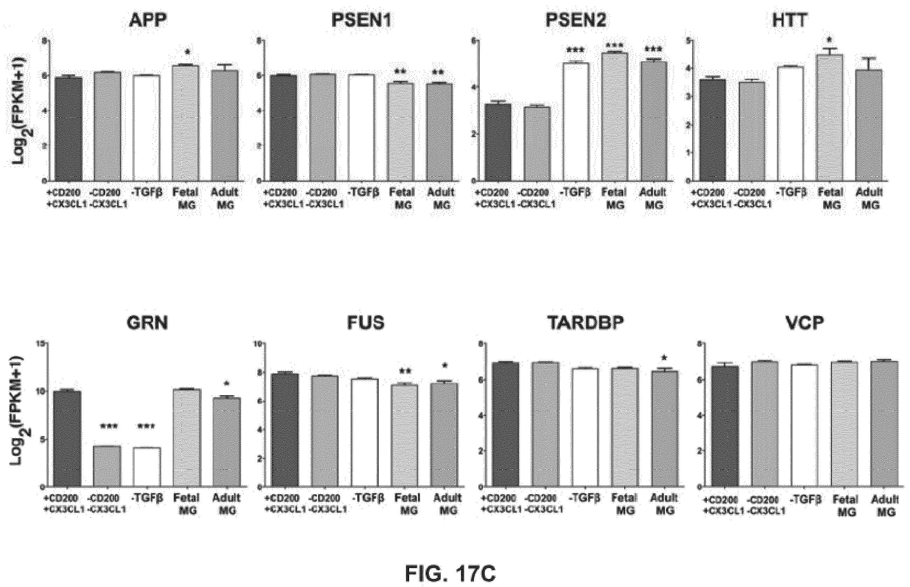

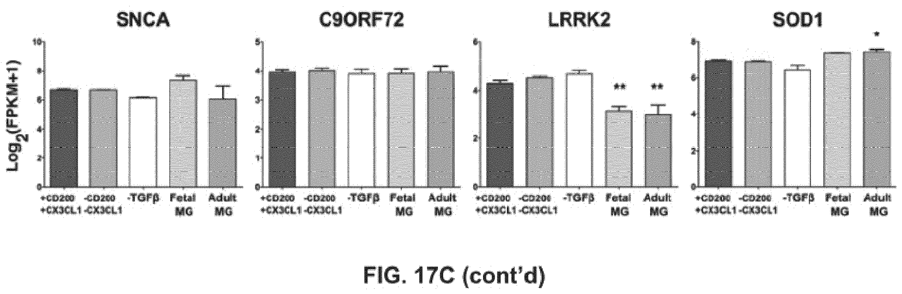

FIGS. 17A-C. Microglia AD-GWAS and other CNS-disease related genes can be studied using iMGLs. (FIGs. 17A-B) iMGL AD-related GWAS genes respond to fAβ differentially if primed with or without CD200 and CX3CL1. iMGL exposure to CNS factors, CD200 and CX3CL1, "primes" their response to fAβ by increasing expression of genes with functions implicated to modulate microglia inflammation and function in AD, like CD33, ABCA7, TYROBP, and TREM2. Stimulation with fAβ of iMGLs not exposed to CD200 or CX3CL1 results in increase expression of AD GWAS-related genes CLU and APOE, genes involved in response to misfolded proteins as well as survival and homeostasis. (**FIG. 17C**) Major neurodegenerative related genes, APP (AD), SCNA (PD) and HTT (HD), are expressed in iMGLs and primary microglia. iMGLs also express genes linked to Amylotrophic Lateral Sclerosis (ALS), Frontal-temporal Dementia (FTD), and Dementia with Lewy Bodies (DLB) and support previous studies implicating microglia dysfunction. Bar graphs of genes implicated in neurodegenerative diseases that are detected in iMGL similarly to Fetal and Adult MG, and expressed as Log₂ (FPKM +1) and presented as mean ± SEM. Like isolated human primary microglia, iMGLs express Valosin Containing Protein (VCP), FUS binding protein (FUS), proganulin (GRN), TDP-43 (TARDBP), LRRK2, and Superoxide Dismutase (SOD). Recent literature implicates microglia dysfunction related to mutations or loss of function of these genes playing a role in the pathogenesis of ALS (SOD1, TARDBP, FUS), FTD (VCP, GRN, TARDBP), PD (LRRK2, SNCA), and DLB (SNCA), suggesting the utility of iMGLs in studying the underlying mechanism of these genes in these neurological diseases. Statistics reflect one-way ANOVA followed by Tukey's multiple-comparison *post-hoc* test. * p<0.05**p<0.001, ***p<0.0001.

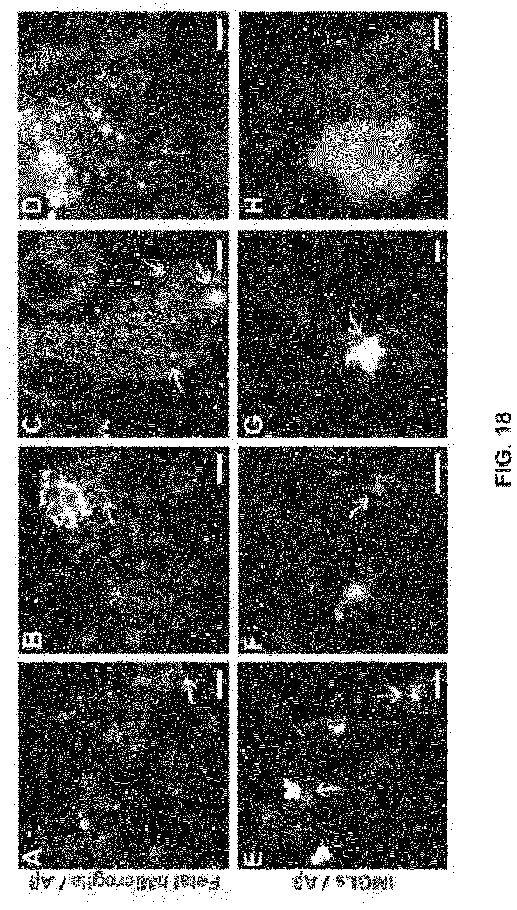

**FIG. 18****.** iPS-derived microglial cells engraft and phagocytose Aβ like human fetal microglia. (**A-D**) Human fetal microglia (hCyto) were transplanted into immune deficient AD mouse model, Rag5xfAD, and respond to beta-amyloid plaques. Fetal microglia are observed surrounding plaques (**C**), and phagocytosing Aβ (**C-D**). (**E-H**) Like fetal microglia, iMGLs (hCyto) surround and phagocytose beta-amyloid plaques. Scale bars (**A, B, E, F**) = 20 µm, (**C, D, H, G**) 5 µm.

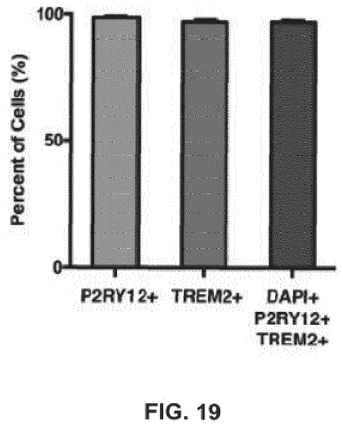

**FIG. 19****.** Assessment of percentage of (i) cells that express P2ry12 (left bar), (ii) cells that express TREM2 (middle bar), and (iii) cells that express P2ry12, TREM2, and DAPI (right bar) from FIG. 1H.

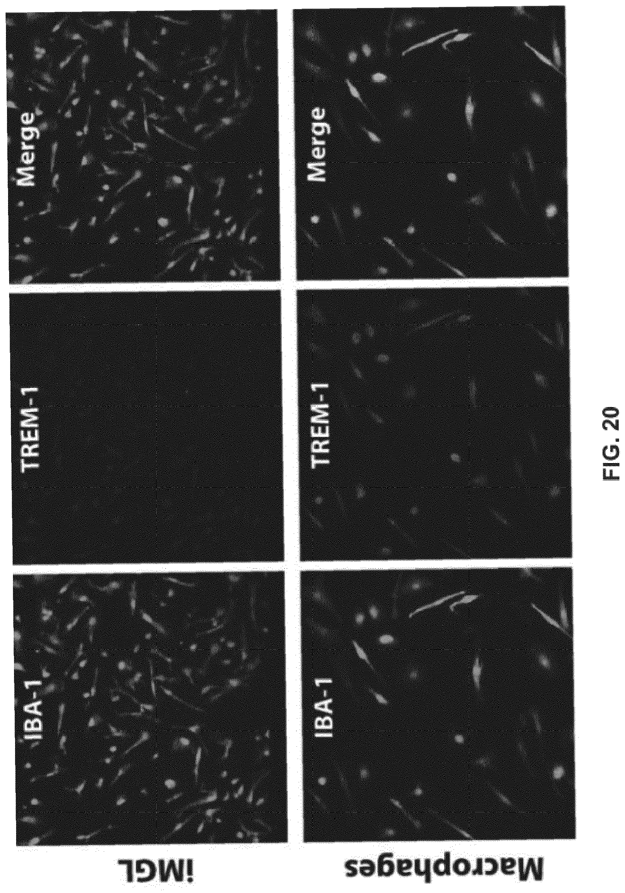

**FIG. 20****.** Expression of the peripheral macrophage marker TREM1 is low in iMGLs. Both iMGLs and macrophages express the myeloid protein, Iba-1 (left column of panels). However, TREM1 expression (middle column of panels) is highly-enriched in macrophages and distinguishes macrophage ontogeny from iMGLS, exemplified by low TREM1 expression that is typical of microglia within the CNS.

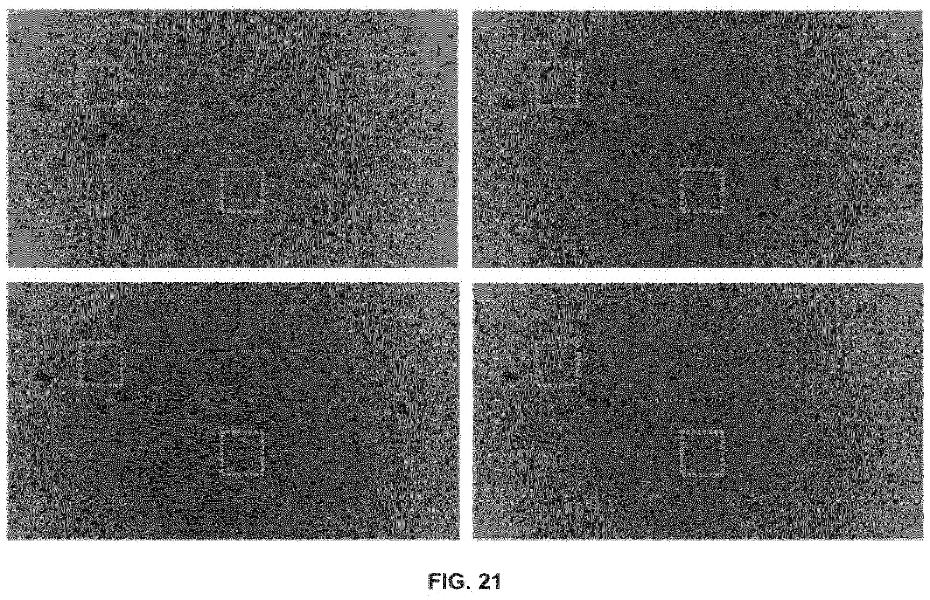

**FIG. 21****.** iMGLs are highly motile in vitro. Time-lapsed phase contrast images (over 24 hour) of iMGL motility in culture reveal that iMGLs (boxed) are highly mobile and survey their environment via projections showing that iMGL exhibit the ability to migrate, for example, in response to injury or stress.

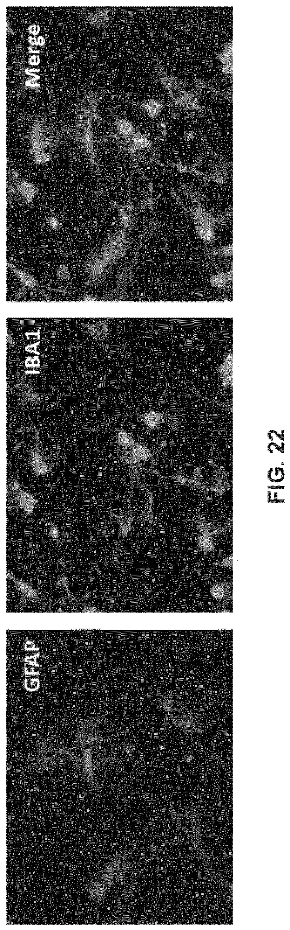

**FIG. 22****.** Co-culturing of iMGLs with iPSC-derived astrocytes leads to ramified iMGLs. When co-cultured with astrocytes that are distinguished by GFAP expression, (left panel). iMGLs (Iba-1, middle panel) ramify and extend projections in a 2D in vitro culture further indicating cues derived from a CNS environment can further educate iMGLs to adopt the in vivo phenotype of microglia found in the brain.

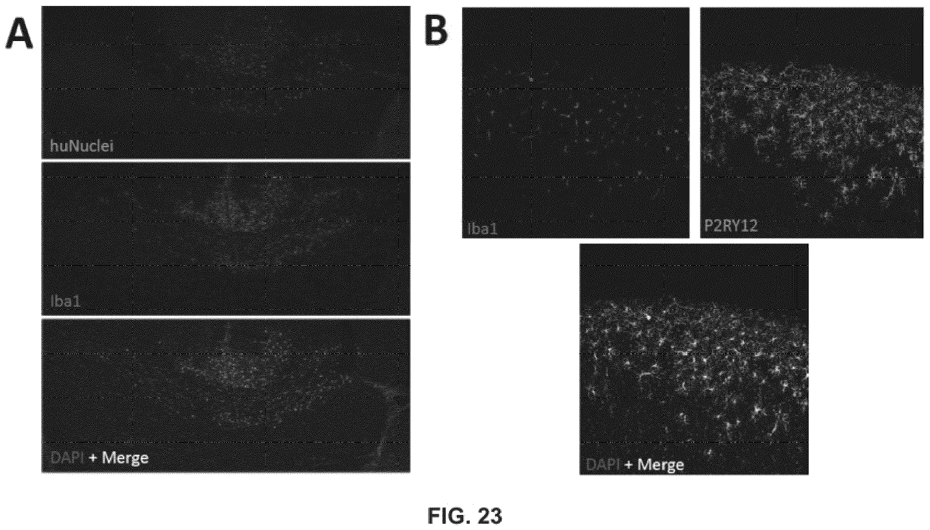

**FIGS. 23A-23B****.** Humanization of mouse brains using human hematopoietic progenitors. iHPCs exhibit the potential to differentiate into microglia, the resident macrophage of the CNS, and out-compete endogenous mouse microglia in MITRG mice. **(A)** Confocal microscopy reveals successful engraftment of iHPCs that are detected with the human nuclei specific antibody (top panel in 23A) and express the myeloid marker, Iba-1 (middle panel in 23A). **(B)** The transplanted iHPCs differentiate into microglia that express P2ry12 (top right panel in 23B) and due to the humanization of MITRG expressing the human CSF1, human cells out-compete the endogenous mouse cells.

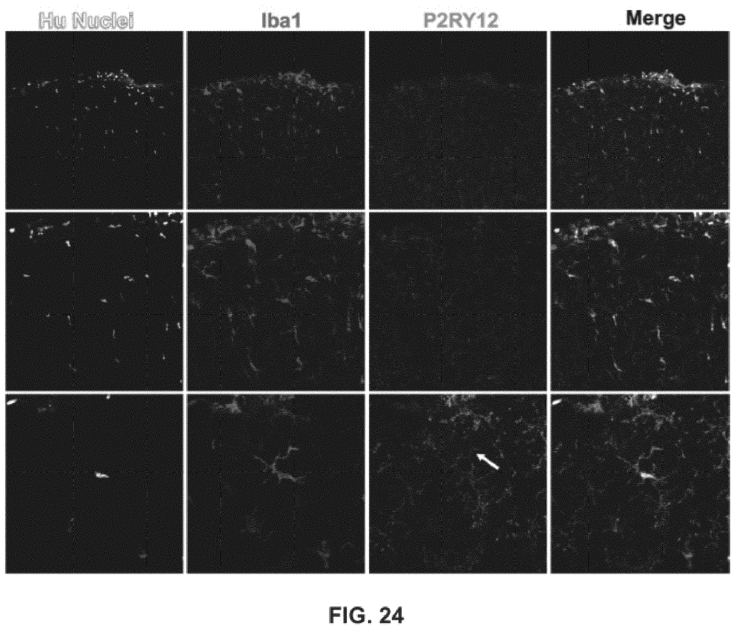

**FIG. 24****.** The microglia-specific expression of P2ry12 is detected after only two weeks in engrafted iHPCs. Two weeks after iHPC transplantation, cells that survive transplantation which are labeled with the human nuclei-specific antibody (left column of panels), begin to express Iba-1 (mid-left column of panels), indicative of cell with a myeloid origin. Furthermore, P2ry12, a microglia-specific gene highly expressed in homeostatic microglia can be detected (mid-right column of panels; arrow).

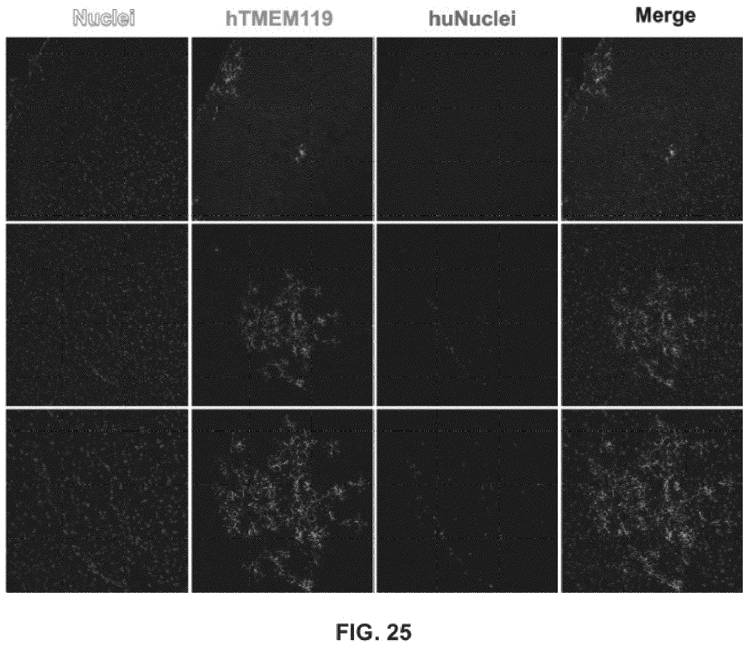

**FIG. 25****.** Transplanted hematopoeitic progenitors differentiate into microglia and express TMEM119 in the mouse brain. After two months, HPCs that have engrafted into mouse brain express TMEM119 (mid-left column of panels), a microglia marker that is expressed prominently within the highly ramified processes of maturing microglia (Bennet et al., 2016). The human specificity of the TMEM119 antibody is demonstrated by the co-localization of TMEM119 with an antibody specific for human nuclei (mid-right column of panels) but not all nuclei (DAPI, left column of panels).

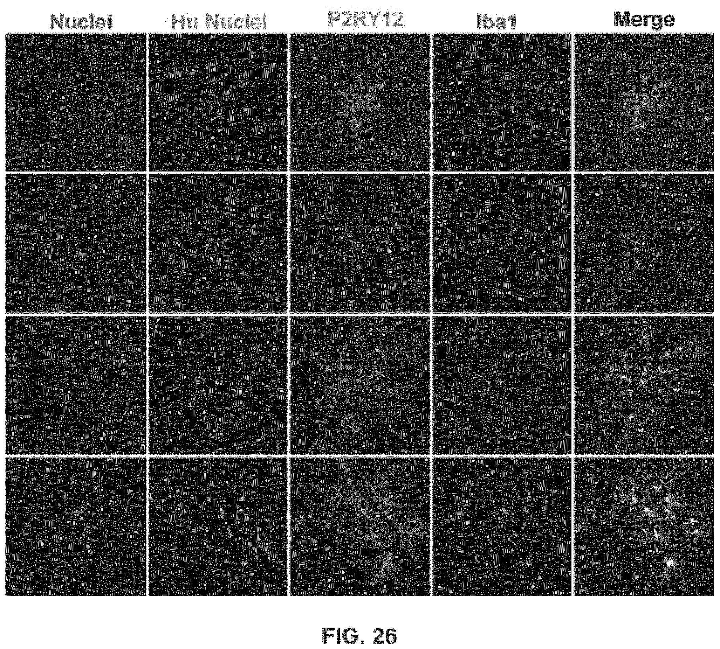

**FIG. 26****.** Transplanted human cells express the homeostatic microglia marker, P2ry12. Engrafted iMGLs which are distinguished by a human-specific nuclei marker (mid-left column of panels) can be differentiated from endogenous mouse cell nuclei that only stain with the non-specific nuclei stain DAPI (left column of panels). Engrafted iMGLs express the homeostatic microglia marker P2ry12 (middle column of panels) which highlight the extended ramified processes that is typical of microglia in vivo. This is in contrast to the commonly used microglia marker, Iba-1, which exhibits a cytosolic cellular distribution in microglia (mid-right column of panels).

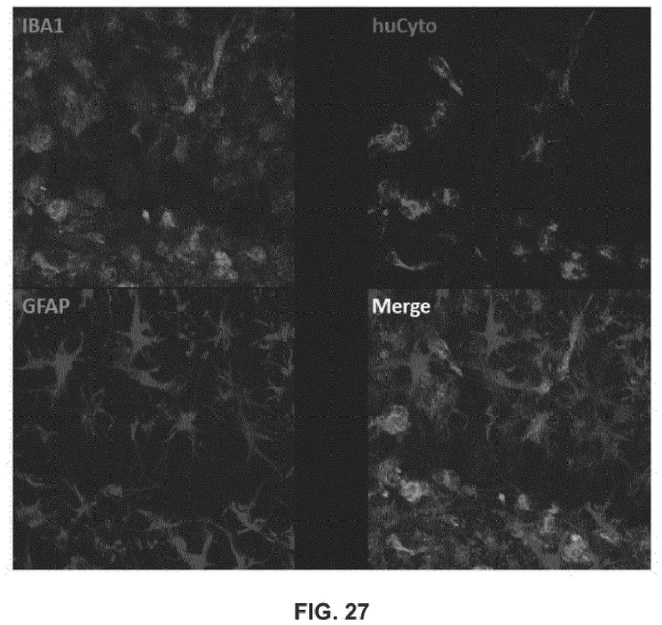

**FIG. 27****.** iMGLs can be utilized to study astrocyte-microglia crosstalk in vivo. Transplanted iMGLs (Iba-1, top-left panel) are observed interacting with endogenous mouse astrocytes in vivo (GFAP, bottom-left panel). Recent studies (Liddelow et al., 2017) implicate astrocyte-microglia crosstalk that influences the immune response in the CNS.

### DETAILED DESCRIPTION

Microglia are the innate immune cells of the CNS and play important roles in synaptic plasticity, neurogenesis, homeostatic functions and immune activity. Microglia also play a critical role in neurological disorders, including AD, highlighting the need to improve our understanding of their function in both health and disease. Yet, studying human microglia is challenging because of the rarity and difficulty in acquiring primary cells from human fetal or adult CNS tissue. Therefore, there is a pressing need to develop a renewable source of human microglia, such as from pluripotent stem cells (PSCs), including induced pluripotent stem cells (iPSCs) and embryonic stem cells (ESCs).

The challenges present in generating microglia from iPSCs are due to their unique developmental origin. Elegant lineage tracing studies show that microglia originate from yolk sac erythromyeloid progenitors (EMP) generated during primitive hematopoiesis. EMPs further develop to early primitive macrophages that migrate into the developing neural tube, and become microglial progenitors. Microglia progenitors then mature and develop ramified processes used to survey their environment, facilitate CNS development, modulate synaptic plasticity, and respond to CNS injury and pathology.

The generation of patient-derived iPSCs has facilitated new opportunities to examine the relationships between genetic risk factors and disease. Recently, genome wide association studies (GWAS) have identified several genes expressed by microglia that are associated with the risk of developing late-onset AD (LOAD). The role of these genes in microglial function and AD are just beginning to be examined in mouse models, but the generation of human microglia-like cells as described herein allows for the interrogation of human-specific genes that cannot be modeled in mice.

In AD, microglia cluster around beta-amyloid plaques highlighting their inefficacy in clearing beta-amyloid. Microglia are also implicated in the neuroinflammatory component of AD etiology, including cytokine/chemokine secretion, which exacerbate disease pathology. Furthermore, AD GWAS genes like TREM2 and CD33 are influenced by AD pathology and likely play a role in AD progression. Microglia are also the primary modulators of brain development, neuronal homeostasis, and numerous neurological disorders. Thus, there is a pressing need to further our understanding of human microglia and the influence of both pathology and disease-associated genes on microglial function.

Some of the embodiments described herein provide methods for the effective and robust generation of human iPSC microglial-like cells (iMGLs) that resemble fetal and adult microglia. These methods produce iMGLs that are useful in investigating neurological diseases like AD. In some of the embodiments described herein, microglial-like cells (iMGL) are differentiated from iPSCs to study their function in neurological diseases, such as Alzheimer's disease (AD).

The iMGLs described herein, develop *in vitro* similarly to microglia *in vivo.* Whole transcriptome analysis demonstrates that they are highly similar to adult and fetal human microglia. Functional assessment of these iMGLs, reveal that they secrete cytokines in response to inflammatory stimuli, migrate and undergo calcium transients, and robustly phagocytose CNS substrates similar to adult/fetal microglia.

These iMGLs can be used to (i) examine the effects of fibrillar Aβ and brain-derived tau oligomers on AD-related gene expression and (ii) identify mechanisms involved in synaptic pruning, among other uses. Further, the iMGLs can be used in high-throughput studies of microglial function, providing important new insight into human neurological disease.

The following sections provide various embodiments of methods to produce iMGLs and various embodiments of the structure and function of the iMGLs. In addition, methods of using iMGLs are also provided. Also provided are non-limiting detailed explanations of the methods.

### Methods of making iMGLs:

In some embodiments, methods of producing human microglial-like cells (iMGLs) from pluripotent stem cells (PSCs) are provided. In some embodiments, the method comprises the steps of: (i) differentiating PSCs using a media supplemented with hematopoietic differentiation factors to produce induced hematopoietic progenitor cells (iHPCs); (ii) isolating CD43⁺ iHPCs; (iii) differentiating the CD43⁺ iHPCs into human microglial-like cells (iMGLs) using a microglial differentiating media; and (iv) maturing the iMGLs. In some embodiments, HPC generation technology allows for collecting media enriched with precursors and carried to (iii) without isolating CD43⁺ iHPCs.

In some embodiments, the method comprises the steps: (i) differentiating PSCs using a media supplemented with hematopoietic differentiation factors; and (ii) differentiating the CD43⁺ iHPCs into iMGLs using a microglial differentiating media.

Some embodiments of the methods and compositions provided herein relate to a method of producing a human iMGL from a cell of a first type comprising the steps of: (i) differentiating a cell of a first type into an iHPC; and (ii) differentiating the iHPC to produce an iMGL. In some embodiments, the cell of a first type is not a PSC or an ESC.

In some embodiments, the PSCs are not derived from embryoid bodies. In some embodiments, the PSCs include single-cell PSCs. In some embodiments, the PSCs are not CD43⁺ before differentiation. In some embodiments, the PSCs are not CD34⁺ before differentiation. In some embodiments, the PSCs are not CD31⁺. In some embodiments, the PSCs are not CD45⁺ before differentiation.

In some embodiments, the PSCs are or include induced PSCs (iPSCs). In some embodiments, the PSCs are or include embryonic stem cells (ESCs). In some embodiments, the PSCs are mammalian PSCs. In some embodiments, the PSCs are human PSCs. In some embodiments, the PSCs are mouse PSCs.

### Differentiating single cell PSCs

In some embodiments, differentiating PSCs to produce iHPCs comprises an incubation period that is between 5 and 15 days. For example, the incubation period is 5 days, 6 days, 7 days, 8 days, 9 days, 10 days, 11 days, 12 days, 13 days, 14 days, or 15 days. In some embodiments, the incubation period is 10 days. In some embodiments, the oxygen percentage that the PSCs are exposed to varies the 10-day period. In some embodiments, during the incubation period the iPSCs are incubated in a hypoxic or normoxic environment. In some embodiments, during days 1 through 10 of the incubation period the PSCs are incubated in a hypoxic or normoxic environment. In some embodiments, during the first part of the 10 day period, the PSCs will be exposed to an oxygen environment between 3% and 7%. In some embodiments, the first part of the 10 day period is 4 days (days 1-4) and the oxygen environment is 5%. In some embodiments, during the second part of the 10 day period, the PSCs will be exposed to an oxygen environment between 15% and 25%. In some embodiments, the second part of the 10 day period is 6 days (days 5-10) and the oxygen environment to which the PSCs are exposed is 20%. In some embodiments, differentiating PSCs to produce iHPCs comprises an incubation period that is between 3 and 21 days. In some embodiments, the incubation period is up to 28 days. In some embodiments, the incubation period is over 28 days. In some embodiments, the incubation period is less than 3 days.

In some embodiments, hematopoietic differentiation factors used to differentiate PSCs comprise FGF2, BMP4, Activin A, LiCl, VEGF, TPO, SCF, IL3, and IL6. The media will comprise any one or more of these factors in any combination. In some embodiments, the PSCs are incubated in different media throughout the incubation period of the PSC differentiation step. In some embodiments, a 10 day incubation period is provided wherein, during day 1 the media comprises FGF2, BMP4, Activin A, and LiCl, during days 3 and 4 the media comprises FGF2 and VEGF, and during days 5 through 10 the media comprises FGF2, VEGF, TPO, SCF, IL3, and IL6. Some embodiments relate to a medium comprising any one or a combination of the factors FGF2, BMP4, Activin A, LiCl, VEGF, TPO, SCF, IL3, and IL6. Some embodiments relate to a medium comprising any one or a combination of the factors FGF2, BMP4, Activin A, and LiCl. Some embodiments relate to a medium comprising any one or a combination of the factors FGF2 and VEGF. Some embodiments relate to a medium comprising any one or a combination of the factors FGF2, VEGF, TPO, SCF, IL3, and IL6.

In some embodiments, the concentration of each of the factors FGF2, BMP4, VEGF, TPO, SCF, IL-3, and IL6 in the media is between 5ng/ml and 100ng/ml. In some embodiments, the concentration of each of the factors FGF2, BMP4, VEGF, TPO, SCF, IL-3, and IL6 in the media is between 30ng/ml and 70ng/ml or between 40ng/ml and 60 ng/ml. In some embodiments, the concentration of each of the factors FGF2, BMP4, VEGF, TPO, SCF, IL-3, and IL6 in the media is 50ng/ml. In some embodiments, the concentration of Activin A in the media is between 9 ng/ml and 16 ng/ml or between 11 ng/ml and 14 ng/ml. In some embodiments, the concentration of Activin A in the media is 12.5ng/ml. In some embodiments, the concentration of LiCl in the media is between 1nM and 3nM. In some embodiments, the concentration of LiCl in the media is between 1mM and 3mM. In some embodiments, the concentration of LiCL in the media is 2mM.

### Isolation of iHPCs

Any method known in the art is used to isolate iHPCs or CD43⁺ iHPCs. In some embodiments, the method used to isolate iHPCs or CD43⁺ iHPCs is FACS. In some embodiments, the isolation step comprises selecting for the CD43⁺ marker. In some embodiments, a marker other than CD43⁺ is used to isolate HPCs. In some embodiments, the isolation step comprises selecting for CD34⁺ cells. In some embodiments, the isolation step comprises selecting for CD31⁺ cells or CD45⁺ cells. In some embodiments, the isolation step comprises selecting for another marker known to identify iHPCs.

In some embodiments, isolating iHPCs results in isolation of iHPCs that are greater than 80% pure, for example, greater than 90%. In some embodiments, isolating CD43⁺ iHPCs results in isolation of CD43⁺ iHPCs that are greater than 80% pure, for example, greater than 90%.

### Differentiating iHPCs into iMGLs

Any method known in the art to mature microglia cells may be used to mature iMGLs.

In some embodiments, differentiating CD43⁺ iHPCs into iMGLS comprises an incubation period of between 20 and 30 days. In some embodiments, the incubation period is 25 days.

In some embodiments, the media used to differentiate the iHPCs into iMGLs comprises any one or combination of the factors CSF-1, IL-34, and TGFβ1. In some embodiments, the media comprises all of the factors CSF-1, IL-34, and TGFβ1. In some embodiments, the concentration of the CSF-1 in the media is between 5ng/ml and 50ng/ml. In some embodiments, the concentration of the CSF-1 in the media is between 15ng/ml and 35ng/ml or between 20ng/ml and 30ng/ml. In some embodiments, the concentration of CSF-1 in the media is 25ng/ml. In some embodiments, the concentration of the IL-34 in the media is between 25ng/ml and 125ng/ml. In some embodiments, the concentration of the IL-34 in the media is between 80ng/ml and 120ng/ml or between 90ng/ml and 110ng/ml. In some embodiments, the concentration of IL-34 in the media is 100ng/ml. In some embodiments, the concentration of the TFGβ-1 in the media is between 2.5ng/ml and 100ng/ml. In some embodiments, the concentration of the TFGβ-1 in the media is between 30ng/ml and 70ng/ml or between 40 ng/ml and 60ng/ml. In some embodiments, the concentration of TGFβ-1 in the media is 50ng/ml. Some embodiments relate to a medium comprising any one or a combination of the factors CSF-1, IL-34, and TGFβ1.

In some embodiments, the media used to differentiate the iHPCs into iMGLs comprises TFGβ-2. In some embodiments, the concentration of the TFGβ-2 in the media is between 2.5ng/ml and 100ng/ml. In some embodiments, the concentration of the TFGβ-2 in the media is between 30ng/ml and 70ng/ml or between 40 ng/ml and 60ng/ml. In some embodiments, the concentration of TGFβ-2 in the media is 50ng/ml.

In some embodiments, the media used to differentiate the iHPCs into iMGLs comprises a TFGβ mimetic. Examples of TGFβ mimetics include IDE-1 and IDE-2. In some embodiments, the TFGβ mimetic has one or more off-target effects and/or affects a SOX signaling pathway. In some embodiments, the concentration of the TFGβ mimetic in the media is between 2.5ng/ml and 100ng/ml. In some embodiments, the concentration of the TFGβ mimetic in the media is between 30ng/ml and 70ng/ml or between 40 ng/ml and 60ng/ml. In some embodiments, the TGFβ mimetic activates a TGFβ signaling pathway.

In some embodiments, the media used to differentiate iHPCs into iMGLs is serum-free media.

### Maturation of iMGLs

In some embodiments, maturing the iMGLs comprises an incubation period between 1 and 5 days. In some embodiments, the incubation period for maturing the iMGLs is 3 days.

In some embodiments, maturation step comprises incubating the iMGLs in media comprising either or both of CD200 and CX3CL1. In some embodiments, the CD200 is human recombinant CD200 and the CX3CL1 is human recombinant CX3CL1.

In some embodiments, the concentration of each of CD200 and CX3CL1 in the media is between 1ng/ml and 1 g/ml In some embodiments, the concentration of each of CD200 and CX3CL1 in the media is between 80ng/ml and 120ng/ml, or between 90ng/ml and 110 ng/ml. In some embodiments, the concentration of each of CD200 and CX3CL1 is 100ng/ml.

### Characteristics of the iMGLs produced

In some embodiments, the iMGLs produced using the methods described herein results in a pure population of iMGLs that is between 70% pure and 100% pure. In some embodiments, the iMGLs produced using the methods described herein results in a pure population of iMGLs that is between 80% pure and 100% pure. For example, the population of iMGLs will be 80% pure, 81% pure, 82% pure, 83% pure, 84% pure, 85% pure, 86% pure, 87% pure, 88% pure, 89% pure, 90% pure, 91% pure, 92% pure, 93% pure, 94% pure, 95% pure, or 96% pure, 97%, 98%, 99%, 99%, or 100%. In some embodiments, the population of iMGLs produced is greater than 96%.

Assessing the purity of the iMGLs is accomplished through utilization of any method known in the art of determining the purity of microglial cells. In some embodiments, the purity levels are assessed by the expression and/or co-localization of the factors P2RY12 and TREM12. In some embodiments, the purity levels are assessed by the expression and/or co-localization of Trem2, Iba1, and/or Pu1.

The iMGLs produced by any of the methods described herein will express any factor or any combination of factors that a typical microglial cell expresses. In some embodiments, the iMGLS produced are c-kit⁻/CD45⁺. In some embodiments, the c-kit⁻/CD45⁺ iMGLs are detected using flow cytometry, immunofluorescence microscopy, qPCR, RNA-seq, or proteinomics. In some embodiments, other cell types are detected using flow cytometry, immunofluorescence microscopy, qPCR, RNA-seq, or proteinomics. In some embodiments, the iMGLs produced comprise two separate populations of iMGLs: (1) CD45⁺/CX3CR1⁻ and (2) CD45⁺/CX3CR1⁺. In some embodiments, the iMGLs produced are CD43⁺, CD235a⁺, or CD41⁺. In some embodiments, the iMGLs produced are CD43⁺/CD235a⁺/CD41⁺.

Any of the methods for producing iMGLs described herein will result in a differentiation step of the CD43⁺ iHPCs in which there is a commitment of cells to a microglial lineage early during the differentiation process. In some embodiments, iMGLs that are c-kit⁻/CD45⁺ are detected on day 14 of the incubation period used for differentiating CD43⁺ iHPCs into iMGLs. Determining whether there is a commitment to an iMGL lineage is done through testing for expression of any factors that are known to be markers for cells that are committed to a microglia fate. In some embodiments, determining whether the cells are committed to an iMGL lineage is determined through assessing expression of the transcription factor PU.1 and/or the microglia-enriched protein Trem2. In some embodiments, the cell markers are detected using flow cytometry, immunofluorescence microscopy, qPCR, RNA-seq, or proteinomics.

In some embodiments, a method of producing iMGLs from induced PSCS is provided that comprises the steps: (i) differentiating PSCs into induced hematopoietic progenitor cells (iHPCs) and (ii) differentiating iHPCs to produce iMGLs. In some embodiments, this method further comprises step (iii) of maturing the iMGLS produced from step (ii). In some embodiments, the PSCs include induced PSCs (iPSCs) or embryonic stem cells (ESCs). In some embodiments, the PSCs are mammalian PSCs, such as from a human or a mouse.

In some embodiments, TRIM14, CABLES1, MMP2, SIGLEC 11 and 12, MITF, and/or SLC2A5 mRNA and/or protein expression is enriched in the produced iMGLs. In some embodiments, COMT, EGR2, EGR3, and/or FFAR2 mRNA and/or protein expression is enriched in the produced iMGLs.

### Compositions of iMGLs

### Gene expression of iMGLs

In some embodiments, iMGLs are provided that express a specific gene profile. Any of the iMGLs described herein will comprise a gene expression profile similar to microglia cells. In some embodiments, any of the compositions of iMGLs described herein comprise expression of any of the following genes: RUNX1, PU.1, CSF1FR, CX3CR1, TGFBR1, RSG10, GAS6, PROS1, P2RY12, GPR34, C1Q, CR3, CABLES 1, BHLHE41, TREM2, ITAM, APOE, SLCO2B1, SLC7A8, PPARD, C9orf72, GRN, LRRK2, TARDBP, and CRYBB1. Any of the iMGLs disclosed herein will comprise expression of any of these genes in any combination.

RUNX1, SPI1, CSF1FR, CX3CR1, TGFBR1, RSG10, GAS6, MERTK, PSEN2, PROS1, P2RY12, P2RY13, GPR34, C1Q, CR3, CABLES1, BHLHE41, TREM2, TYROBP, ITGAM, APOE, SLCO2B1, SLC7A8, PPARD, TMEM119, GPR56, C9orf72, GRN, LRRK2, TARDBP, and CRYBB1

In some embodiments, in any of the compositions of iMGLs described herein TREM2 and P2RY12 are co-expressed. In some embodiments, any of the compositions of iMGLs described herein do not express any one or more of the genes KLF2, TREM1, MPT, ITGAL, and ADGRES.

### Functional properties of the iMGLs and methods of using iMGLs

### Chemokine secretion

Any of the iMGLs described herein will secrete a chemokine profile similar to microglia cells in response to any stimuli known in the art to stimulate chemokines in microglia cells. In some embodiments, the chemokines secreted are any one or more of TNFα, CCL2, CCL4, and CXCL10, in any combination, and are secreted in response to stimulation by lipopolysaccharide, IFNγ, or IL-1β.

In some embodiments, a method of stimulating chemokine secretion from iMGLs is provided. The method comprises (i) treating iMGLs with any factor known in the art to stimulate cytokine secretion in microglia cells and (ii) secreting chemokines from the microglia cells. In some embodiments, the factor used to treat the iMGLs is lipopolysaccharide, INFγ, or IL-1β. The chemokines secreted may comprise any chemokines known to be secreted by microglia cells. In some embodiments, the chemokines secreted comprise any one or more of the following: TNFα, CCL2, CCL4, and CXCL10.

### Migration and calcium transients

Any of the iMGLS described herein will migrate in response to ADP treatment and/or ADP treatment will trigger calcium transients. In some embodiments, inhibition of P2ry12 negates ADP mediated migration of iMGLs and/or ADP mediated calcium transients. In some embodiments, the inhibition of P2ry12 occurs through the inhibitor PSB0739.

In some embodiments, a method of migrating iMGLs is provided. The method comprises (i) treating iMGLs with ADP and (ii) migrating the iMGLs. In some embodiments, a method of producing calcium transients is provided. The method comprises (i) treating the iMGLs with ADP and (ii) producing calcium transients in iMGLs.

### Phagocytoses by iMGLs

Any of the iMGLs described herein are capable of phagocytosis. Any of the iMGLs described herein are able to phagocytose any factor known in the art that microglia can phagocytose. In some embodiments, the factor(s) that iMGLs phagocytose comprise any one or more of the following: Aβ, fluorescently labeled Aβ, tau, and pHrodo-labeled brain-derived tau oligomers.

In some embodiments, a method of iMGLs phagocytoses is provided. The method comprises (i) exposing iMGLs to any one or more of the compounds: Aβ, fluorescently labeled Aβ, tau, and pHrodo-labeled brain-derived tau oligomers and (ii) phagocytosing the compound.

Any of the iMGLs provided herein are capable of phagocytosing human synaptosomes (hS). In some embodiments, a method of iMGLs phagocytosing hS is provided. The method comprises (i) exposing the iMGLS to hS and (ii) phagocytosing hS. In some embodiments, hS are fluorescently labeled.

### Utility of iMGLS in studying Alzheimer's disease

Any of the iMGLs described herein are capable of regulating gene expression in response to different stimuli. In some embodiments, the stimuli comprise neurons, for example, rat-hippocampal neurons. In some embodiments, any of the iMGLs described herein are capable of differentially regulating any one or more the genes: CABLES, TRIM4, MITF, MMP2, and SLCA25. In some embodiments, the iMGLs upregulate any one or more the genes: TYROPB, CD33, and PICALM.

In some embodiments, methods of regulating gene expression in iMGLs are provided. One of the methods comprises (i) co-culturing iMGLs with neurons and (ii) differentially regulating genes in iMGLs. The neurons co-cultured with iMGLs will comprise be any neurons from any species. In some embodiments, the neurons are rat-hippocampal neurons.

Another method comprises (i) exposing iMGLs to any one or more the compounds: Aβ, fluorescently labeled Aβ, tau, and pHrodo-labeled brain-derived tau oligomers and (ii) differentially regulating genes. The differentially regulated genes will comprise any combination of genes that would be differentially regulated in microglia in response to Aβ, fluorescently labeled Aβ, tau, and pHrodo-labeled brain-derived tau oligomers. In some embodiments, the differentially regulated genes are upregulated genes comprising any one or more of CD33, TYROPB, and PICALM, in any combination.

### Methods of using iMGLs

In some embodiments, a method of assessing gene expression in iMGLs in response to neuronal cues is provided. The method comprises, according to several embodiments, (i) exposing iMGLs to one or more of the factors CX3CL1, CD200, and TGFβ, in any combination and (ii) assessing one or more the of the differentially regulated genes in any combination: P2ry12, EGR1, TGFβ1, ETV5, CX3CR1, APOE, BIN1, CD33, GPR84, COMT, APP, PSEN1, PSEN2, HTT, GRN, FUS, TARDP, VCP, SNCA, C9ORF72, LRRK2, and SOD1.

In some embodiments, a method of assessing engraftment of iMGLs into a cortex is provided. The method comprises, according to several embodiments, (i) transplanting iMGLs into a cortex and (ii) assessing engraftment of the iMGLs into the cortex. In some embodiments, step (ii) occurs at least 2 weeks after step (i), for example, at least 3 weeks after step (i), at least 4 weeks after step (i), at least 5 weeks after step (i), at least 6 weeks after step (i), at least 7 weeks after step (i), at least 8 weeks after step (i), at least 9 weeks after step (i), at least 10 weeks after step (i), at least 11 weeks after step (i), at least 12 weeks after step (i), at least 13 weeks after step (i), at least 14 weeks after step (i), at least 15 weeks after step (i), at least 16 weeks after step (i), at least 17 weeks after (i), at least 18 weeks after step (i), at least 19 weeks after step (i), or at least 20 weeks after step (i). In some embodiments, step (ii) occurs 2 months after step (i). In some embodiments, the method further comprises transplanting the iMGLS into the cortext of a mouse. In some embodiments, the mouse is a MITRG mouse.

In some embodiments, a method of assessing iMGL interaction with AD neuropathy is provided. The method comprises, in several embodiments, (i) transplanting iMGLs into hippocampi and (ii) assessing interaction of the iMGLs in the hippocampi. In some embodiments, the method comprises assessing migration of iMGLs towards plaques. In some embodiments, the method comprises assessing iMGL phagocytosis of fibrillary Aβ.

In some embodiments, a method of studying human microglia in a 3D neuronal environment is provided comprising transplanting iMGLs into a mammalian brain. In some embodiments, the mammalian brain is a mouse brain. In some embodiments, the iMGLs are transplanted into the hippocampi of the mouse brain. In some embodiments, the mouse is a wild-type mouse. In some embodiments, the mouse is an AD mouse strain.

### EXAMPLES

Some aspects of the embodiments discussed above are disclosed in further detail in the following examples, which are not in any way intended to limit the scope of the present disclosure.

### Example 1

### Producing human microglia-like (iMGLs) cells from induced pluripotent stem cells (iPSCs)

A two-step fully-defined protocol was developed to successfully generate microglia-like cells (iMGLs) from iPSCs in just over five weeks (FIG. 1A). The methods and protocols of this and the other Examples, may be used in like manner to generate iMGLs from other PSCs, including ESCs. This approach was utilized to successfully produce iMGLs from over 10 independent iPSC lines. First, iPSCs were differentiated into hematopoietic progenitors (iHPCs), which recapitulates microglia ontogeny as iHPCs represent early primitive hematopoietic cells derived from the yolk sac that give rise to microglia during development. This protocol (**FIG. 1Bi**) yielded primitive iHPCs that are CD43⁺/CD235a⁺/CD41⁺ after 10 days. FACS sorting for CD43⁺ cells revealed that this approach produced iHPCs with a >90% purity (**FIG. 1Bii**).

Second, CD43⁺ iHPCs were grown in serum-free differentiation medium (formulated in house) containing CSF-1, IL-34, and TGFβ1. By day 14, cells expressed the myeloid-associated transcription factor PU.1 and the microglia-enriched protein TREM2 (FIG. 1A iii) demonstrating an early commitment toward microglial fate. Because this protocol yielded large amounts of iMGLs, their development was followed *in vitro* characterizing them every 4 days by flow cytometry. Day 14 early iMGLs were c-kit-/CD45⁺ **(****FIG. 1C****),** suggesting commitment towards a myeloid lineage. Additionally, cells were further subdivided into CD45⁺/CX3CR1⁻ (A1) and CD45⁺/CX3CR1⁺ (A2) populations. CD45 expression was consistently monitored in developing iMGLs and compared to monocyte-derived macrophages (MD-Mϕ). While CD45 expression increased with maturation, levels never reached that of macrophages (**FIG. 1D**), consistent with murine development. A small population of iMGLs (-10%) also expressed intermediate CD11b levels by day 14 that also increased as cells matured, but again never reached macrophage levels (**FIGS. 1E** **and** **1F**).

By day 38, iMGLs exhibited high purity as assessed by purinergic receptor P2RY12 and TREM2 co-localization and quantification (>96%) (**FIG. 1H**). One million iPSCs produced 30-40 million iMGLs with this protocol, suggesting that this approach can be readily scaled-up for high content screening. The resulting iMGLs resemble human microglia, but not monocytes or macrophages by cytospin/Giemsa Staining (**FIG. 1G**) and protein expression (**FIG**. **1I**). Like mouse microglia development *in vivo,* iMGLs developed *in vitro* expressed PU.1, TREM2, and CD11b^{int}/CD45^{low}, and resemble fetal microglia. As iMGLs mature *in vitro* they became more ramified (FIG. 1A iv), similar to microglia *in vivo.*

### Example 2

### Transcriptome analysis of the iMGLs

The transcriptome of the iMGLs was profiled in comparison to human primary fetal microglia (Fetal MG) and adult microglia (Adult MG). The CD14⁺/CD16⁻ monocytes (CD 14 M), CD14⁺/CD16⁺ inflammatory monocytes (CD 16 M), myeloid dendritic cells (Blood DCs), iHPCs, and iPSCs were also examined, in order to compare them to stem cells and other myeloid molecular signatures. Correlational analysis and Principal Component Analysis (PCA) revealed striking similarity of iMGLs to Fetal MG and Adult MG (Fetal MG and Adult MG are located in the same circled cluster in **FIG. 2A****;** see also, **FIG. 9A**). Furthermore, the first principal component PC1 (21.3 % variance, **FIG. 2A** arrows) defined the differentiation time-series from iPSC through iHPC to iMGL cells while PC2 and PC3 defined the dendritic and monocyte trajectories, respectively.

Biclustering analysis using 300 microglial, macrophage, and other immune related genes adapted from previous studies identified similarities between groups and highlighted common gene clusters. This analysis again showed that iMGLs cluster with microglia but are distinct from other myeloid cells, iHPCs and iPSCs (**FIG. 2B**). Importantly, iMGLs, Fetal MG, and Adult MG expressed canonical microglial genes such as P2RY12, GPR34, C1Q, CABLES1, BHLHE41, TREM2, ITAM PROS1, APOE, SLCO2B1, SLC7A8, PPARD, and CRYBB1 (**FIG. 2C****; Table 1**). When compared to monocytes, iMGLs expressed the myeloid genes, RUNX1, PU.1, and CSF1R (**FIG. 8A**), but did not express monocyte-specific transcription factors, IRF1, KLF4, NR4A1 (**FIG. 8B**). Differential analysis between iMGLs, CD14 M, and CD16 M (**FIGS. 8D** **and** **8E**) further emphasized that iMGLs predominantly expressed microglial genes (greater than two-fold change and p<0.001) including CX3CR1, TGFBR1, RGS10, and GAS6, but not monocyte and macrophage genes KLF2, TREM1, MPO, ITGAL, and ADGRES. At the protein level, iMGLs, like primary microglia are CD45^{lo} compared to CD45^{hi} MD-Mϕ, and expressed the microglia surface proteins CX3CR1, TGFBR1, and PROS1 (**FIGS. 10A, 10B, and 10C**). Tables 2 shows top GO pathways enriched in adult MG compared to fetal MG and iMGLs. Table 3 shows GO pathways enriched in fetal MG compared to adult MG and iMGLs. Table 4 shows GO pathways enriched in iMGLs compared to fetal MG and adult MG. Collectively, unbiased whole-transcriptome analysis strongly established iMGLs as a cell model that highly resembles primary human microglia that can be used to study microglia physiology and function in human health and disease.

**TABLE 1**

| | GENES | | | | | |
|---|---|---|---|---|---|---|
| **COMPARISONS** | **P2RY12** | **GPR34** | **CABLES1** | **BHLHE41** | **TREM2** | **OLFML3** |
| **CD14+ M VS. CD16+ M** | 0.7965 | > 0.9999 | > 0.9999 | > 0.9999 | 0.9987 | 0.9871 |
| **CD14+ M VS. BLOOD DC** | 0.7531 | 0.0063 | 0.5606 | > 0.9999 | 0.6405 | > 0.9999 |
| **CD14+ M VS. IMGL** | < 0.0001 | < 0.0001 | < 0.0001 | 0.0064 | < 0.0001 | < 0.0001 |
| **CD14+ M VS. FETAL MG** | < 0.0001 | < 0.0001 | < 0.0001 | < 0.0001 | < 0.0001 | < 0.0001 |
| **CD14+ M VS. ADULT MG** | < 0.0001 | < 0.0001 | < 0.0001 | < 0.0001 | < 0.0001 | < 0.0001 |
| **CD16+ M VS. BLOOD DC** | 0.2047 | 0.0129 | 0.6603 | > 0.9999 | 0.8586 | 0.9976 |
| **CD16+ M VS. IMGL** | < 0.0001 | < 0.0001 | < 0.0001 | 0.0111 | < 0.0001 | < 0.0001 |
| **CD16+ M VS. FETAL MG** | 0.0004 | < 0.0001 | < 0.0001 | < 0.0001 | < 0.0001 | < 0.0001 |
| **CD16+ M VS. ADULT MG** | 0.0002 | < 0.0001 | < 0.0001 | < 0.0001 | < 0.0001 | < 0.0001 |
| **BLOOD DC VS. IMGL** | < 0.0001 | < 0.0001 | < 0.0001 | 0.0256 | < 0.0001 | < 0.0001 |
| **BLOOD DC VS. FETAL MG** | < 0.0001 | < 0.0001 | < 0.0001 | 0.0001 | < 0.0001 | < 0.0001 |
| **BLOOD DC VS. ADULT MG** | < 0.0001 | < 0.0001 | < 0.0001 | < 0.0001 | < 0.0001 | < 0.0001 |
| **IMGL VS. FETAL MG** | < 0.0001 | > 0.9999 | 0.9483 | 0.0256 | 0.0633 | < 0.0001 |
| **IMGL VS. ADULT MG** | < 0.0001 | 0.8258 | 0.3015 | 0.0001 | 0.0633 | < 0.0001 |
| **FETAL MG VS. ADULT MG** | 0.9987 | 0.9431 | 0.1407 | 0.2995 | > 0.9999 | 0.9998 |

| **COMPARISONS** | **PROS1** | **APOE** | **SLC02B1** | **SLC7A8** | **PPARD** | **CRYBB1** |
|---|---|---|---|---|---|---|
| **CD14+ M VS. CD16+ M** | 0.8814 | > 0.9999 | 0.9999 | > 0.9999 | 0.4125 | 0.0011 |
| **CD14+ M VS. BLOOD DC** | 0.4077 | 0.9103 | 0.9994 | 0.9965 | 0.9185 | 0.6963 |
| **CD14+ M VS. IMGL** | < 0.0001 | < 0.0001 | < 0.0001 | < 0.0001 | < 0.0001 | < 0.0001 |
| **CD14+ M VS. FETAL MG** | < 0.0001 | < 0.0001 | < 0.0001 | < 0.0001 | < 0.0001 | < 0.0001 |
| **CD14+ M VS. ADULT MG** | < 0.0001 | < 0.0001 | < 0.0001 | < 0.0001 | < 0.0001 | < 0.0001 |
| **CD16+ M VS. BLOOD DC** | 0.9391 | 0.9455 | 0.9941 | 0.9987 | 0.138 | 0.0002 |
| **CD16+ M VS. IMGL** | < 0.0001 | < 0.0001 | < 0.0001 | < 0.0001 | < 0.0001 | < 0.0001 |
| **CD16+ M VS. FETAL MG** | < 0.0001 | < 0.0001 | < 0.0001 | < 0.0001 | < 0.0001 | < 0.0001 |
| **CD16+ M VS. ADULT MG** | < 0.0001 | < 0.0001 | < 0.0001 | < 0.0001 | < 0.0001 | < 0.0001 |
| **BLOOD DC VS. IMGL** | < 0.0001 | < 0.0001 | < 0.0001 | < 0.0001 | < 0.0001 | < 0.0001 |
| **BLOOD DC VS. FETAL MG** | < 0.0001 | < 0.0001 | < 0.0001 | < 0.0001 | 0.0004 | < 0.0001 |
| **BLOOD DC VS. ADULT MG** | < 0.0001 | < 0.0001 | < 0.0001 | < 0.0001 | < 0.0001 | < 0.0001 |
| **IMGL VS. FETAL MG** | 0.0008 | < 0.0001 | 0.2803 | 0.0127 | 0.091 | > 0.9999 |
| **IMGL VS. ADULT MG** | 0.2533 | < 0.0001 | 0.9909 | 0.4987 | 0.403 | < 0.0001 |
| **FETAL MG VS. ADULT MG** | 0.1787 | > 0.9999 | 0.7213 | 0.4966 | 0.9658 | < 0.0001 |

**Table 2**

| **Adult MG vs Fetal MG** | | | | **Adult MG vs iMGL** | | | |
|---|---|---|---|---|---|---|---|
| **Description** | **GO ID** | **LogP (-)** | **Log (q-value)** | **Description** | **GO ID** | **LogP (-)** | **Log(q-value)** |
| Extracellular matrix organization | 0030198 | 38.10 | -34.18 | Regulation of cell migration | 0030334 | 13.23 | -9.58 |
| Circulatory system development | 0072359 | 36.87 | -33.23 | Regulation of cell adhesion | 0030155 | 11.46 | -8.12 |
| Single organism cell adhesion | 0098602 | 32.24 | -29.04 | Actin filament-based process | 0030029 | 11.03 | -7.78 |
| Regulation of nervous system development | 0051960 | 28.56 | -25.55 | Regulation of anatomical structure morphogenesis | 0022603 | 10.95 | -7.74 |
| Regulation of cellular component movement | 0051270 | 26.49 | -23.54 | Cell junction organization | 0034330 | 10.29 | -7.12 |
| Adaptive immune response | 0002250 | 25.87 | -22.96 | Enzyme linked receptor protein signaling pathway | 0007167 | 9.73 | -6.76 |
| Response to cytokine | 0034097 | 20.02 | -17.49 | Circulatory system development | 0072359 | 8.59 | -5.79 |
| Epithelial cell proliferation | 0050673 | 19.25 | -16.74 | Single-organism catabolic process | 0044712 | 8.13 | -5.42 |
| Central nervous system development | 0007417 | 19.12 | -16.63 | Oxidation-reduction process | 0055114 | 7.78 | -5.11 |
| Negative regulation of cell proliferation | 0008285 | 18.82 | -16.34 | Plasma membrane organization | 0007009 | 7.35 | -4.76 |
| Tissue morphogenesis | 0048729 | 18.50 | -16.06 | Cellular response to oxygen-containing compound | 1901701 | 7.31 | -4.73 |
| Muscle structure development | 0061061 | 17.98 | -15.56 | Negative regulation of cell proliferation | 0008285 | 7.28 | -4.72 |
| Single organism cell adhesion | 0050808 | 17.86 | -15.48 | Positive regulation of phosphorylation | 0042327 | 7.14 | -4.63 |
| Regulation of nervous system development | 0042063 | 17.26 | -14.91 | Renal system development | 0072001 | 6.77 | 4.30 |
| Regulation of Growth | 0040008 | 16.62 | -14.32 | | | | |

**Table 3**

| **Fetal vs Adult MG** | | | | **Fetal MG vs iMGL** | | | |
|---|---|---|---|---|---|---|---|
| **Description** | **GO ID** | **LogP (-)** | **Log (q-value)** | **Description** | **GO ID** | **LogP (-)** | **Log(q-value)** |
| Leukocyte chemotaxis | 0030595 | 6.92 | -3.07 | Single-organism catabolic process | 0044712 | 10.41 | -6.64 |
| Response to acidic pH | 0010447 | 4.07 | -1.33 | Regulation of cell migration | 0030334 | 9.90 | -6.26 |
| Inorganic ion homeostasis | 0098771 | 4.04 | -1.31 | Iron ion transport | 0006826 | 8.68 | -5.52 |
| Regulation of cell migration | 0030334 | 3.80 | -1.18 | Divalent metal ion transport | 0070838 | 8.44 | -5.34 |
| Circulatory system process | 0003013 | 3.72 | -1.13 | Carbohydrate metabolic process | 0005975 | 7.39 | -4.59 |
| Melanosome organization | 0032438 | 3.70 | -1.13 | Small GTPase mediated signal transduction | 0007264 | 6.69 | -3.99 |
| Anion transport | 0006820 | 3.54 | -1.01 | Angiogenesis | 0001525 | 6.64 | -3.97 |
| Macrophage migration | 1905517 | 3.47 | -0.95 | Positive regulation of transport | 0051050 | 6.15 | -3.59 |
| Transmembrane receptor protein tyrosine kinase signaling pathway | 0007169 | 3.23 | -0.81 | Positive regulation of intracellular signal transduction | 1902533 | 6.12 | -3.57 |
| Negative regulation of receptor activity | 2000272 | 3.14 | -0.76 | Aminoglycan metabolic process | 0006022 | 6.03 | -3.50 |
| Positive regulation of phagocytosis, engulfment | 0060100 | 3.13 | -0.76 | Cell projection assembly | 0030031 | 5.81 | -3.31 |
| Sterol import | 0035376 | 3.13 | -0.76 | Cell-substrate adhesion | 0031589 | 5.68 | -3.21 |
| Behavior | 0007610 | 3.11 | -0.75 | | | | |
| Positive regulation of transport | 0051050 | 3.02 | -0.70 | | | | |
| Vesicle organization | 0016050 | 2.96 | -0.65 | | | | |

**Table 4**

| **iMGL vs Fetal MG** | | | | **iMGL vs Adult MG** | | | |
|---|---|---|---|---|---|---|---|
| **Description** | **GO ID** | **LogP (-)** | **Log (q-value)** | **Description** | **GO ID** | **LogP (-)** | **Log(q-value)** |
| Single organism cell adhesion | 0098602 | 30.48 | -26.23 | Mitotic cell cycle process | 1903047 | 28.46 | -24.22 |
| Mitotic cell cycle process | 1903047 | 21.34 | -17.87 | Regulation of cell cycle | 0051726 | 19.26 | -15.94 |
| Immune system development | 0002520 | 18.56 | -15.55 | DNA replication | 0006260 | 17.22 | -14.09 |
| Regulation of cellular component movement | 0051270 | 16.11 | -13.32 | Chromatin organization | 0006325 | 11.44 | -8.64 |
| Mitotic cell cycle phase transition | 0044772 | 15.94 | -13.18 | Regulation of nuclear division | 0051783 | 11.25 | -8.47 |
| Leukocyte migration | 0050900 | 15.19 | -12.49 | Immune system development | 0002520 | 10.90 | -8.15 |
| Taxis | 0042330 | 14.76 | -12.12 | DNA-dependent DNA replication | 0006261 | 10.22 | -7.51 |
| Positive regulation of cell differentiation | 0045597 | 14.76 | -12.12 | Negative regulation of transcription from RNA polymerase II promoter | 0000122 | 10.20 | -7.50 |
| Inflammatory response | 0006954 | 14.64 | -12.03 | Microtubule cytoskeleton organization | 0000226 | 9.96 | -7.27 |
| Anatomical structure formation involved in morphogenesis | 0048646 | 13.83 | -11.27 | Leukocyte activation | 0045321 | 9.57 | -6.94 |
| Positive regulation of cell proliferation | 0008284 | 13.82 | -11.26 | Regulation of small GTPase mediated signal transduction | 0051056 | 9.16 | -6.56 |
| Positive regulation of intracellular signal transduction | 1902533 | 12.20 | -9.73 | Cell cycle G2/M phase transition | 0044839 | 9.01 | -6.44 |
| Positive regulation of hydrolase activity | 0051345 | 11.80 | -9.35 | Signal transduction by p53 class mediator | 0072331 | 8.47 | -5.99 |
| Negative regulation of multicellular organismal process | 0051241 | 11.67 | -9.23 | Regulation of transcription involved in G1/S transition of mitotic cell cycle | 0000083 | 8.34 | -5.88 |
| Negative regulation of cell proliferation | 0008285 | 11.62 | -9.18 | Cellular response to oxygen-containing compound | 1901701 | 8.15 | -5.73 |

### Example 3

### Functional analysis of iMGLs

iMGLs were validated as surrogates of microglia using both functional and physiological assays. Cytokine/chemokine secretion by iMGLs stimulated by Lipopolysaccharide (LPS), and by IL-1β and IFNy (two cytokines that are elevated in AD patients and mouse models) were measured. Results shows that iMGLs secreted 10 of the examined cytokines at low but detectable levels (**Table 5**). However, in response to IFNy or IL-1β, iMGLs secreted 8 different chemokines including TNFα, CCL2, CCL4, and CXCL10. As expected, iMGLs robustly responded to LPS with induction of all measured cytokines except for CCL3 (see, **Table 5** for values). Collectively, this data shows that iMGLs differentially release cytokines/chemokines based on their cell-surface receptor stimuli, a finding that closely aligns with the responses observed in acutely isolated primary microglia(Rustenhoven et al., 2016).

Because iMGLs express the microglial-enriched purinergic receptor P2ry12, which can sense extracellular nucleotides leaked from degenerating neurons and has been shown to be critical for microglial homeostatic function (**FIGS. 1H** **and** **2C**), ADP-P2ry12 mediated chemotaxis and calcium transients were assessed. It was determined that iMGLs migrated robustly in response to ADP and ADP also triggered calcium transients (**FIGS. 3D and 3E**), which can both be negated, by a P2ry12-specific inhibitor, PSB0739. These physiological findings further underscore that iMGLs express functional surface receptors, enabling quantitative analyses of microglial physiology.

Microglia along with astrocytes, also play a critical role in synaptic pruning. Because *in vitro* synaptosome phagocytosis assays are an established surrogate to study pruning, the ability of iMGLs to phagocytose human synaptosomes (hS) was quantitatively assessed. In comparison to MD-Mϕ, iMGL phagocytosis of pHrodo-labeled hS was less robust (**FIGS. 3F 3G**). However, iMGLs preferentially internalized hS when compared to *E. coli* particles and normalized to MD-Mϕ (**FIGS. 10D** **and** **10E**) supporting the notion that iMGLs and microglia are more polarized toward homeostatic functions than MD-Mϕ.

Because microglia (and iMGLs) express both C1q and CR3 (CD11b/CD18 dimer), iMGLs were used to assess whether synaptic pruning in human microglia primarily involves this pathway. Using an additive-free CD11b antibody, iMGL phagocytosis of hS was significantly reduced (-40.0%, ***p<0.0001) (**FIGS. 3H and 3I**). In contrast, an inhibitor of MERTK (UNC569), also implicated in synaptic pruning, only marginally decreased iMGL hS phagocytosis (-12.6%, *p<0.05) (**FIGS. 3H and 3I**). Similar to studies in murine KO studies, that data indicates that MERTK plays a minor role in human microglia-mediated synaptic pruning, and demonstrates that C1q/CR3 is integral for microglia-mediated synaptic pruning in humans.

**TABLE 5**

| | | | **Treatments¹** | | | | | |
|---|---|---|---|---|---|---|---|---|
| | Vehicle | | IFNγ | | IL-1β | | LPS | |
| **CYTOKINES** | **mean ± SE** | **p-value** | **mean ± SE** | **p-value** | **mean ± SE** | **p-value** | **mean ± SE** | **p-value** |
| **TNFA** | 2.56 ± 0.16 | NA | 58.82 ± 8.80 | 0.0008 | 29.74 ± 0.65 | 0.0471 | 116.49 ± 9.77 | < 0.0001 |
| **IL6** | 0.00 ± 0.00 | NA | 12.22 ± 1.44 | 0.5649 | 13.92 ± 0.41 | 0.4736 | 274.39 ± 15.25 | < 0.0001 |
| **IL8** | 339.21 ± 11.29 | NA | 3549.05 ± 181.22 | < 0.0001 | 3,004.54 ± 47.58 | < 0.0001 | 4,347.96 ± 75.61 | < 0.0001 |
| **IL10** | 0.00 ± 0.00 | NA | 4.59 ± 2.35 | 0.1599 | 4.42 ± 1.33 | | 31.31 ± 1.52 | < 0.0001 |
| **IL1A** | 1.59 ± 0.07 | NA | 1.48 ± 0.25 | 0.9999 | 4.89 ± 1.45 | 0.2535 | 30.55 ± 2.19 | < 0.0001 |
| **CCL2** | 96.59 ± 6.27 | NA | 993.26 ± 55.76 | 0.0052 | 275.98 ± 19.54 | 0.7069 | 5,695.46 ± 275.72 | < 0.0001 |
| **CCL3** | 104.91 ± 7.70 | NA | 295.39 ± 19.72 | 0.0043 | 556.24 ± 54.01 | < 0.0001 | 0.00 ± 0.00 | 0.0807 |
| **CCL4** | 3,140.81 ± 165.84 | NA | 4,514.72 ± 10.01 | < 0.0001 | 4,492.26 ± 51.35 | < 0.0001 | 4,594.57 ± 33.96 | < 0.0001 |
| **CXCL10** | 9.62 ± 1.48 | NA | 0.00 ± 0.00 | 0.1762 | 69.49 ± 4.73 | < 0.0001 | 73.72 ± 4.53 | < 0.0001 |
| **CCL17** | 4.70 ± 0.83 | NA | 25.82 ± 1.98 | 0.0168 | 21.75 ± 1.94 | 0.0464 | 92.96 ± 7.70 | < 0.0001 |

### Example 4

### Validating utility of using iMGLS to study Alzheimer's disease

Previous reports have shown that impaired microglia clearance of beta-amyloid (Aβ) is implicated in the pathophysiology of AD. Therefore, iMGLs were examined to determine with they can phagocytose Aβ or tau, two hallmark AD pathologies. Similar to primary microglia, iMGLs internalized fluorescently labeled fibrillar Aβ (**FIG. 4B****,** bottom). iMGLs also recognized and internalized pHrodo-labled brain-derived tau oligomers (BDTOs) (**FIG. 4B****,** top). Fluorescence emitted indicated trafficking of pHrodo-conjugated BDTOs to the acidic lysosomal compartment, which showed that iMGLs can actively ingest extracellular tau that may be released during neuronal cell death. These data support recent findings that microglia may play a role in tau propagation in AD and other tauopathies. Together, these findings suggest that iMGLs could be utlized to identify compounds in high-throughput drug-screening assays that enhance Aβ degradation or block exosome-mediated tau release.

Microglia genes are implicated in late onset AD, yet how they modify disease risk remains largely unknown. Thus, iMGLs were investigated to determine how these genes might influence microglia function and AD risk. Hierarchical clustering using just these 25 AD-GWAS genes demonstrated that iMGLs resemble microglia and not peripheral myeloid cells (**FIG. 4A**). In their investigated basal state, iMGLs and microglia expressed many AD-GWAS-related genes including those without murine orthologs i.e. CD33, MS4A4A, CR1. Thus, iMGLs can be used to study how altered expression of these genes influence microglia phenotype in a way that cannot be recapitulated in transgenic mice. Next, fAβ or BDTO treatment was investigated to determine how it influences AD-GWAS gene expression in microglia. Following fAβ exposure, iMGLs increased expression of 10 genes (**Table 6**) including ABCA7 (5.79 ± 0.44), CD33 (6.02 ± 0.41), TREM2 (4.86 ± 0.50, and APOE (2.52 ± 0.19), genes implicated in Aβ clearance/degradation. BDTOs increased expression of 4 genes including CD2AP (4.62 ± 0.45), previously implicated in tau-mediated toxicity. In addition, 6 genes were differentially elevated in fAβ compared to BDTOs (**Table 6**). In addition, CD33, TYROBP, and PICALM, genes more enriched in other myeloid cells at baseline, were upregulated by fAβ and BDTOs suggesting that proteinopathies may alter microglia phenotype to resemble invading peripheral myeloid cells (Stalder et al., 2005, Prinz et al., 2011, Chan et al., 2007). In addition to AD-GWAS genes, iMGLs express C9orf72, GRN, LRRK2, and TARDBP and can be used to study other neurological diseases such as ALS, FTD, and DLB in which microglia play a prominent role in pathogenesis (**FIG**. **11**).

**TABLE 6**

| **TREATMENTS¹** | | | | | | |
|---|---|---|---|---|---|---|
| **FAB** | | **BDTO** | | **fAβ vs. BDTO** | | |
| **GENES** | **Fold Change (over vehicle) mean ± SE** | **Genes** | **Fold Change (over vehicle) mean ± SE** | **Genes** | **Fold difference mean** | **p-value** |
| **MS4A6A** | 6.32 ± 0.32 | **CD2AP** | 4.62 ± 0.45 | **MS4A6A** | 4.731 | < 0.0001 |
| **CD33** | 6.02 ± 0.41 | **CLU** | 3.84 ± 0.67 | **CD33** | 5.178 | < 0.0001 |
| **ABCA7** | 5.79 ± 0.44 | **BIN1** | 2.56 ± 0.66 | **ABCA7** | 3.333 | 0.0014 |
| **TYROBP** | 4.99 ± 0.31 | **ABCA7** | 2.46 ± 0.70 | **TYROBP** | 3.756 | 0.0002 |
| **TREM2** | 4.86 ± 0.50 | | | **TREM2** | 3.426 | 0.0009 |
| **ZCWPW1** | 3.41 ± 0.42 | | | **ZCWPW1** | 2.610 | 0.0323 |
| **PTK2B** | 2.97 ± 0.16 | | | **PTK2B** | 2.483 | 0.0525 |
| **APOE** | 2.52 ± 0.19 | | | | | |
| **BIN1** | 2.34 ± 0.69 | | | **CD2AP** | -4.144 | < 0.0001 |
| **CLU** | 2.24 ± 0.78 | | | | | |

| | | | | | | |
|---|---|---|---|---|---|---|
| **¹TREATMENTS**: **FAB (5 MG/ML) OR BDTO (5 MG/ML) 24 H.** | | | | | | |

### Example 5

### Interaction and function of iMGLs in the neuronal environment

In the brain, neurons and glia interact with microglia and influence function and gene expression. Therefore, iMGLs were cultured with rat-hippocampal neurons (21 div) to assess how iMGLs respond to neuronal cues (**FIG**. **5A**). Rat-hippocampal neurons were used because they readily form synapses in culture and can be generated with limited variability. iMGLs were subsequently separated from neurons by FACs with human specific CD45 and CD11b antibodies and profiled at the transcriptome level (**FIG**. **5B**). Differential gene expression analysis revealed that neuronal co-culturing upregulated 156 and downregulated 244 iMGL genes (**FIGS**. **5C and 5D**). FFAR2 and COL26A1 are two genes differentially expressed in iMGLs cultured with only defined factors and indicate a developmentally primed microglia profile. In contrast, co-culturing microglia with neurons increased expression of Siglec11 and 12, human-specific sialic-acid binding proteins that interact with the neuronal glycocalyx. Additionally, the increased expression of microglial genes CABLES1, TRIM14, MITF, MMP2, and SLCA25 implicate both neuronal surface cues and soluble factors in microglia maturation (**FIGS. 5E** **and** **12**).

A fundamental characteristic of microglia is the surveillance of the CNS environment with their highly ramified processes. To investigate how iMGLs might interact within a CNS environment, iMGLs were cultured with human iPSC (hiPSC) 3D brain-organoids (BORGs). BORGs include neurons and astrocytes that self-organize into a cortical-like network, but lack microglia (**FIG. 6****, Panel B**). To test if iMGLs invade BORGs similarly to how microglia enter the developing neural tube, iMGLs were added to BORG cultures. By day three, iMGLs had embedded into the BORGS and were no longer detectable within the media, suggesting rapid iMGL chemotaxis toward neuronal cues (**FIG. 6****, Panel A**). The iMGLs also tiled and extended varying degrees of ramified processes within the 3D organoid environment (**FIG. 6****, Panel B)**. iMGL projections were observed in a vast majority of cells and exhibited similar morphology to microglia *in vivo* (**FIG. 6****, Panel B**). IMARIS 3D image reconstruction of select iMGLs highlights the development of ramified iMGLs in BORGs. To determine whether iMGLs respond to neuronal injury, BORGS were pierced with a 25-gauge needle (white long arrow, **FIG. 6****, Panel C**). After injury, iMGLs clustered near the injury site and at BORG edges (**FIG. 6****, Panel C**), and adopted a more amoeboid morphology, resembling "activated" microglia found in injured or diseased brains (**FIG**. **6**, **Panel C**). Collectively, these data demonstrate that iMGLs can integrate within a 3D brain environment, mature, ramify, and respond to injury similar to brain microglia.

### Example 6

### iMGL interaction with neurons, astrocytes, and endothelial cells in the brain

Neurons, astrocytes, and endothelial cells in the brain interact with microglia to influence gene expression and function. The differentiation protocol attempted to recapitulate CNS cues present in the brain by including signals derived from these other cell types including CX3CL1, CD200, and TGFβ. Whole transcriptome RNA-seq analysis confirmed the importance of these factors for establishing microglia *in vitro* (FIGS. 16A-E and 17A-E). TGFβ, a glia-derived cytokine, is needed for murine microglia development *in vivo* and in maintaining the microglial-specific transcriptome signature. Differential gene expression analysis confirmed TGFβ's role in maintaining the human microglia transcriptome signature; 1262 genes were differentially expressed in iMGLs with TGFβ, whereas 1517 genes were differentially expressed in iMGLs after TGFβ removal (24 hours). Many of the differentially expressed genes are identified as core microglial signature targets including P2RY12, TGFβR1, and CD33, and transcription factors EGR1 and ETV5, and APOE (FIGS. 16A-C). Examination of gene ontology highlighted neurodegenerative disease pathways including AD, Parkinson's, and Huntington's diseases that are TGFβ dependent (**FIG. 16D**). Furthermore, removal of TGFβ led to significant changes in many of the human microglia homeostatic targets also identified as AD GWAS loci genes including TREM2, APOE, ABCA7, SPI1 (CELF1 locus), PILRA (ZCWPW1 locus), and the HLA-DR and MS4A gene clusters (Karch et al., 2016), suggesting many identified AD GWAS genes function in the maintenance of microglia homeostasis (**FIG. 16E**) and underscoring the utility of iMGLs to interrogate AD GWAS gene function.

### Example 7

### Effect of CX3CL1 and CD200 on the iMGL phenotype

CX3CL1 and CD200 are both neuronal- and endothelial-derived cues that can further educate iMGLs toward an endogenous microglia phenotype. CX3CL1 and CD200 were tested to determine how inclusion or exclusion of these factors modulates iMGL phenotype. The addition of CD200 and CX3CL1 to iMGLs increased the expression of select genes like COMT (**FIG. 16B**), CD52, a cell surface receptor that binds Siglec-10 and interacts with DAP12 as part of the microglia sensome, a HLADRB5, a member of the MHC II complex implicated in AD, while maintaining similar expression levels of core-microglial genes (e.g. P2RY12, TYROBP, OLFML3) and AD-risk genes (**FIG. 17A**). Results showed that CD200 and CX3CL1 modulated iMGL response to CNS stimuli, such as fAβ. In the absence of CD200 and CX3CL1, fAβ stimulated the expression of AD-GWAS genes implicated in interacting with misfolded folded protein, surface receptors, or anti-apoptotic events such as CLU (APOJ). Whereas cells exposed to these two factors respond differentially to fAβ, increased expression of genes involved in cell surface recognition of neuronal motifs, or phagocytosis of CNS substrates, including MS4A genes, TREM2, TYROBP, CD33, and ABCA7 (FIG. 178B). These studies further support the notion that CD200-CD200R1 and/or CX3CL1-CX3CR1 axis can modulate microglia to response to neurodegenerative conditions. Thus, exposure to soluble CNS factors, like CD200 and CX3CL1, may allow for access to microglial-specific transcriptional regulator elements.

### Example 8

### Examining effect of direct contact of iMGL with CNS environment on iMGL maturation

Next, it was examined whether iMGL maturation can be achieved with direct contact with the CNS environment. iMGLs were cultured with rat-hippocampal neurons (21 DIV) to assess how iMGLs respond to neuronal surface cues (**FIG. 5A**). Rat-hippocampal neurons were used because they readily form synapses in culture and can be generated with limited variability. iMGLs were subsequently separated from neurons by FACs with human specific CD45 and CD11b antibodies and profiled at the transcriptome level (**FIG. 5B**). Differential gene expression analysis revealed that neuronal co-culturing upregulated 156 and downregulated 244 iMGL genes (FIGS. 5C and D). FFAR2 and COL26A1 are two genes differentially expressed in iMGLs cultured with only defined factors and indicate a developmentally primed microglia profile. In contrast, co-culturing microglia with neurons increased expression of Siglec11 and 12, human-specific sialic-acid binding proteins that interact with the neuronal glycocalyx, function in neuroprotection, and suppress pro-inflammatory signaling, and thus maintain a microglia homeostatic state. Additionally, increased expression was observed of microglial genes CABLES 1, TRIM14, MITF, MMP2, and SLC2A5. Overall, these results implicate both soluble and surface CNS cues as factors in microglia maturation (FIGS. 5A-F)**.**

### Example 9

### iMGL interaction in human brain environment

A fundamental characteristic of microglia is the surveillance of the CNS environment with their highly ramified processes. To investigate how iMGLs might interact within a human brain environment, iMGLs were cultured with hiPSC 3D brain-organoids (BORGs). BORGs include neurons, astrocytes, and oligodendrocytes that self-organize into a cortical-like network, but lack microglia (**FIG**. **6**). To test if iMGLs invade BORGs similarly to how microglia enter the developing neural tube, iMGLs were added to BORG cultures. By day three, iMGLs had embedded into the BORGS and were no longer detectable within the media suggesting rapid iMGL chemotaxis toward CNS cues (**FIG. 6** **panels A-C**). By day 7, the iMGLs also tiled and extended varying degrees of ramified processes within the 3D organoid environment (**FIG. 6** **panels D-F)**. To determine whether iMGLs respond to neuronal injury, BORGS were pierced with a 25-gauge needle. After injury, iMGLs clustered near the injury site and adopted a more amoeboid morphology, resembling "activated" microglia found in injured or diseased brains **(****FIG. 6** **panels G-I)**. Collectively, these data demonstrate that iMGLs can integrate within an *in vitro* 3D brain and CNS environment in which the iMGLs can mature, ramify, and respond to injury similar to brain microglia.

### Example 10

### Investigating iMGLs within the context of the CNS environment in vivo

iMGLs were examined within the context of a CNS environment *in vivo.* iMGLs (day 38) were transplanted into the cortex of MITRG mice that are Rag2-deficient and IL2rγ-deficient mice and also express the human forms of four cytokines knocked-in (M-CSF^{h};IL-3/GM-CSF^{h};TPO^{h}), allowing for xenotransplantation and survival of myeloid and other leukocytes (**FIG**. **13**). Two months after transplantation, the extent of engraftment in MITRG cortices was assessed by immunohistochemistry. Human iMGLs were distinguished from endogenous microglia by using either human specific nuclear or cytoplasmic markers ku80 (hNuclei) and SC121 (hCyto), respectively. P2ry12 and human-specific Tmem119 antibodies were used to assess the homeostatic state and identity of transplanted microglia. Transplanted human iMGLs co-expressing both ku80 and P2ry12 were abundant within MITRG brains suggestive of their long-term engraftment potential (**FIG. 13** **panels A-D**). Higher magnification images showed P2ry12 expression in highly ramified iMGLs resembling quiescent cortical microglia in which the membrane distribution accentuates the finer extended processes (**FIG. 13** **panels B-D**). Tmem119 was also expressed in both hCyto⁺ soma and the processes of highly arborized iMGLs (**FIG. 13** **panels E-H**). High magnification images of hCyto⁺ cells show Tmem119 is predominately membrane-bound and in agreement with published work. Taken together, these findings suggest that long-term survival and engraftment of iMGLs result in highly branched microglia-like cells that express Iba1, P2ry12 and Tmem119 (**FIG. 13** **panels I-L**), and resemble endogenous quiescent microglia. Also, the morphology and high expression of the homeostatic P2ry12 receptor suggests that transplanted iMGLs are actively surveying their neuronal environment that translates to their potential use in studying human microglia in mouse CNS-disease models.

### Example 11

### Transplanting iMGLs into hippocampi to determine how iMGLs interact with AD neuropathology

iMGLs were transplanted into the hippocampi of xenotransplantation-compatible AD mice, previously generated and characterized, to examine how iMGLs interact with AD neuropathology *in vivo* **(****FIG. 13** **panels M-P and** **FIG. 18**). Transplanted iMGLs engraft and migrate along white matter tracts, similar to microglia in development **(****FIG. 13** **panel M**). In many instances, iMGLs migrated and extended processes towards Aβ plaques to begin walling them off (**FIG. 13** **panels N-P**). A number of iMGLs also began to phagocytose fibrillar Aβ (**FIG. 13** **panels N-P,** **FIG. 18** **panels E-H**). Similarly, human fetal microglia migrated towards Aβ, extended processes, and phagocytosed Aβ when transplanted in the same AD transgenic model (**FIG. 18** **panels A-D**).

### Experimental methods and materials

### Reagents

All cell culture flasks, reagents, supplements, cytokines, and general reagents were purchased from ThermoFisher (Carlsbad, CA) unless otherwise noted.

### Maintenance and Culture of Human Pluripotent Stem Cells (hPSCs)

All stem cell work was performed with approval from UC Irvine Human Stem Cell Research Oversight (hSCRO) and IBC committees. Use of human tissue was performed in accordance and approval of Institutional Review Board (IRB). Human iPSC cell lines ADRC F5 and ADRC F14 (control subjects) were generated by the UCI ADRC Induced Pluripotent Stem Cell Core using non-integrating Sendai virus (Cytotune). iPSCs were confirmed to be karyotype normal by G-banding, sterile, and pluripotent via Pluritest (UCLA) Analysis. iPSCs were maintained feeder-free on matrigel (MTG) in complete TeSR-E8 medium (Stemcell Technologies) in a humidified incubator (5% CO₂, 37°C).

### Differentiation of iPSCs to Hematopoietic Progenitor Cells (iHPCs)

Human iPSC derived hematopoietic progenitors were generated using defined conditions with several modifications to previously published protocols (Kennedy et al., 2007, Sturgeon et al., 2014). Briefly, iPSCs were triturated to generate a single-cell suspension and seeded in 6-well plates at 1-6 × 10⁵ cells per well in E8 medium + Y-27632 ROCK Inhibitor (10 µM; R&D Systems). In some embodiments, Y-27632 is substituted with Thiazovivin (R&D systems). Cells were cultured for 24 hours under normoxic (20% O₂) conditions after which the E8 media was changed to differentiation media composed of a base media and cytokines: IMDM/F12 (50:50), insulin (0.02 mg/ml), holo-transferrin (0.011 mg/ml), sodium selenite (0.0134 mg/ml), L-ascorbic acid 2-Phosphate magnesium (64 µg/ml; Sigma), monothioglycerol (400 µM), PVA (5 mg/ml; Sigma), L-alanyl-L-glutamine (2mM), chemically-defined lipid concentrate (1X), non-essential amino acids (NEAA; 1X), FGF2 (50 ng/ml), BMP4 (50 ng/ml), Activin-A (12.5 ng/ml), and LiCl (2mM) in hypoxia (5%O₂). After two days, media was changed to base media supplemented with FGF2 (50 ng/ml) and VEGF (50 ng/ml). On day 4, media was changed to media containing FGF2 (50 ng/ml), VEGF (50 ng/ml), TPO (50 ng/ml), SCF (50 ng/ml), IL-6 (50 ng/ml), and IL-3 (50 ng/ml). On Day 6, media was supplemented with aforementioned medium. Cells were cultured for an additional 4 days (10 days total), after which, CD43⁺ cells were isolated by FACS for iMGL differentiation. Additionally, iPSC-derived HPCs (Cellular Dynamics) were identified as a commercial source of CD43⁺ progenitors.

### Generation of Microglia-like Cells from iHPCs

CD43⁺ iHPCs were plated in Matrigel-coated 6-well plates (BD Biosciences) with serum-free complete differentiation media at a density of 1-2 ×10⁵ cells per well. Differentiation media consists of M-CSF (25 ng/ml), IL-34 (100 ng/ml; Peprotech), and TGFβ-1 (50 ng/ml; Militenyi) added to a base media (phenol-free DMEM/F12 (1:1), insulin (0.2 mg/ml), holo-transferrin (0.011 mg/ml), sodium selenite (0.0134 mg/ml), Penicillin/streptomycin (1% v/v), B27 (1% v/v), N2 (0.5%, v/v), monothioglycerol (200 µM), and additional insulin (4 µg/ml) just before addition to cells). Cells were supplemented with complete differentiation media every two days. At day 12, early iMGLs were collected (300x g for 5 mins at 25°C) and a 50% media change was performed. After 25 days of microglial differentiation (35 days from iPSC), iMGLs were cultured in complete differentiation media supplemented with CD200 (100 ng/ml, Novoprotein) and CX3CL1 (100 ng/ml; Peprotech) for an additional three days, cultured with hippocampal neurons, or cultured with human brain-organoids.

### Isolation of PBMCs from Human Blood

Human peripheral blood mononuclear cells (PBMCs) were isolated from healthy donors using Ficoll-paque (GE Healthcare) gradient separation. In brief, blood was layered on top of Ficoll-Paque and centrifuged in swinging bucket rotator without brake (400x g, 40 minutes, 18°C). After centrifugation, plasma and upper layers were removed and PBMCs isolated from the interphase. Cells were then washed once with ice-cold PBS and used immediately.

### Isolation of Monocytes from PBMCs

CD14 and CD16 monocytes were isolated via negative selection from PBMCs using the Easy Sep^{™} Monocyte Enrichment Kit (Stemcell Technologies) according to manufacturer's instructions. Isolated cells were washed three times with PBS and sorted by FACs for either RNA-sequence analysis or used for further macrophage differentiation.

### Monocyte-derived Macrophages

Isolated monocytes were plated onto tissue culture treated 6-wells at 2 ×10⁶ cells/ml in RPMI-1640 media at 37°C 5%CO₂ incubator. After two hours, media was aspirated to waste and adherent monocytes washed three times with DPBS and replaced with complete media composed of RPMI-1640, FBS (10% v/v), Penicillin/streptomycin (1% v/v), L-alanyl-L-glutamine (2mM). To generate MD-Mφ, M-CSF (25 ng/ml) was added to wells and cells differentiated for 5 days.

### RNA-seq library construction

Cells were harvested and washed three times with DPBS and stored in RNAlater, RNA preservation solution. RNA was extracted from all cell types using using RNeasy Mini Kit (Qiagen) following manufacturer's guidelines. RNA integrity (RIN) was measured for all samples using the Bioanalzyer Agilent 2100 series. All sequencing libraries analyzed were generated from RNA samples measuring a RIN score ≥ 9. The Illumina TruSeq mRNA stranded protocol was used to obtain poly-A mRNA from all samples. 200 ng of isolated mRNA was used to construct RNA-seq libraries. Libraries were quantified and normalized using the Library Quantification Kit from Kapa Biosystems and sequenced as paired-end 100 bp reads on the Illumina HiSeq 2500 platform.

### RNA-seq analysis

RNA-seq reads were mapped to the hg38 reference genome using STAR aligner and mapped to Gencode version 24 gene annotations using RSEM. Genes with expression (< 1 FPKM) across all samples were filtered from all subsequent analysis. Differential gene expression analysis was performed on TMM normalized counts with EdgeR(Robinson et al., 2010). Multiple biological replicates were used for all comparative analysis. A p-value ≤ 0.001 and a 2-fold change in expression were used in determining significant differentially expressed genes for respective comparisons. PCA analysis was performed using the R package rgl and plotted using plot3d. Clustering was performed using R hclust2 and visualized using Java Tree View 3.0.

### ADP migration and calcium imaging assays

Trans-well migration assays to ADP was performed as previously described(De Simone et al., 2010; Moore et al., 2015). iMGLs (5.5x104 cells/well) were cultured in serum-free basal media without cytokines for 1hour. Next, iMGLS were pre-exposed to DMSO or PSB0739 (50 µM, Tocris) for 1hr at 37°C in 5% CO2 cell culture incubator. Cells were then washed three times with basal medium and plated in trans-well migration chambers (5 µm polycarbonate inserts in 24 wells; Corning) containing Adenosine 5'-phosphate (ADP, 100 µM; Sigma) in the bottom chamber in 37°C in 5% CO2. After 4 hours, cells were washed three times and fixed in PFA (4%) for 15 minutes at room temperature. Cells were stained with Hoechst stain for 10 mins to visualize nuclei of cells. A blinded observer counted total cells per slide and then scrubbed cells off top surface, washed with PBS, and recounted to record migrated cells. Migration was reported as migrated over total cells per well. Fluorescent images of cells were captured using Olympus IX71 inverted microscope.

For calcium imaging, iMGLs were plated on poly-L-lysine-coated coverslips and 1 hour later were incubated with Fura-2-AM (Molecular Probes) calcium dye diluted in Ringer solution containing (in mM): NaCl 140, KCl 4.5, CaCl₂ 2, MgCl₂ 1, HEPES 10, glucose 10, sucrose 5, pH=7.4. After a 1-hour incubation, the dye was washed out 3 times using Ringer solution and treated for 1 hour with either P2RY12 inhibitor PSB0739 (50 µM, Tocris) or Vehicle (DMSO) and used for experiments. Baseline Ca²⁺ signal (I₃₄₀/I₃₈₀) were measured for more than 100 s and then ADP (10 µM) was introduced under stead flow after baseline measurement. Ca²⁺ recordings were performed on Zeiss (Axiovert 35)-based imaging setup and data acquisition was conducted with Metafluor software (Molecular Devices). Data analysis was performed using Metafluor, Origin Pro, and Prism 6.0.

### Immunocytochemistry and immunohistochemistry

Cells were washed with cold PBS and fixed with cold PFA (4%) for 20 min at 25°C followed by three washes with PBS. Cells were blocked with PBS with either 0.05% goat or donkey serum, and with Triton X100 (0.01%) for 1 hour at 25°C. Primary antibodies (1:500) were added in blocking solution overnight at 4°C. Cells were then washed three times with PBS and stained with Alexa Fluor^{®} conjugated secondary antibodies at 1:400 for 1 hour at 25°C. After secondary staining, cells were washed three times followed by coverslip with DAPI-counterstain mounting media (Fluoromount, southern Biotech). Primary antibodies used for immunocytochemistry analysis include: β-3Tubulin (Biolegend), GFAP (Abcam), Iba1 (Wako), ITGB5 (Abcam), MMP-9 (Novus), MerTK (Biolegend), P2RY12 (Sigma), PROS1 (Abcam), PU.1 (Cell Signaling Technology) hCytoplasm (SC121; Takara Bio Inc.), TREM2 (R&D Systems), TGFβR1 (Abcam)

For ICC, cells were washed three times with DPBS (1X) and fixed with cold PFA (4% w/v) for 20 min at room temperature followed by three washes with PBS (1X). Cells were blocked with blocking solution (1X PBS, 5% goat or donkey serum, 0.2% Triton X-100) for 1 h at room temperature. ICC primary antibodies were added at respective dilutions (see below) in blocking solution and placed at 4°C overnight. The next day, cells were washed 3 times with PBS for 5 min and stained with Alexa Fluor^{®} conjugated secondary antibodies at 1:400 for 1 h at room temperature in the dark.

After secondary staining, cells were washed 3 times with PBS and coverslipped with DAPI-counterstain mounting media (Fluoromount, southern Biotech). For BORG IHC, tissue were collected and dropped-fixed in PFA (4% w/v) for 30 min at room temperature and then washed three times with PBS. BORGs were then placed in sucrose solution (30% w/v) overnight before being embedded in O.C.T (Tissue-Tek). Embedded tissue was sectioned at 20 µm using a cryostat and mounted slides were stored at -20°C until staining. For BORG staining, mounted tissue was removed from storage and warmed by placing at room temperature for 30 min. Tissue were rehydrated and washed with room temperature PBS (1X) 3 times for 5 min.

Heat-mediated antigen retrieval was performed by using Citrate Buffer (10mM Citrate, 0.05% Tween 20, pH=6.0) at 97°C for 20 min and then allowed to cool to room temperature. After antigen retrieval, slides were washed three times with PBS. Slides were then washed once in PBS-A solution (1X PBS with 0.1% Triton X-100) for 15 min. Tissue was blocked using PBS-B solution (PBS-A, 0.2% BSA, and 1.5% goat or donkey serum) for 1 h at room temperature. After block, primary antibodies were added to PBS-B solution (250-350 µl/ slide) at appropriate dilutions (see below) and incubated overnight at room temperature. The next day, slides were washed with PBS-A solution 3 times for 5 min each. Tissue were blocked for 1 h using PBS-B solution at room temperature. After block, slides were incubated with Alexa Fluor^{®} conjugated secondary antibodies (all at 1:500) and Hoechst stain (1X) in PBS-B (for 250-300 µl/slide) for 2 h at room temperature in the dark.

After secondary staining, slides were washed 5 times with PBS for 5 min. Slides were cover slipped using fluoromount (Southern Biotech). For mouse brain IHC, brains were collected, fixed, and processed as mentioned above. Free-floating sections were blocked in blocking solution (1X PBS, 0.2% Triton X-100, and 10% goat serum) for 1 h at room temperature with gentle shaking. For human TMEM119 staining, heat mediated antigen retrieval was performed prior to blocking, as performed previously (Bennett et al., 2016). Free-floating tissue antigen retrieval was performed by placing floating sections in a 1.5 ml micro centrifuge tube containing 1 ml of Citrate Buffer solution and placing in a preheated temperature block set at 100°C. Tissue was heated for 10 min at 100°C then removed and allowed to come to room temperature for 20 min before washing with PBS 3 times for 5 min and then proceeding with blocking step. For AD mouse brain staining of amyloid plaques, floating sections were placed in 1X Amylo-Glo ^{®} RTD^{™} (Biosensis) staining solution for 10 min at room temperature without shaking.

After staining, sections were washed in PBS 3 times for 5 minutes each and briefly rinsed in MiliQ DI water before being placed back in to PBS followed by blocking. Primary antibodies were added to staining solution (1X PBS, 0.2% Triton X-100, and 1% goat serum) at appropriate dilutions (see below) and incubated overnight at 4°C with slight shaking. The next day, sections were washed 3 times with PBS and stained with Alexa Fluor^{®} conjugated secondary antibodies at 1:400 for 1 h at room temperature with slight shaking in the dark. After secondary staining, sections were washed in PBS 3 times for 5 min and mounted on glass slides. After mounting, slides were cover slipped with DAPI-counterstain mounting media (Fluoromount, southern Biotech). Primary antibodies:
rabbit anti-Amyloid Fibrils (OC) (1: 1,000, EMD Millipore, AB2286)
rabbit anti-Amyloid Oligomeric (A11) (1:1,000, EMD Millipore, AB9234)
mouse anti-Amyloid 1-16aa (6e10) (1:1,000, Biolegend, 803001)
mouse anti-β3Tubulin (1:500; Biolegend, 801201)
mouse anti-human Cytoplasm (SC121,1:100; Takara Bio Inc., Y40410),
mouse anti-human Nuclei (ku80, 1:100; Abeam, ab79220)
chicken anti-GFAP (1:500; Abeam, ab4674)
rabbit anti-Iba1 (1:500; Wako; 019-19741)
goat anti-Iba1 (1:100; Abeam ab5076) * recommend use with Alexa Fluor 488 or 555 secondary antibody only.
mouse anti-ITGB5 (1:500;Abcam, ab177004)
mouse anti-MMP-9 (1:500; EMD Millipore, AB19016)
mouse anti-human Mertk (1:500; Biolegend, 367602)
rabbit anti-P2ry12 (1:125; Sigma; HPA014518)
rabbit anti-Pros (1:500; Abeam, ab97387)
rabbit anti-PU.1 (1:500; Cell Signaling Technology, 2266S)
rabbit anti-human Tmem119 (1:100; Abeam, ab185333)
goat anti-human Trem2 (1: 100; R&D Systems, AF1828)
rabbit anti-Tgfbr1 (1:500; Abeam, ab31013 ).

### Confocal Microscopy and Brightfield Imaging

Immunofluorescent sections were visualized and images captured using an Olympus FX1200 confocal microscope. To avoid non-specific bleed-through each laser line was excited and detected independently. All images shown represent either a single confocal z-slice or z-stack. Bright field images of cell cultures were captured on an Evos XL Cell Imaging microscope.

### Flow Cytometer Analysis

Cells were suspended in FACs buffer (DPBS, 2% BSA, and 0.05mM EDTA) and incubated with human Fc block (BD Bioscience) for 15 min at 4°C. For detection of microglial surface markers, cells were stained with anti CD11b-FITC clone ICRF44, anti CD45-APC/Cy7 clone HI30, anti CX3CR1-APC clone 2A9-1, anti CD115-PE clone 9-4D, and anti CD117-PerCP-Cy5.5 clone 104D2. Live/dead cells were gated using ZombieViolet^{™} live/dead stain, all from Biolegend (San Diego, CA). Cells were run on FACs Aria II (BD Biosciences) and analyzed with FlowJo software (FlowJo).

### Cytospin and May-Grunwald Giemsa Stain

1×10⁵ cells were suspended in 100 µl of FACs buffer and added to Shandon glass slides (Biomedical Polymers) and assembled in a cytology funnel apparatus. Assembled slides containing cells were loaded in a cytospin instrument and centrifuged (500 rpm, 5 min). Slides were allowed to air-dry for two minutes and immediately stained in 100% May-Grunwald stain (Sigma) for 5 min. Next, slides were washed in PBS for 1.5 min and immediately placed in 4% Giemsa stain (Sigma) for 20 min at room temperature. Slides were washed in double-distilled H₂O 6 times and allowed to air-dry for 10 min. Slides were preserved using glass coverslips and permount (Sigma).

### RNA Isolation and qPCR Analysis

Cells were stored in RNAlater stabilizing reagent and RNA was isolated using Qiagen RNeasy Mini Kit (Valencia, CA) following manufacturer's guidelines. qPCR analysis was performed using a ViiA^{™} 7 Real-Time PCR System and using Taqman qPCR primers. Analysis of AD-GWAS genes utilized a custom Taqman Low Density Array card using the primers described below.

### Rat Cortical and Hippocampal Neuron Isolation

All procedures were performed under an IUCAC approved protocol. Primary cortical and hippocampal neuron cultures were derived from embryonic rat (E18). Briefly, dissected tissue was dissociated with trypsin, triturated, and plated on 6-well plates coated with poly-L-lysine coated in serum-free Neurobasal supplemented with B27(1% v/v) (NB medium). Cells were plated at a density of 5 × 10⁶ cells/ml and maintained in culture until used.

### iMGL Co-culture with Rat Neurons

Rat hippocampal or cortical neurons were cultured for 21 days with 50% media change every 3-4 days. iMGLs were cultured with neurons at a 1:5 ratio (1 ×10⁶ iMGL to 5 × 10⁶ neurons) in 50% iMGL and 50% NB medium. After 3 days, iMGLs were collected for RNA isolation.

### Mesoscale Multiplex Cytokine and Chemokine Assay

iMGLs culture media was replaced with basal media for 2 hours prior to stimulation with IFNγ (20 ng/ml), IL1β (20 ng/ml), and LPS (100 ng/ml) for 24 hours, after which cells were collected for RNA and conditioned media assessed for cytokine secretion. To simultaneously assess multiple cytokine and chemokine analytes from iMGL conditioned media, conditioned media from each treatment group was processed and analyzed using the V-PLEX human cytokine 30-plex kit (Mesoscale) according to the manufacturer protocol.

### 3D Brain-Organoid Cell Culture

Human 3D brain organoids were generated as previously described with some modifications (Lancaster et al., 2013) with modifications detailed in Supplemental Information.

### Fibrillar Aβ Preparation.

Fibrillar fluorescent amyloid-beta (fAβ₁₋₄₂) was generated. Briefly, fluorescently labeled Aβ peptide (Anaspec; Fremont, CA) was first dissolved in 0.1% NH₄OH to 1 mg/ml, then further diluted in sterile endotoxin-free water and incubated for 7 days at 37°C. fAβ was thoroughly mixed prior to cell exposure.

### BDTO Preparation

Tau oligomers were isolated by immunoprecipitation with the T22 antibody using PBS-soluble fractions of homogenates prepared from AD brain. These were then purified by fast protein liquid chromatography (FPLC) using PBS (pH 7.4). Additional analyses include Western blots to detect contamination with monomeric tau or large tau aggregates (tau-5, normally appear on top of the stacking gel) and using a mouse anti-IgG to identify non-specific bands. BDTOs were subsequently conjugated to pHrodo-Red according to the manufacturer's protocol.

### Human synaptosomes

Human tissue samples were obtained at autopsy and minced, slowly frozen in 0.32 M sucrose with 10% DMSO and stored at -80 °C. To obtain a crude synaptosome fraction, tissue was thawed in a 37 °C water bath and homogenized in 10 mm Tris buffer (pH 7.4) with proteinase inhibitors (Roche) and phosphatase inhibitors (Sigma-Aldrich) using a glass/Teflon homogenizer (clearance 0.1-0.15 mm). The homogenate was centrifuged at 1000 g at 4 °C for 10 min, the supernatant was removed and centrifuged again at 10 000 g at 4 °C for 20 min. Resulting pellets were resuspended in sucrose/Tris solution and stored at -80 °C. Synaptosomes were conjugated to pHrodo-Red according to the manufacturer protocol.

### Phagocytosis assays

iMGLs and MD-Mφ, were incubated with mouse anti CD16/32 Fc-receptor block (2 mg/ml; BD Biosciences) for 15 minutes at 4°C. Cells were then stained with anti CD45-APC clone (mouse cells; Tonbo Biosciences; San Diego, CA) at 1:200 in flow cytometer buffer. Samples were then analyzed using Amnis Imagestreamer^{x} Mark II Imaging Flow Cytometer (Millipore). *E.coli,* human synaptosome, fAβ, and BDTO phagocytosis was analyzed using the IDEAS software onboard Internalization Wizard algorithm. Additive free Anti-CD11b antibody (Biolegend) was used for CD11b blockade.

### Statistical Analysis

Statistical analysis was performed using Graphpad Prism 6 software. Comparisons involving more than two groups utilized 1-way ANOVA followed by Tukey's *post hoc* test and corrected p-values for multiple comparisons were reported. Comparisons of two groups utilized two-tailed Students t-test. All differences were considered significantly different when p<0.05. Statistical analysis for RNA-sequencing is detailed above and all other statistical analysis are reported in the figure legends.

### ADP migration and calcium imaging assays

iMGLs (5.5×10⁴ cells/well) were cultured in serum-free basal media without cytokines for 1hour. Next, iMGLS were pre-exposed to DMSO or PSB0739 (50 µM, Tocris) for 1hr at 37°C in 5% CO₂ cell culture incubator. Cells were then washed three times with basal medium and plated in trans-well migration chambers (5 µm polycarbonate inserts in 24 wells; Corning) containing Adenosine 5'-phosphate (ADP, 100 µM; Sigma) in the bottom chamber in 37°C in 5% CO₂. After 4 hours, cells were washed three times and fixed in PFA (4%) for 15 minutes at room temperature. Cells were stained with Hoechst stain for 10 mins to visualize nuclei of cells. A blinded observer counted total cells per slide and then scrubbed cells off top surface, washed with PBS, and recounted to record migrated cells. Migration was reported as migrated over total cells per well. Fluorescent images of cells were captured using Olympus IX71 inverted microscope.

### 3D Brain-Organoid Cell Culture

iPSCs were cultured and maintained on Vitronectin XF (Stem Cell Technologies) in 6-well tissue culture treated plates (BD Falcon) and maintained with TeSR-E8 media (Stem Cell Technologies) daily, at 37°C with 5% CO₂. At approximately 80% confluency, iPSCs were detached from the Vitronectin XF substrate using the standard ReLeSR protocol (Stem Cell Technologies) and centrifuged, pelleted, and suspended in embryoid body (EB) media, which consists of KO DMEM/F12 (Invitrogen), KOSR (20% v/v) (v/v), L-alanyl-L-glutamine (2mM), NEAA (1X), 2-Mercaptoethanol (0.1mM), rhubFGF (4 µg/ml), and HSA (0.1% v/v) and ROCK inhibitor (50 µM), to form EBs. Approximately 1×10⁴ cells were plated per well of a standard V-bottom 96-well plate coated with Lipidure (1% v/v; AMSBio) to avoid having the EBs attach to the 96-well plate. After 4 days in EB media with bFGF (4 ng/ml) and ROCK inhibitor (50 µM), both the bFGF and ROCK inhibitor were discontinued leaving the brain organoids in basic EB media for an additional 3 days (7 days total). After the EB media phase, the EB media is replaced with neural epithelium (NE) media which consists of DMEM/F12, N2 supplement (0.1 % v/v), L-alanyl-L-glutamine (2mM), MEM-NEAA (0.1% v/v), Heparin solution (0.2mg/ml; Sigma), and filtered using 0.22 µm PES filter (EMD Milipore). The brain organoids were transferred to an ultra-low attachment 24-well plate (Corning) using cut P200 pipette tips, with 1-2 EBs per well in 1 ml NE media. The EBs were neuralized in the NE media for five days, after which they were transferred into Matrigel (Corning) using a mold created from siloconized parafilm and a sterile empty P200 box. The brain organoids were kept in a 6 cm suspension petri dish with differentiation media consisting of KO DMEM/F12 (50%), Neurobasal medium (50%), N2 supplement (0.1% v/v), B27 without vitamin A supplement (0.1% v/v), Insulin solution (0.1% v/v;Sigma), 2-Mercaptoethanol (0.1mM), L-alanyl-L-glutamine (2mM), MEM-NEAA (1x), and Penicillin/Streptomycin (0.1% v/v). After five days of being exposed to differentiation media containing B27 without vitamin A, the differentiation media was replaced by a formulation that is identical except for the replacement of B27 without vitamin A to B27 with vitamin A; at this time point, the brain organoids are also transferred to a 125 ml spinning flask bioreactor (Corning) siliconized with Sigmacote (Sigma), where they were fed differentiation media with vitamin A weekly for 8 weeks. After 12 weeks, Borgs were utilized for iMGL co-culture studies.

### Human Adult and Fetal Microglia Isolation

Briefly, normal appearing cortical tissue was resected from pharmacologically intractable non-malignant cases of temporal lobe epilepsy. Tissue was cleaned extensively and mechanically dissociated. A single cell suspension was generated following gentle enzymatic digestion using trypsin and DNAse prior to passing through a nylon mesh filter. The single cell suspension underwent a fickle ultracentrifugation step to remove myelin. Dissociated cells were centrifuged, counted, and plated at 2×10⁶ cells/mL in MEM supplemented with 5% FBS, 0.1% P/S and 0.1% glutamine. Microglia were grown for 3 days, collected and plated at 1×10⁵ cells/mL and maintained in culture for 6 days during which time cells received two treatments of TGFβ (20 ng/mL) on days 3 and 5. Human fetal brain tissue was obtained from the Fetal Tissue Repository (Albert Einstein College of Medicine, Bronx, NY). Total RNA was isolated using standard Trizol (Invitrogen) protocols and stored at -80 °C. In some embodiments, a suspension of small clumps of cells are produced and used in a similar manner as the single cell suspension.

### iMGL Transplantation in MITRG and Rag5xfAD Brains

All animal procedures were performed in accordance with NIH and University of California guidelines approved IAUC protocols (IAUC #2011-3004). MITRG mice were purchased from Jax (The Jackson Laboratory, #017711) and have been previously characterized (Rongvaux et al., 2014). MITRG mice allow for xenotransplantation and is designed to support human myeloid engraftment. iMGLs were harvested at day 38 and suspended in injection buffer: 1X HBSS with M-CSF (10 ng/ml), IL-34 (50 ng/ml), and TGFβ-1 (25 ng/ml). iMGLs were delivered using stereotactic surgery as previously described (Blurton-Jones, et al, 2009) using the following coordinates; AP: -0.6, ML: ± 2.0, DV: -1.65. Brains were collected from mice at day 60 post-transplantation per established protocols (Blurton-Jones, et al, 2009). Rag5xfAD mice were generated in this lab and previously characterized (Marsh et al., 2016). Rag5xfAD mice display robust beta-amyloid pathology and allow for xenotransplantation of human cells. iMGLs were transplanted into the hippocampi using the following coordinates; AP: -2.06, ML: ± 1.75, DV: -1.95. After transplantation mice were killed and brains collected using previously established protocol. Briefly, mice were anesthetized using sodium-barbiturate and perfused through the leftventricle with cold 1X HBSS for 4 min. Perfused mice were decapitated and brain extracted and dropped-fixed in PFA (4% w/v) for 48 hours at 4°C. Brains were then washed 3 times with PBS and sunk in sucrose (30% w/v) solution for 48 hours before coronal sectioning (40 µm) using a microtome (Leica). Free-floating sections were stored in PBS sodium azide (0.05%) solution at 4°C until IHC was performed.

### Dot blot

Serial dilutions of proteins (2 µl) were blotted on a pre-wet nitrocellulose paper and allowed to dry. After drying, blots were blocked with 5% BSA in 1x Tris-buffered saline with Tween 20 (TBST) for 1 h at room temperature with slight shaking. Next, blots were incubated with primary antibodies (see below) at room temperature for 1 hour. Blots were then washed 3 times for 5 min each with TBST. Blots were then incubated with HRP conjugated secondary antibody (Santa Cruz) at 1:10,000 for 1 h at room temperature with mild shaking. After 1 h, blots were washed 3 times for 5 min each with TBST. After wash, blots were dried on filter paper and incubated with Pierce ECL Western blotting development substrate (Thermo Fisher) for 10 min in the dark. Blots were imaged on ChemiDoc XRS+ imaging system (BioRad).

### AD-GWAS qPCR Primers

The following validated and available Taqman primers were used: APOE Hs00171168_m1, CR1 Hs00559342_m1, CD33 Hs01076281_m1, ABCA7 Hs01105117_m1, TREM2 Hs00219132_m1, TREMI,2 Hs01077557_m1, TYROBP (DAP12) Hs00182426_m1, PICALM Hs00200318_m1, CLU Hs00156548_m1, MS4A6A Hs01556747_m1, BIN1 Hs00184913_m1, CD2AP Hs00961451_m1, CASS4 Hs00220503_m1, MEF2C Hs00231149_m1, DSG2 Hs00170071_m1, MS4A4A Hs01106863_m1, ZCWPW1 Hs00215881_m1, INPP5D Hs00183290_m1, and PTK2B Hs00169444_m1.

It is contemplated that various combinations or subcombinations of the specific features and aspects of the embodiments disclosed above may be made and still fall within one or more of the inventions. Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed inventions. Thus, it is intended that the scope of the present inventions herein disclosed should not be limited by the particular disclosed embodiments described above. Moreover, while the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents, and alternatives falling within the spirit and scope of the various embodiments described and the appended claims. Any methods disclosed herein need not be performed in the order recited. The methods disclosed herein include certain actions taken by a practitioner; however, they can also include any third-party instruction of those actions, either expressly or by implication. For example, actions such as "administering a population of expanded NK cells" include "instructing the administration of a population of expanded NK cells." In addition, where features or aspects of the disclosure are described in terms of Markush groups, those skilled in the art will recognize that the disclosure is also thereby described in terms of any individual member or subgroup of members of the Markush group.

The ranges disclosed herein also encompass any and all overlap, subranges, and combinations thereof. Language such as "up to," "at least," "greater than," "less than," "between," and the like includes the number recited. Numbers preceded by a term such as "about" or "approximately" include the recited numbers. For example, "about 10 nanometers" includes "10 nanometers."

### Example 12

iHPC transplantation allows for studying human microglial development in a complete brain environment. Normal development and aging of human microglia in a complete CNS environment can be studied by transplanting iHPCs in the brains of mice (see FIGS. 24 and 25).

### References

Abbas, N., Bednar, I., Mix, E., Marie, S., Paterson, D., Ljungberg, A., Morris, C., Winblad, B., Nordberg, A., and Zhu, J. (2002). Up-regulation of the inflammatory cytokines IFN-gamma and IL-12 and down-regulation of IL-4 in cerebral cortex regions of APP(SWE) transgenic mice. J Neuroimmunol 126, 50-57.
Abdollahi, A., Lord, K.A., Hoffman-Liebermann, B., and Liebermann, D.A. (1991). Interferon regulatory factor 1 is a myeloid differentiation primary response gene induced by interleukin 6 and leukemia inhibitory factor: role in growth inhibition. Cell Growth Differ 2, 401-407.
Abutbul, S., Shapiro, J., Szaingurten-Solodkin, I., Levy, N., Carmy, Y., Baron, R., Jung, S., and Monsonego, A. (2012). TGF-beta signaling through SMAD2/3 induces the quiescent microglial phenotype within the CNS environment. Glia 60, 1160-1171.
Aguzzi, A., Barres, B.A., and Bennett, M.L. (2013). Microglia: scapegoat, saboteur, or something else? Science 339, 156-161.
Andreasson, K.I., Bachstetter, A.D., Colonna, M., Ginhoux, F., Holmes, C., Lamb, B., Landreth, G., Lee, D.C., Low, D., Lynch, M.A., et al. (2016). Targeting innate immunity for neurodegenerative disorders of the central nervous system. J Neurochem 138, 653-693.
Asai, H., Ikezu, S., Tsunoda, S., Medalla, M., Luebke, J., Haydar, T., Wolozin, B., Butovsky, O., Kugler, S., and Ikezu, T. (2015). Depletion of microglia and inhibition of exosome synthesis halt tau propagation. Nat Neurosci 18, 1584-1593.
Bachstetter, A.D., Van Eldik, L.J., Schmitt, F.A., Neltner, J.H., Ighodaro, E.T., Webster, S.J., Patel, E., Abner, E.L., Kryscio, R.J., and Nelson, P.T. (2015). Diseaserelated microglia heterogeneity in the hippocampus of Alzheimer's disease, dementia with Lewy bodies, and hippocampal sclerosis of aging. Acta Neuropathol Commun 3, 32.
Baron, R., Babcock, A.A., Nemirovsky, A., Finsen, B., and Monsonego, A. (2014). Accelerated microglial pathology is associated with Abeta plaques in mouse models of Alzheimer's disease. Aging Cell 13, 584-595.
Bennett, M.L., Bennett, F.C., Liddelow, S.A., Ajami, B., Zamanian, J.L., Fernhoff, N.B., Mulinyawe, S.B., Bohlen, C.J., Adil, A., Tucker, A., et al. (2016). New tools for studying microglia in the mouse and human CNS. Proc Natl Acad Sci U S A 113, E1738-1746.
Biber, K., Moller, T., Boddeke, E., and Prinz, M. (2016). Central nervous system myeloid cells as drug targets: current status and translational challenges. Nat Rev Drug Discov 15, 110-124.
Biber, K., Neumann, H., Inoue, K., and Boddeke, H.W. (2007). Neuronal 'On' and 'Off' signals control microglia. Trends Neurosci 30, 596-602.
Blum-Degen, D., Muller, T., Kuhn, W., Gerlach, M., Przuntek, H., and Riederer, P. (1995). Interleukin-1 beta and interleukin-6 are elevated in the cerebrospinal fluid of Alzheimer's and de novo Parkinson's disease patients. Neurosci Lett 202, 17-20.
Bradshaw, E.M., Chibnik, L.B., Keenan, B.T., Ottoboni, L., Raj, T., Tang, A., Rosenkrantz, L.L., Imboywa, S., Lee, M., Von Korff, A., et al. (2013). CD33 Alzheimer's disease locus: altered monocyte function and amyloid biology. Nat Neurosci 16, 848-850.
Butovsky, O., Jedrychowski, M.P., Moore, C.S., Cialic, R., Lanser, A.J., Gabriely, G., Koeglsperger, T., Dake, B., Wu, P.M., Doykan, C.E., et al. (2014). Identification of a unique TGF-beta-dependent molecular and functional signature in microglia. Nat Neurosci 17, 131-143.
Chan, W.Y., Kohsaka, S., and Rezaie, P. (2007). The origin and cell lineage of microglia: new concepts. Brain Res Rev 53, 344-354.
Chung, W.S., Clarke, L.E., Wang, G.X., Stafford, B.K., Sher, A., Chakraborty, C., Joung, J., Foo, L.C., Thompson, A., Chen, C., et al. (2013). Astrocytes mediate synapse elimination through MEGF10 and MERTK pathways. Nature 504, 394-400.
Crotti, A., Benner, C., Kerman, B.E., Gosselin, D., Lagier-Tourenne, C., Zuccato, C., Cattaneo, E., Gage, F.H., Cleveland, D.W., and Glass, C.K. (2014). Mutant Huntingtin promotes autonomous microglia activation via myeloid lineage-determining factors. Nat Neurosci 17, 513-521.
De Simone, R., Niturad, C.E., De Nuccio, C., Ajmone-Cat, M.A., Visentin, S., and Minghetti, L. (2010). TGF-beta and LPS modulate ADP-induced migration of microglial cells through P2Y1 and P2Y12 receptor expression. J Neurochem 115, 450-459.
Dobin, A., Davis, C.A., Schlesinger, F., Drenkow, J., Zaleski, C., Jha, S., Batut, P., Chaisson, M., and Gingeras, T.R. (2013). STAR: ultrafast universal RNA-seq aligner. Bioinformatics 29, 15-21.
Durafourt, B.A., Moore, C.S., Blain, M., and Antel, J.P. (2013). Isolating, culturing, and polarizing primary human adult and fetal microglia. Methods Mol Biol 1041, 199-211.
Erny, D., Hrabe de Angelis, A.L., Jaitin, D., Wieghofer, P., Staszewski, O., David, E., Keren-Shaul, H., Mahlakoiv, T., Jakobshagen, K., Buch, T., et al. (2015). Host microbiota constantly control maturation and function of microglia in the CNS. Nat Neurosci 18, 965-977.
Fu, Y., Hsiao, J.H., Paxinos, G., Halliday, G.M., and Kim, W.S. (2016). ABCA7 Mediates Phagocytic Clearance of Amyloid-beta in the Brain. J Alzheimers Dis 54, 569-584.
Ginhoux, F., Greter, M., Leboeuf, M., Nandi, S., See, P., Gokhan, S., Mehler, M.F., Conway, S.J., Ng, L.G., Stanley, E.R., et al. (2010). Fate mapping analysis reveals that adult microglia derive from primitive macrophages. Science 330, 841-845.
Glass, C.K., and Natoli, G. (2016). Molecular control of activation and priming in macrophages. Nat Immunol 17, 26-33.
Gosselin, D., Link, V.M., Romanoski, C.E., Fonseca, G.J., Eichenfield, D.Z., Spann, N.J., Stender, J.D., Chun, H.B., Garner, H., Geissmann, F., et al. (2014). Environment drives selection and function of enhancers controlling tissue-specific macrophage identities. Cell 159, 1327-1340.
Grabert, K., Michoel, T., Karavolos, M.H., Clohisey, S., Baillie, J.K., Stevens, M.P., Freeman, T.C., Summers, K.M., and McColl, B.W. (2016). Microglial brain region-dependent diversity and selective regional sensitivities to aging. Nat Neurosci 19, 504-516.
Greer, P.L., Bear, D.M., Lassance, J.M., Bloom, M.L., Tsukahara, T., Pashkovski, S.L., Masuda, F.K., Nowlan, A.C., Kirchner, R., Hoekstra, H.E., et al. (2016). A Family of non-GPCR Chemosensors Defines an Alternative Logic for Mammalian Olfaction. Cell 165, 1734-1748.
Greter, M., Lelios, I., Pelczar, P., Hoeffel, G., Price, J., Leboeuf, M., Kundig, T.M., Frei, K., Ginhoux, F., Merad, M., et al. (2012). Stroma-derived interleukin-34 controls the development and maintenance of langerhans cells and the maintenance of microglia. Immunity 37, 1050-1060.
Guillot-Sestier, M.V., and Town, T. (2013). Innate immunity in Alzheimer's disease: a complex affair. CNS Neurol Disord Drug Targets 12, 593-607.
Gylys, K.H., Fein, J.A., and Cole, G.M. (2000). Quantitative characterization of crude synaptosomal fraction (P-2) components by flow cytometry. J Neurosci Res 61, 186-192.
Hanna, R.N., Carlin, L.M., Hubbeling, H.G., Nackiewicz, D., Green, A.M., Punt, J.A., Geissmann, F., and Hedrick, C.C. (2011). The transcription factor NR4A1 (Nur77) controls bone marrow differentiation and the survival of Ly6C- monocytes. Nat Immunol 12, 778-785.
Haynes, S.E., Hollopeter, G., Yang, G., Kurpius, D., Dailey, M.E., Gan, W.B., and Julius, D. (2006). The P2Y12 receptor regulates microglial activation by extracellular nucleotides. Nat Neurosci 9, 1512-1519.
Hickman, S.E., Allison, E.K., and El Khoury, J. (2008). Microglial dysfunction and defective beta-amyloid clearance pathways in aging Alzheimer's disease mice. J Neurosci 28, 8354-8360.
Hickman, S.E., Kingery, N.D., Ohsumi, T.K., Borowsky, M.L., Wang, L.C., Means, T.K., and El Khoury, J. (2013). The microglial sensome revealed by direct RNA sequencing. Nat Neurosci 16, 1896-1905.
Hong, S., Beja-Glasser, V.F., Nfonoyim, B.M., Frouin, A., Li, S., Ramakrishnan, S., Merry, K.M., Shi, Q., Rosenthal, A., Barres, B.A., et al. (2016a). Complement and microglia mediate early synapse loss in Alzheimer mouse models. Science 352, 712-716.
Hong, S., Dissing-Olesen, L., and Stevens, B. (2016b). New insights on the role of microglia in synaptic pruning in health and disease. Curr Opin Neurobiol 36, 128-134.
Karch, C.M., Ezerskiy, L.A., Bertelsen, S., Alzheimer's Disease Genetics, C., and Goate, A.M. (2016). Alzheimer's Disease Risk Polymorphisms Regulate Gene Expression in the ZCWPW1 and the CELF1 Loci. PLoS One 11, e0148717.
Kennedy, M., D'Souza, S.L., Lynch-Kattman, M., Schwantz, S., and Keller, G. (2007). Development of the hemangioblast defines the onset of hematopoiesis in human ES cell differentiation cultures. Blood 109, 2679-2687.
Kettenmann, H., Hanisch, U.K., Noda, M., and Verkhratsky, A. (2011). Physiology of microglia. Physiol Rev 91, 461-553.
Kierdorf, K., Erny, D., Goldmann, T., Sander, V., Schulz, C., Perdiguero, E.G., Wieghofer, P., Heinrich, A., Riemke, P., Holscher, C., et al. (2013). Microglia emerge from erythromyeloid precursors via Pu.1- and Irf8-dependent pathways. Nat Neurosci 16, 273-280.
Kierdorf, K., and Prinz, M. (2013). Factors regulating microglia activation. Front Cell Neurosci 7, 44.
Koenigsknecht-Talboo, J., and Landreth, G.E. (2005). Microglial phagocytosis induced by fibrillar beta-amyloid and IgGs are differentially regulated by proinflammatory cytokines. J Neurosci 25, 8240-8249.
Lancaster, M.A., Renner, M., Martin, C.A., Wenzel, D., Bicknell, L.S., Hurles, M.E., Homfray, T., Penninger, J.M., Jackson, A.P., and Knoblich, J.A. (2013). Cerebral organoids model human brain development and microcephaly. Nature 501, 373-379.
Lasagna-Reeves, C.A., Castillo-Carranza, D.L., Sengupta, U., Sarmiento, J., Troncoso, J., Jackson, G.R., and Kayed, R. (2012). Identification of oligomers at early stages of tau aggregation in Alzheimer's disease. FASEB J 26, 1946-1959.
Lavin, Y., Winter, D., Blecher-Gonen, R., David, E., Keren-Shaul, H., Merad, M., Jung, S., and Amit, I. (2014). Tissue-resident macrophage enhancer landscapes are shaped by the local microenvironment. Cell 159, 1312-1326.
Li, B., and Dewey, C.N. (2011). RSEM: accurate transcript quantification from RNA-Seq data with or without a reference genome. BMC Bioinformatics 12, 323.
Linnartz-Gerlach, B., Mathews, M., and Neumann, H. (2014). Sensing the neuronal glycocalyx by glial sialic acid binding immunoglobulin-like lectins. Neuroscience 275, 113-124.
Liu, Z., Condello, C., Schain, A., Harb, R., and Grutzendler, J. (2010). CX3CR1 in microglia regulates brain amyloid deposition through selective protofibrillar amyloid-beta phagocytosis. J Neurosci 30, 17091-17101.
Loo, D.T., Copani, A., Pike, C.J., Whittemore, E.R., Walencewicz, A.J., and Cotman, C.W. (1993). Apoptosis is induced by beta-amyloid in cultured central nervous system neurons. Proc Natl Acad Sci U S A 90, 7951-7955.
Lui, H., Zhang, J., Makinson, S.R., Cahill, M.K., Kelley, K.W., Huang, H.Y., Shang, Y., Oldham, M.C., Martens, L.H., Gao, F., et al. (2016). Progranulin Deficiency Promotes Circuit-Specific Synaptic Pruning by Microglia via Complement Activation. Cell 165, 921-935.
Marsh, S.E., Abud, E.M., Lakatos, A., Karimzadeh, A., Yeung, S.T., Davtyan, H., Fote, G.M., Lau, L., Weinger, J.G., Lane, T.E., et al. (2016). The adaptive immune system restrains Alzheimer's disease pathogenesis by modulating microglial function. Proc Natl Acad Sci U S A 113, E1316-1325.
Matcovitch-Natan, O., Winter, D.R., Giladi, A., Vargas Aguilar, S., Spinrad, A., Sarrazin, S., Ben-Yehuda, H., David, E., Zelada Gonzalez, F., Perrin, P., et al. (2016). Microglia development follows a stepwise program to regulate brain homeostasis. Science 353, aad8670.
Mildner, A., Huang, H., Radke, J., Stenzel, W., and Priller, J. (2017). P2Y12 receptor is expressed on human microglia under physiological conditions throughout development and is sensitive to neuroinflammatory diseases. Glia 65, 375-387.
Moore, C.S., Ase, A.R., Kinsara, A., Rao, V.T., Michell-Robinson, M., Leong, S.Y., Butovsky, O., Ludwin, S.K., Seguela, P., Bar-Or, A., et al. (2015). P2Y12 expression and function in alternatively activated human microglia. Neurol Neuroimmunol Neuroinflamm 2, e80.
Muffat, J., Li, Y., Yuan, B., Mitalipova, M., Omer, A., Corcoran, S., Bakiasi, G., Tsai, L.H., Aubourg, P., Ransohoff, R.M., et al. (2016). Efficient derivation of microglia-like cells from human pluripotent stem cells. Nat Med.
O'Rourke, J.G., Bogdanik, L., Yanez, A., Lall, D., Wolf, A.J., Muhammad, A.K., Ho, R., Carmona, S., Vit, J.P., Zarrow, J., et al. (2016). C9orf72 is required for proper macrophage and microglial function in mice. Science 351, 1324-1329.
Paolicelli, R.C., Bolasco, G., Pagani, F., Maggi, L., Scianni, M., Panzanelli, P., Giustetto, M., Ferreira, T.A., Guiducci, E., Dumas, L., et al. (2011). Synaptic pruning by microglia is necessary for normal brain development. Science 333, 1456-1458.
Patel, N.S., Paris, D., Mathura, V., Quadros, A.N., Crawford, F.C., and Mullan, M.J. (2005). Inflammatory cytokine levels correlate with amyloid load in transgenic mouse models of Alzheimer's disease. J Neuroinflammation 2, 9.
Prinz, M., and Priller, J. (2010). Tickets to the brain: role of CCR2 and CX3CR1 in myeloid cell entry in the CNS. J Neuroimmunol 224, 80-84.
Prinz, M., and Priller, J. (2014). Microglia and brain macrophages in the molecular age: from origin to neuropsychiatric disease. Nat Rev Neurosci 15, 300-312.
Prinz, M., Priller, J., Sisodia, S.S., and Ransohoff, R.M. (2011). Heterogeneity of CNS myeloid cells and their roles in neurodegeneration. Nat Neurosci 14, 1227-1235.
Rezaie, P., and Male, D. (1999). Colonisation of the developing human brain and spinal cord by microglia: a review. Microsc Res Tech 45, 359-382.
Robinson, M.D., McCarthy, D.J., and Smyth, G.K. (2010). edgeR: a Bioconductor package for differential expression analysis of digital gene expression data. Bioinformatics 26, 139-140.
Rongvaux, A., Willinger, T., Martinek, J., Strowig, T., Gearty, S.V., Teichmann, L.L., Saito, Y., Marches, F., Halene, S., Palucka, A.K., et al. (2014). Development and function of human innate immune cells in a humanized mouse model. Nat Biotechnol 32, 364-372.
Rustenhoven, J., Park, T.I., Schweder, P., Scotter, J., Correia, J., Smith, A.M., Gibbons, H.M., Oldfield, RL., Bergin, P.S., Mee, E.W., et al. (2016). Isolation of highly enriched primary human microglia for functional studies. Sci Rep 6, 19371.
Schilling, T., Nitsch, R, Heinemann, U., Haas, D., and Eder, C. (2001). Astrocyte-released cytokines induce ramification and outward K+ channel expression in microglia via distinct signalling pathways. Eur J Neurosci 14, 463-473.
Schulz, C., Gomez Perdiguero, E., Chorro, L., Szabo-Rogers, H., Cagnard, N., Kierdorf, K., Prinz, M., Wu, B., Jacobsen, S.E., Pollard, J.W., et al. (2012). A lineage of myeloid cells independent of Myb and hematopoietic stem cells. Science 336, 86-90.
Shulman, J.M., Imboywa, S., Giagtzoglou, N., Powers, M.P., Hu, Y., Devenport, D., Chipendo, P., Chibnik, L.B., Diamond, A., Perrimon, N., et al. (2014). Functional screening in Drosophila identifies Alzheimer's disease susceptibility genes and implicates Tau-mediated mechanisms. Hum Mol Genet 23, 870-877.
Sirkis, D.W., Bonham, L.W., Aparicio, R.E., Geier, E.G., Ramos, E.M., Wang, Q., Karydas, A., Miller, Z.A., Miller, B.L., Coppola, G., et al. (2016). Rare TREM2 variants associated with Alzheimer's disease display reduced cell surface expression. Acta Neuropathol Commun 4, 98.
Stalder, A.K., Ermini, F., Bondolfi, L., Krenger, W., Burbach, G.J., Deller, T., Coomaraswamy, J., Staufenbiel, M., Landmann, R., and Jucker, M. (2005). Invasion of hematopoietic cells into the brain of amyloid precursor protein transgenic mice. J Neurosci 25, 11125-11132.
Stephan, A.H., Barres, B.A., and Stevens, B. (2012). The complement system: an unexpected role in synaptic pruning during development and disease. Annu Rev Neurosci 35, 369-389.
Sturgeon, C.M., Ditadi, A., Awong, G., Kennedy, M., and Keller, G. (2014). Wnt signaling controls the specification of definitive and primitive hematopoiesis from human pluripotent stem cells. Nat Biotechnol 32, 554-561.
Villegas-Llerena, C., Phillips, A., Garcia-Reitboeck, P., Hardy, J., and Pocock, J.M. (2015). Microglial genes regulating neuroinflammation in the progression of Alzheimer's disease. Curr Opin Neurobiol 36, 74-81.
Wang, W.Y., Tan, M.S., Yu, J.T., and Tan, L. (2015). Role of proinflammatory cytokines released from microglia in Alzheimer's disease. Ann Transl Med 3, 136.
Wang, Y., Szretter, K.J., Vermi, W., Gilfillan, S., Rossini, C., Cella, M., Barrow, A.D., Diamond, M.S., and Colonna, M. (2012). IL-34 is a tissue-restricted ligand of CSF1R required for the development of Langerhans cells and microglia. Nat Immunol 13, 753-760.
Yamasaki, R., Lu, H., Butovsky, O., Ohno, N., Rietsch, A.M., Cialic, R., Wu, P.M., Doykan, C.E., Lin, J., Cotleur, A.C., et al. (2014). Differential roles of microglia and monocytes in the inflamed central nervous system. J Exp Med 211, 1533-1549.
Yeh, F.L., Wang, Y., Tom, I., Gonzalez, L.C., and Sheng, M. (2016). TREM2 Binds to Apolipoproteins, Including APOE and CLU/APOJ, and Thereby Facilitates Uptake of Amyloid-Beta by Microglia. Neuron 91, 328-340.
Zhang, Y., Chen, K., Sloan, S.A., Bennett, M.L., Scholze, A.R., O'Keeffe, S., Phatnani, H.P., Guarnieri, P., Caneda, C., Ruderisch, N., et al. (2014). An RNA-sequencing transcriptome and splicing database of glia, neurons, and vascular cells of the cerebral cortex. J Neurosci 34, 11929-11947.

### THE FOLLOWING STATEMENTS ARE NOT THE CLAIMS BUT ARE FURTHER ASPECTS OF THE INVENTION:

1. A method of producing human microglial-like cells (iMGLs) from pluripotent stem cells (PSCs) comprising the steps of:
   (i) differentiating PSCs using a media supplemented with hematopoietic differentiation factors to produce induced hematopoietic progenitor cells (iHPCs); and
   (ii) differentiating CD43⁺ iHPCs into human microglial-like cells (iMGLs) using a microglial differentiating media.
2. The method of statement 1, wherein step (i) comprises an incubation period that is between 3 days and 21 days.
3. The method of statement 2, wherein the incubation period of step (i) is 10 days.
4. The method of statement 3, wherein during days 1 through 10 of the incubation period the PSCs are incubated in a hypoxic or normoxic environment.
5. The method according to statements 3 or 4,
   wherein during days 1 and 2 of the incubation period the media comprises the hematopoietic differentiation factors of FGF2, BMP4, Activin A, LiCl, and VEGF;
   wherein during days 3 and 4 of the incubation period the media comprises the hematopoietic differentiation factors of FGF2 and VEGF; and
   wherein during days 5 through 10 of the incubation period the media comprises the
   hematopoietic differentiation factors of FGF2, VEGF, TPO, SCF, IL3, and IL6.
6. The method of statement 5,
   wherein the concentration of each of FGF2, BMP4, VEGF, TPO, SCF, IL-3, and IL-6 in the media is between 5ng/ml and 100ng/ml, and
   wherein the concentration of Activin A in the media is between 0.1ng/ml and 30ng/ml.
7. The method of statement 6,
   wherein the concentration of each of FGF2, BMP4, VEGF, TPO, SCF, IL-3, and IL-6 in the media is about 50ng/ml,
   wherein the concentration of Activin A in the media is about 12.5ng/ml, and wherein the concentration of LiCl is about 2mM.
8. The method of statement 1, further comprising isolating the CD43⁺ iHPCs using fluorescence activated cell sorting (FACS).
9. The method of statement 8, wherein isolating the CD43⁺ iHPCs is done through selecting for a CD43⁺ marker.
10. The method of statement 9, wherein the isolated iHPCs are greater than 80% pure.
11. The method of statement 10, wherein the isolated iHPCs are greater than 90% pure.
12. The method of statement 1, wherein step (ii) further comprises an incubation period that is between 20 and 30 days.
13. The method of statement 12, wherein step (ii) further comprises an incubation period that is about 25 days.
14. The method of statement 1, wherein the microglial differentiating media of step (ii) comprises the factors CSF-1, IL-34, and TGFβ1.
15. The method of statement 14, wherein the microglial differentiating media is serum free.
16. The method of statement 14 or 15,
   wherein the concentration of CSF-1 in the media is between 5ng/ml and 50ng/ml,
   wherein the concentration of IL-34 in the media is between 25ng/ml and 125ng/ml, and
   wherein the concentration of TGFβ1 in the media is between 2.5ng/ml and 100ng/ml.
17. The method of statement 16,
   wherein the concentration of CSF-1 is about 25ng/ml,
   wherein the concentration of IL-34 is about 100ng/ml, and
   wherein the concentration of TGFβ1 is about 50ng/ml.
18. The method of statement 1, wherein step (ii) comprises maturing the iMGLs with an incubation period that is between 1 and 5 days.
19. The method of statement 18, wherein step (ii) comprises maturing the iMGLs with an incubation period that is 3 days.
20. The method of statement 19, wherein step (ii) comprises maturing the iMGLs with incubating the iMGLs in media comprising CD200 and CX3CL1.
21. The method of statement 20, wherein CD200 is human recombinant CD200 and CX3CL1 is human recombinant CX3CL1.
22. The method of statement 20 or 21, wherein the concentration of each of CD200 and CX3CL1 in the media is between 1ng/ml and 1µg/ml.
23. The method of statement 22, wherein the concentration of each of CD200 and CX3CL1 is 100 ng/ml.
24. The method of statement 1, wherein the iMGLs produced are c-kit⁻/CD45⁺.
25. The method of statement 24, wherein the c-kit⁻/CD45⁺ iMGLs can be detected on day 14 of the 25 day incubation period.
26. The method of statement 25, further comprising testing for expression of factors that are known markers for commitment to a microglia fate.
27. The method of statement 26, wherein the expression of factors tested comprise PU.1 and TREM2.
28. The method of statement 1, wherein the iMGLs comprise two separate populations of iMGLs: (1) CD45⁺/CX3CR1⁻ and (2) CD45⁺/CX3CR1⁺.
29. The method of statement 1, wherein the iMGLs produced at least 70% pure.
30. The method of statement 29, wherein the iMGLs produced at least 96% pure.
31. The method of statement 29 or 30, wherein purity levels are assessed using purinergic receptor, P2ry12 expression, Trem2 expression, Iba1 expression, or Pu1 expression.
32. The method of statement 1, wherein the CD43⁺ iHPCs are CD235a⁺/CD41a⁺.
33. A method of producing human microglial-like cells (iMGLs) from pluripotent stem cells (PSCs) comprising the steps of:
   (i) differentiating PSCs into induced hematopoietic progenitors (iHPCs);
      and
   (ii) differentiating the iHPCs to produce iMGLs.
34. The method of statement 33, further comprising step (iii) of maturing the iMGLs by incubating the iMGLS in media comprising CD200 and CX3CL1.
35. The method of statement 33, wherein the iHPCs are CD43⁺/CD235a⁺/CD41⁺.
36. The method of statement 33, wherein the iHPCs are differentiated into the iMGLS in step (ii) by placing the iHPCs in a serum-free differentiating media.
37. The method of statement 36, wherein the serum-free differentiating media comprises MCSF, IL-34, and TGFβ1.
38. The method of statement 33, wherein the iMGLs produced are c-kit⁻/CD45⁺.
39. The method of statement 33, wherein the iMGLs comprise two separate populations of iMGLs: (1) CD45⁺/CX3R1⁻ and (2) CD45⁺/CX3R1⁺.
40. The method of statement 33, wherein step (i) comprises an incubation period between 5 and 15 days; and step (ii) comprises an incubation period between 20 and 30 days.
41. The method of statement 40, wherein step (i) comprises an incubation period of 10 days; and step (ii) comprises an incubation period of 25 days.
42. The method of statement 34, wherein step (iii) comprises an incubation period between 1 and 5 days.
43. The method of statement 42, wherein step (iii) comprises an incubation period of 3 days.
44. A composition of iMGLs comprising expression of any combination of the following genes: RUNX1, PU.1, CSF1FR, CX3CR1, TGFBR1, RSG10, GAS6, PROS1, P2RY12, GPR34, C1Q, CR3, CABLES1, BHLHE41, TREM2, ITAM, APOE, SLCO2B1, SLC7A8, PPARD, C9orf72, GRN, LRRK2, TARDBP, and CRYBB1.
45. The composition of statement 44 wherein the TREM2 and P2RY12 co-localize.
46. The composition of statement 44 or 45, wherein the genes KLF2, TREM1, MPT, ITGAL, and ADGRE5 are not expressed.
47. A composition of iMGLs, comprising expression of CD33, MS4A4A, and CR1, when the iMGLs are in their basal state.
48. The composition of any of statements 44-47, wherein the iMGLs secrete any combination of the chemokines: TNFα, CCL2, CCL4, and CXCL10 in response to stimulation by lipopolysaccharides, IFGγ, or IL-1β.
49. The composition of any of statements 44-47, wherein the iMGLs migrate in response to ADP.
50. The composition of any of statements 44-47, wherein ADP stimulation results in the generation of calcium transients in the iMGLs.
51. The composition of any of statements 44-47, wherein the iMGLs are capable of phagocytosing human synaptosomes.
52. The composition of any of statements 44-47, wherein the iMGLs are capable of synaptic pruning.
53. The composition of statement 52, wherein the synaptic pruning is mediated through a C1q/CR3 pathway.
54. The composition of any of statements 44-47, wherein the iMGLs are capable of phagocytosing Aβ and tau.
55. The composition of any of statements 44-47, wherein the iMGLs are capable of phagocytosing fluorescently labeled fibrillar Aβ and pHrodo-labeled brain-derived tau oligomers.
56. A method of profiling secretion of inflammatory molecules from iMGLs comprising:
   (i) treating the iMGLs with lipopolysaccharide, IFNγ, TNFα, or IL-1β;
      and
   (ii) measuring inflammation markers secreted by the iMGLs.
57. The method of statement 56, wherein the inflammation markers secreted by the iMGLs comprise inflammatory markers selected from CCL2, CCL4, and CXCL10.
58. A method of assessing iMGL migration comprising:
   (i) treating the iMGLs with ADP; and
   (ii) assessing iMGL motility and migration in response to chemical stimuli.
59. A method of producing calcium transients in iMGLs comprising:
   (i) treating the iMGLS with ADP; and
   (ii) interrogating calcium flux signals in the iMGLs; wherein the calcium flux signals are produced in response to electrical, biological, or chemical stimulation.
60. A method of studying microglia phagocytosis of compounds comprising:
   (i) exposing iMGLs to a compound selected from the group consisting of Aβ, Tau, fluorescently labeled Aβ, and pHrodo-labeled brain-derived tau oligomers, wherein the compound is phagocytosed, endocytosed, or ingested by the iMGLs; and
   (ii) measuring the phagocytosis, endocytosis, or ingestion of the compound.
61. A method of establishing an iMGL gene expression profile resembling the *in vivo* state of the iMGLs, comprising:
   co-culturing iMGLs with neurons, astrocytes, or other cells of the central nervous system, thereby recapitulating a more *in vivo* state for the iMGLs than would otherwise be present for the iMGLs if the iMGLs were not co-cultured with the neurons, astrocytes, or other cells of the central nervous system.
62. The method of statement 61, wherein the neurons are rat-hippocampal neurons.
63. The method of statement 61 or 62, wherein the differentially regulated genes are CABLES, TRIM4, MITF, MMP2, and SLCA25; and wherein the differentially regulated genes are upregulated in iMGLs.
64. A method of integrating iMGLs into a 3D CNS environment, comprising:
   co-culturing iMGLs with hiPSC 3D brain-organoids (BORGs), wherein the iMGLs migrate into the BORGs and populate the BORGS, or are incorporated into the BORGs.
65. A method of studying microglia dysregulation in health and disease using iMGLs, comprising:
   (i) exposing iMGLs to a compound selected from the group consisting of Aβ, Tau, fluorescently labeled Aβ, pHrodo-labeled brain-derived tau oligomers, and alpha-synuclein; and
   (ii) profiling an iMGL -omic signature selected from RNA-seq, proteomics, metabolomics, and lipidomics.
66. The method of statement 65, wherein the differentially regulated genes are CD33, TYROPB, and PICALM; and wherein the differentially regulated genes are upregulated in iMGLS.
67. A method of investigating the role of microglia in synaptic pruning and plasticity comprising: (i) exposing iMGLs to human synaptosomes and (ii) assessing human synaptosome phagocytosis by the iMGLs.
68. A method of assessing gene regulation in iMGLs in response to neuronal cues comprising:
   (i) exposing iMGLs to factors present in the central nervous system selected from the group consisting of CX3CL1, CD200, and TGFβ; and
   (ii) assessing differentially regulated genes comprising genes selected from the group consisting P2RY12, EGR1, TGFβR1, ETV5, CX3CR1, APOE, BIN1, CD33, GPR84, COMT, APP, PSEN1, PSEN2, HTT, GRN, FUS, TARDP, VCP, SNCA, C9ORF72, LRRK2, and SOD1.
69. A method of assessing engraftment of iMGLs into a cortex comprising:
   (i) transplanting iMGLs into a cortex
   (ii) assessing engraftment of the iMGLs into the cortex.
70. The method of statement 69, wherein step (ii) occurs two months after step (i).
71. The method of statement 69 or 70, further comprising transplanting iMGLS into the cortex of a mouse.
72. The method of statement 71, wherein the mouse is a MITRG mouse.
73. A method of assessing iMGL interaction with AD neuropathy comprising:
   (i) transplanting iMGLs into a mouse brain; and
   (ii) assessing iMGLs interacting with the mouse brain.
74. The method of statement 73, wherein step (ii) comprises assessing iMGL migration towards plaques.
75. The method of statement 73, wherein step (ii) comprises assessing iMGL phagocytosis of fibrillar Aβ.
76. A method of studying human microglia in a 3D neuronal environment comprising: transplanting iMGLs into a mammalian brain.
77. The method of statement 76, wherein the mammalian brain is a mouse.
78. The method of statement 77, wherein the iMGLs are transplanted into hippocampi of the mouse.
79. The method of statement 76 or 77, wherein the mouse is a wild-type mouse.
80. The method of statement 76 or 77, wherein the mouse is an AD mouse strain.
81. The method of statement 3, wherein during days 1 through 4 of the incubation period the PSCs are incubated in a 5% oxygen environment and during days 5 through 10 of the incubation period the PSCs are incubated in a 20% oxygen environment.
82. The method of statement 5,
   wherein the concentration of each of FGF2, BMP4, VEGF, TPO, SCF, IL-3, and IL-6 in the media is between 30ng/ml and 70ng/ml,
   wherein the concentration of Activin A in the media is between 11ng/ml and 14ng/ml, and
   wherein LiCl is between 1mM and 3mM.
83. The method of statement 14 or 15,
   wherein the concentration of CSF-1 in the media is between 15ng/ml and 35ng/ml,
   wherein the concentration of IL-34 in the media is between 80ng/ml and 120ng/ml, and
   wherein the concentration of TGFβ1 in the media is between 30ng/ml and 70ng/ml.
84. The method of statement 1, wherein the microglial differentiating media of step (iii) comprises the factors CSF-1, IL-34, and TGFβ2.
85. The method of statement 84,
   wherein the concentration of CSF-1 in the media is between 5ng/ml and 50ng/ml,
   wherein the concentration of IL-34 in the media is between 25ng/ml and 125ng/ml, and
   wherein the concentration of TGFβ1 in the media is between 2.5ng/ml and 100ng/ml.
86. The method of statement 1, wherein the microglial differentiating media of step (iii) comprises the factors CSF-1, IL-34, and a TGFβ mimetic.
87. The method of statement 86, wherein the TGFβ mimetic activates a TGFβ signaling pathway.
88. The method of statement 1, wherein the PSCs comprise single-cell PSCs or small clumps of PSCs.
89. The method of statement 1, further comprising maturing the iMGLs.
90. The method of statement 1 or 33, wherein the PSCs are not derived from embryoid bodies.
91. The method of statement 24, wherein the c-kit⁻/CD45⁺ iMGLs are detected using flow cytometry, immunofluorescence microscopy, qPCR, RNA-seq, or proteinomics.
92. The method of statement 1 or 33, wherein the PSCs comprise induced PSCs (iPSCs).
93. The method of statement 1 or 33, wherein the PSCs comprise embryonic stem cells (ESCs).
94. The method of any of statements 1, 33, 92, or 93, wherein the PSCs are mammalian PSCs.
95. The method of any of statements 1, 33, 92, or 93, wherein the PSCs are human PSCs.
96. The method of any of statements 1, 33, 92, or 93, wherein the PSCs are mouse PSCs.
97. The method of statement 35, wherein the iHPCs are CD34⁺, CD31⁺, or CD45⁺.
98. The method of statement 1 or 33, wherein the PSCs are not CD43⁺, CD235a⁺, CD41⁺, CD34⁺, CD31⁺, or CD45⁺ before differentiation.
99. A method of producing a human microglial-like cell (iMGL) from a cell of a first type comprising the steps of:
   (i) differentiating a cell of a first type into an induced hematopoietic progenitor cell (iHPC); and
   (ii) differentiating the iHPC to produce an iMGL.
100. A media for supporting generation of human microglial-like cells (iHPCs), the media comprising FGF2, BMP4, Activin A, and LiCl.
101. A media for supporting generation of human microglial-like cells (iHPCs), the media comprising FGF2 and VEGF.
102. A media for supporting generation of human microglial-like cells (iHPCs), the media comprising FGF2, VEGF, TPO, SCF, IL3, and IL6.
103. A kit comprising a media for supporting generation of human microglial-like cells (iHPCs), the media comprising FGF2, BMP4, Activin A, and LiCl.
104. A kit comprising a media for supporting generation of human microglial-like cells (iHPCs), the media comprising FGF2 and VEGF.
105. A kit comprising a media for supporting generation of human microglial-like cells (iHPCs), the media comprising FGF2, VEGF, TPO, SCF, IL3, and IL6.
106. A media for supporting generation of human microglial-like cells (iMGLs), the media comprising CSF-1, IL-34, and TGFβ1.
107. A kit comprising a media for supporting generation of human microglial-like cells (iMGLs), the media comprising CSF-1, IL-34, and TGFβ1.
108. A media for supporting maturation or maintenance of human microglial-like cells (iMGLs), the media comprising CD200 and CX3CL1.
109. A kit comprising a media for supporting maturation or maintenance of human microglial-like cells (iMGLs), the media comprising CD200 and CX3CL1.

## Claims

1. A composition comprising a cell population comprising human microglial-like cells (iMGLs), wherein at least 80% of the cells of the population are iMGLs that express P2RY12 and TREM2.

2. The composition of claim 1, wherein the iMGLs were differentiated using a serum-free microglial differentiating media.

3. The composition of claim 0 or 2, wherein at least 96% of the cells of the population are iMGLs that express P2RY12 and TREM2.

4. The composition of any one of claims 0-3, wherein the iMGLs are capable of phagocytosing human synaptosomes.

5. The composition of any one of claims 0-4, wherein the iMGLs are capable of phagocytosing one or more of Aβ, fluorescently labeled Aβ, tau, or pHrodo-labeled brain-derived tau oligomers.

6. The composition of any one of claims 0-5, wherein IFNγ induces secretion of TNFα, CCL2, and CCL4.

7. The composition of any one of claims 0-6, wherein IL-1β induces secretion of TNFα, CCL2, CCL4, and CXCL10.

8. The composition of any one of claims 0-7, wherein ADP stimulation results in the generation of calcium transients in the iMGLs.

9. The composition of any one of claims 0-8, wherein the iMGLs migrate in response to ADP.

10. The composition of any one of claims 0-9, wherein the iMGLs migrate into a human brain organoid (BORG).

11. A method of producing human microglial-like cells (iMGLs) comprising:
contacting human induced hematopoietic progenitor cells (iHPCs) with a microglial differentiating medium comprising CSF-1, IL-34, and TGFβ1 or CSF-1, IL-34, and a TGFβ mimetic to differentiate the iHPCs into iMGLs.

12. The method of claim 11, wherein the microglial differentiating medium is a serum-free medium.

13. The method of claim 11 or 12, further comprising:
contacting human pluripotent stem cells (PSCs) with media supplemented with hematopoietic differentiation factors to differentiate the PSCs into the iHPCs.

14. The method of any one of claims 13, wherein the hematopoietic differentiation factors comprise FGF2 and VEGF.

15. The method of any one of claims 11-14, wherein at least 96% of the iHPCs differentiate into iMGLs that express P2RY12 and TREM2.

16. The composition of any one of claims 1-10 for use in a method of assessing engraftment of iMGLs into neural tissue, wherein the method comprises (i) transplanting iMGLs into the neural tissue and (ii) assessing engraftment of the iMGLs into the neural tissue.
